# EUROPEAN PATENT SPECIFICATION

(11) **EP 2 683 732 B1**
(45) Date of publication and mention of the grant of the patent: **24.08.2016**
(21) Application number: 12719604.6
(22) Date of filing: 12.03.2012
(51) Int. Cl.: C07K 14/385, C12N 15/65, C12N 15/80

(54) **VECTOR-HOST SYSTEM**
VEKTOR-HOST-SYSTEM
SYSTÈME VECTEUR-HÔTE

(30) Priority: 11.03.2011 EP 11157871
(43) Date of publication of application: 15.01.2014
(73) Proprietor: DSM IP Assets B.V., 6411 TE Heerlen (NL)
(72) Inventor: BOVENBERG, Roelof Ary Lans, NL-6100 AC Echt (NL); KIEL, Jan Andries Kornelis Willem, NL-9700 CC Groningen (NL); WENZEL, Thibaut José, NL-6100 AC Echt (NL); LOS, Alrik Pieter, NL-6100 AC Echt (NL)
(74) Representative: Monaco, Vania
(86) International application number: PCT/EP2012/054302
(87) International publication number: WO 2012/123429

(56) References cited:
- WO-A1-2008/138835
- WO-A2-03/000881
- HAGG PETER ET AL: "A host/plasmid system that is not dependent on antibiotics and antibiotic resistance genes for stable plasmid maintenance in Escherichia coli", JOURNAL OF BIOTECHNOLOGY, ELSEVIER SCIENCE PUBLISHERS, AMSTERDAM, NL, vol. 111, no. 1, 1 July 2004 (2004-07-01), pages 17-30, XP002316099, ISSN: 0168-1656, DOI: DOI:10.1016/J.JBIOTEC.2004.03.010
- P. HANACHI: "Characterization of the p33 Subunit of Eukaryotic Translation Initiation Factor-3 from Saccharomyces cerevisiae", JOURNAL OF BIOLOGICAL CHEMISTRY, vol. 274, no. 13, 26 March 1999 (1999-03-26), pages 8546-8553, XP055003934, ISSN: 0021-9258, DOI: 10.1074/jbc.274.13.8546
- NEUZA D S P CARVALHO ET AL: "Expanding the ku70 toolbox for filamentous fungi: establishment of complementation vectors and recipient strains for advanced gene analyses", APPLIED MICROBIOLOGY AND BIOTECHNOLOGY, SPRINGER, BERLIN, DE, vol. 87, no. 4, 27 April 2010 (2010-04-27) , pages 1463-1473, XP019841586, ISSN: 1432-0614
- FIERRO F ET AL: "Autonomously replicating plasmids carrying the AMA1 region in Penicillium chrysogenum.", CURRENT GENETICS APR 1996 LNKD- PUBMED:8625429, vol. 29, no. 5, April 1996 (1996-04), pages 482-489, XP008154994, ISSN: 0172-8083
- O BAÑUELOS: "Co-transformation with autonomous replicating and integrative plasmids in Penicillium chrysogenum is highly efficient and leads in some cases to rescue of the intact integrative plasmid", FUNGAL GENETICS AND BIOLOGY, vol. 40, no. 2, 1 November 2003 (2003-11-01), pages 83-92, XP55003871, ISSN: 1087-1845, DOI: 10.1016/S1087-1845(03)00081-1
- NAGIEC ET AL: "Sphingolipid synthesis as a target for antifungal drugs. Complementation of the inositol phosphorylceramide synthase defect in a mutant strain of Saccharomyces cerevisiae by the Aur1 gene", JOURNAL OF BIOLOGICAL CHEMISTRY, AMERICAN SOCIETY FOR BIOCHEMISTRY AND MOLECULAR BIOLOGY, vol. 272, no. 15, 11 April 1997 (1997-04-11), pages 9809-9817, XP002119020, ISSN: 0021-9258, DOI: 10.1074/JBC.272.15.9809
- HASHIDA-OKADO T ET AL: "Transformation system for prototrophic industrial yeasts using the AUR1 gene as a dominant selection marker", FEBS LETTERS, ELSEVIER, AMSTERDAM, NL, vol. 425, no. 1, 20 March 1998 (1998-03-20), pages 117-122, XP004262096, ISSN: 0014-5793, DOI: 10.1016/S0014-5793(98)00211-7

### SEQUENCE LISTING

<110> DSM IP Assets B.V.
<120> vector-host system
<130> 27970-WO-PCT
<160> 70
<170> PatentIn version 3.5
<210> 1
   <211> 55
   <212> DNA
   <213> Penicillium chrysogenum
<400> 1
   ggggacaagt ttgtacaaaa aagcaggctg atcgaaggaa gcagtcccta cactc 55
<210> 2
   <211> 55
   <212> DNA
   <213> Penicillium chrysogenum
<400> 2
   ggggaccact ttgtacaaga aagctgggtt gagactgaac aatgtgaaga cggag 55
<210> 3
   <211> 55
   <212> DNA
   <213> Penicillium chrysogenum
<400> 3
   ggggacaact ttgtatagaa aagttgagca tattctttca ctgttgcaga tctgc 55
<210> 4
   <211> 51
   <212> DNA
   <213> Penicillium chrysogenum
<400> 4
   ggggactgct tttttgtaca aacttgctat cccatccaga tgagtgcttc g 51
<210> 5
   <211> 53
   <212> DNA
   <213> Penicillium chrysogenum
<400> 5
   ggggacagct ttcttgtaca aagtggacac catgtctcca accgggaagt gag 53
<210> 6
   <211> 51
   <212> DNA
   <213> Penicillium chrysogenum
<400> 6
   ggggacaact ttgtataata aagttgggtg cttgggatgt tccatggtag c 51
<210> 7
   <211> 25
   <212> DNA
   <213> Penicillium chrysogenum
<400> 7
   cagtttacac tcaaccccaa tccag 25
<210> 8
   <211> 22
   <212> DNA
   <213> Penicillium chrysogenum
<400> 8
   aggttggtgg agaagccatt ag 22
<210> 9
   <211> 53
   <212> DNA
   <213> Penicillium chrysogenum
<400> 9
   ggggacagct ttcttgtaca aagtggatgg gaaactaacc acgtgcttgt acg 53
<210> 10
   <211> 51
   <212> DNA
   <213> Penicillium chrysogenum
<400> 10
   ggggacaact ttgtataata aagttgttca ccctgtctcg acttccttgt c 51
<210> 11
   <211> 57
   <212> DNA
   <213> Penicillium chrysogenum
<400> 11
   ggggacaact ttgtataata aagttgtggg ccctcaccct gtctcgactt ccttgtc 57
<210> 12
   <211> 33
   <212> DNA
   <213> Aspergillus nidulans
<400> 12
   agaggtaccg agttatagac ggtccggcat agg 33
<210> 13
   <211> 33
   <212> DNA
   <213> Aspergillus nidulans
<400> 13
   agaggatccg tttgctgtct atgtggggga ctg 33
<210> 14
   <211> 27
   <212> DNA
   <213> Aspergillus nidulans
<400> 14
   ggggtgcttc taaggtatga gtcgcaa 27
<210> 15
   <211> 35
   <212> DNA
   <213> Aspergillus nidulans
<400> 15
   agaacgcgtt aacgcagggt ttgagaactc cgatc 35
<210> 16
   <211> 33
   <212> DNA
   <213> Penicillium chrysogenum
<400> 16
   agaggatccg aggaagacgt gatcagagta agc 33
<210> 17
   <211> 42
   <212> DNA
   <213> Penicillium chrysogenum
<400> 17
   gaaagcggcc gcggtaccgt gcttgggatg ttccatggta gc 42
<210> 18
   <211> 22
   <212> DNA
   <213> Penicillium chrysogenum
<400> 18
   cacgtagcat acaaccgtgt cg 22
<210> 19
   <211> 24
   <212> DNA
   <213> Penicillium chrysogenum
<400> 19
   gatgccttgt gggaaattaa ccag 24
<210> 20
   <211> 55
   <212> DNA
   <213> Penicillium chrysogenum
<400> 20
   ggggacaagt ttgtacaaaa aagcaggctg agaggaagac gtgatcagag taagc 55
<210> 21
   <211> 54
   <212> DNA
   <213> Penicillium chrysogenum
<400> 21
   ggggaccact ttgtacaaga aagctgggtt gtgcttggga tgttccatgg tagc 54
<210> 22
   <211> 24
   <212> DNA
   <213> Penicillium chrysogenum
<400> 22
   gttcttgaat agccgaggac tcac 24
<210> 23
   <211> 24
   <212> DNA
   <213> Penicillium chrysogenum
<400> 23
   catcctcccc ttctgttggc atag 24
<210> 24
   <211> 37
   <212> DNA
   <213> Aspergillus nidulans
<400> 24
   agaaagcttg gtaccgttgc accaatcgcc gtttagg 37
<210> 25
   <211> 47
   <212> DNA
   <213> Aspergillus nidulans
<400> 25
   agaagatctg tcgacgaatt cggtgaaggt tgtgttatgt tttgtgg 47
<210> 26
   <211> 33
   <212> DNA
   <213> Aspergillus nidulans
<400> 26
   agaagatctg atcgttggtg tcgatgtcag ctc 33
<210> 27
   <211> 28
   <212> DNA
   <213> Aspergillus nidulans
<400> 27
   ggggtacaca gtacacgagg acttctag 28
<210> 28
   <211> 1545
   <212> DNA
   <213> Trichoderma reesei
<400> 28
   atgtatcgga agttggccgt catctcggcc ttcttggcca cagctcgtgc tcagtcggcc        60
   tgcactctcc aatcggagac tcacccgcct ctgacatggc agaaatgctc gtctggtggc       120
   acgtgcactc aacagacagg ctccgtggtc atcgacgcca actggcgctg gactcacgct       180
   acgaacagca gcacgaactg ctacgatggc aacacttgga gctcgaccct atgtcctgac       240
   aacgagacct gcgcgaagaa ctgctgtctg gacggtgccg cctacgcgtc cacgtacgga       300
   gttaccacga gcggtaacag cctctccatt ggctttgtca cccagtctgc gcagaagaac       360
   gttggcgctc gcctttacct tatggcgagc gacacgacct accaggaatt caccctgctt       420
   ggcaacgagt tctctttcga tgttgatgtt tcgcagctgc cgtgcggctt gaacggagct       480
   ctctacttcg tgtccatgga cgcggatggt ggcgtgagca agtatcccac caacaccgct       540
   ggcgccaagt acggcacggg gtactgtgac agccagtgtc cccgcgatct gaagttcatc       600
   aatggccagg ccaacgttga gggctgggag ccgtcatcca acaacgcgaa cacgggcatt       660
   ggaggacacg gaagctgctg ctctgagatg gatatctggg aggccaactc catctccgag       720
   gctcttaccc cccacccttg cacgactgtc ggccaggaga tctgcgaggg tgatgggtgc       780
   ggcggaactt actccgataa cagatatggc ggcacttgcg atcccgatgg ctgcgactgg       840
   aacccatacc gcctgggcaa caccagcttc tacggccctg gctcaagctt taccctcgat       900
   accaccaaga aattgaccgt tgtcacccag ttcgagacgt cgggtgccat caaccgatac       960
   tatgtccaga atggcgtcac tttccagcag cccaacgccg agcttggtag ttactctggc      1020
   aacgagctca acgatgatta ctgcacagct gaggaggcag aattcggcgg atcctctttc      1080
   tcagacaagg gcggcctgac tcagttcaag aaggctacct ctggcggcat ggttctggtc      1140
   atgagtctgt gggatgatta ctacgccaac atgctgtggc tggactccac ctacccgaca      1200
   aacgagacct cctccacacc cggtgccgtg cgcggaagct gctccaccag ctccggtgtc      1260
   cctgctcagg tcgaatctca gtctcccaac gccaaggtca ccttctccaa catcaagttc      1320
   ggacccattg gcagcaccgg caaccctagc ggcggcaacc ctcccggcgg aaacccgcct      1380
   ggcaccacca ccacccgccg cccagccact accactggaa gctctcccgg acctacccag      1440
   tctcactacg gccagtgcgg cggtattggc tacagcggcc ccacggtctg cgccagcggc      1500
   acaacttgcc aggtcctgaa cccttactac tctcagtgcc tgtaa                      1545
<210> 29
   <211> 4680
   <212> DNA
   <213> Aspergillus niger
<400> 29
   gggggcactg tcgatcttat cacattcaca atcatcgagc tatcgcctaa tatgcgtcta 60
   aaggaggaag cacccggtac tggatctcta tgtggaagca cctttgtcaa cagacgcttt 120
   gaagagatgc taaatgaccg cctctcctcc ctccctggct gggacaggga tacactggat 180
   gaagcaatgc atcgatttga aactgtcgct aaaagaactt tcagtggaaa tacagacgat 240
   tacttcatgt tccctgttcc aggtatagca gacagccagg aagtcggggt tcgtcgcggc 300
   cgattccgag ttactggcca agagatgcag caactgtttt tgcctatcct acgagacatt 360
   gaggaccttg tccgagagca gatcgagacc tctgacgctc aggtaaaagc aattttcctg 420
   gttggagggt ttggacagag cccatacctt cgcacatatc ttcgcgactg cttctctcct 480
   gaagtcgaag tgatagcacc agttgacggc tggactgctg ttgtcagagg cgcgttgacg 540
   aagactcttg gggaggtttc cgacacagag ataaaaacat acgtcgattc ccgaaaggca 600
   agggaaaact atggaatgat ttgttcgact agattcattg ataaagtgca tgatgcaaag 660
   aagaagtaag tggcgactgc ctgctcaatg tgttccagca tccgtcttcc atttcttcgt 720
   cccagtggcc taataattcc aggtactgga atgccaaaga aggaaagttc tatattgatg 780
   ttatgcattg gtttgtttcc aaggtatgga ccgatattcg aacacacttc tggtctttga 840
   taacaagttc agggggacga tatcgaagaa gcgaaggcca ttaagacgaa ctggtctcag 900
   cataagcttg ccaaggacgg cacattcgac tcaatccgcg tcaatctcta caggctcgat 960
   actcctatgg gtgagaagcc accgttgtac ttcaatcgcc gtgagtactc tcgtgtagct 1020
   gattatggat gatgattatg agttattgac atttcgttct tagacgtgaa gcaacatgcc 1080
   aagttgaatc caatccttaa tcagattgaa aagaatcgca tcccaatctg ccatggtgca 1140
   gataacgagc tctactacac gatcggattt caaatccatg cggtgtacta ttccgctcac 1200
   tgtgaataca tgttctggta tgaaggctgc aatcatggaa gcgtcaaagc cgaatacgtt 1260
   tgaccgttaa cctttatgag ctttcctttg gacattatcc taccaccata tctcagtgac 1320
   aaaaactagt caatcgtttg ctaaggagct tagaagtagg acgaaaagaa gaatggttac 1380
   ccattgaagg acgaacactt gcgttaggcg ttgtcgacat tctgatctat tttagcagcg 1440
   agaaatctag ataaccattg agtcaattct ccaactaata tgctgtctaa tatgtatcta 1500
   aagttaaaga aaatctacaa aaagacctgc ttgatgataa aatgacgttc gatgcatcca 1560
   taccaattgt agccattgtc ccgtgtatca cgtgatccag gcaccgaagc accagccaca 1620
   gattgcatgc gccacaggcc atttgccgcg ccgaacaaat ccacaacgga tcttgcatct 1680
   ttcgctgaaa tccagaaatt cagcaacaat cggcgacatc atcaaccccc ccccatcaca 1740
   cgcacaatgt cgaagcttgg aaagtaagcg gaaactcccc acccccaaaa aaccagggcc 1800
   acgaagcgac aaaaaagaag aaaaagaaag aaagaaaatc tgaccaggac ctttttccac 1860
   tttccagccg cgccgactgg gccgacgacg aggagttcga cgacccctcc gctctccccc 1920
   cgcagcaaat cacgaccaac aaagatggca cgaagacgat cgtctcctac cgattcaacg 1980
   acgaaggcaa gaaggtgaag gtgacccgcc ggatcaaaac gaccgttgtg cgcgaacatg 2040
   tcaacccgca ggtcgcggag cggaggtcct gggccaagtt cggtctggag aagggcaacg 2100
   cgcccggacc ttcgttcgat acgacctccg tgggtgagaa cattgttttc cggcccagcg 2160
   tcaactggaa gcttcaggcg gctgaggcgg agaagaacgg tggcgagaag ggcagtgtga 2220
   aggatcagct gaaggacaag aaggtcaagt gtcgtatttg cagtggcgag cactttactg 2280
   ctcgctgtcc cttcaaggat actatggctc ctgtcgacga gcccactgct ggtggagagg 2340
   ctggtgatga ggattctccg gctgctggcg ctttgggtgc tggtacttct agctacgtgc 2400
   cccctcatct gcggaagggt gctgctggtg gcggagagag aatggctggc aagtatgaga 2460
   aggatgattt ggcgactctg agagttacga acgtgagttt tcccatcccc tccttgtttt 2520
   gatttactgt tctgtgtttg gctatttgct ggtctccccg gaggggttgg gggaggggtc 2580
   gagggacagg ggatgctagt tttcctcttt ctgtctcttt ctctcctcct ttcccttgcc 2640
   tgctctgccc tccctttgcc ctctaaacac tcctattggt gtcgttcaga gggagaaaga 2700
   aagaggcagc agccctttcg ggcagacaca tggatgtcat gagaacagca atcagcttcc 2760
   tctaatttcc ttgcttctat tgcatttgct tcccacggac acatcactga ctgacaataa 2820
   acccaaacag gtgagcgagt tggcagagga aggagaactg cgggatctgt tcgaacgctt 2880
   cggtcgtgtc accagagtct tccttgccag agacagagaa acccagagag ccaagggctt 2940
   cgctttcatc agctttgcgg atcggagcga tgctgcacgt gcttgcgaga agatggatgg 3000
   ctgtaagtta tccctcccgg ttacgtttcc atttttcgtc atattctgtt ttgttttttt 3060
   ccgccatcgt tgcaatgtca gcagcaaagc aagttatcgg aatcgcaact aacatttcct 3120
   cttcatctag tcggttaccg tcaccttatc ctgcgcgtcg aattcgccaa gcgtgccact 3180
   tagatttctc caatttccaa ttcccatttt ttcttcccat tttattctcc tccttattaa 3240
   cttcttcttt tgcttcttgg ttttccgtac gtatcatgac atgacaccct tgggtctctt 3300
   ttcgtctggc aggcgggttc tccatccatc cacactttcc atggctgaag aagatcttca 3360
   tctcatctat tgactacttt tctttgtact gtatgatccg atcgataatg atgattactg 3420
   aagaaaaaaa atgggggagg acatcccaat gtgtgtgcgt gtgtcgtctt ctctttgctt 3480
   tttgggtcga tttgatacga agaaaagcag cgtggtctat ttgtcttatt cacgcactac 3540
   tcttttccgg ttctatgtgt ctatttatca tgctgagttg caagtcaagg caaggcaagg 3600
   caagcttgct tgcaagccct cttttccttg tttacttttc cgtgcattga tcgatcgcgg 3660
   gtggaagtat acttagatcg agggagaagg ttcaacccca aaaaacaaaa gtatttcaat     3720
   ttgctcgttc gagtcacact acatggagta gagtagttag gtaaaggttc ggtcctgctt     3780
   acttgggaac ctagtagtgc tgtccaggtt ctaggacggg gaggaacagg ttttattggc     3840
   tattggggta atgctccgta ttatcgtccg ttcagtactg acagcctggc tggggtctac     3900
   ttttcaatca tatggtggat gttgtagagc ctcttgcttg gtatatctcg ggtaattggt     3960
   tattagatcc ttgctaagat gactcgatgc gtattagctg acaagctcct ctgatttggc     4020
   ggcatgtgtc aggaaggcct ggatttgagc cacccgggac gcgacaaggc agcattgact     4080
   gtgctgatca ggctttgcag agacgagatg catggtggat aatgaccatg ggtctccaga     4140
   gtgcggacca acgagccaag atcacggtca gtgtgtaggt ggttcggggg tcaacagcta     4200
   gctgtctagg gacgtcaggg atgccgggac actgatcatg acaataccga agggtgcctg     4260
   tgtagtcgtt tggtaaagat ggaaggctgg ctagtagcgt ttttgtatga gcttcgtcat     4320
   gagtgtcatc ggcaaggcag ctctgcatgt gtgtctggct agggaagaaa atcggcatag     4380
   caagcgggaa cgacgcattg ctcgagtaga cgactcaacc ttgtatggag agcatgacca     4440
   gatgtgagat cgatatcgag ccgcccatga cctccgggct agaggacggc ttctcctagc     4500
   atacagatgg atgcacggga ctaatcagag cgtggcgcca ggccagtggt ttcgatctcc     4560
   agtcaaaatc aatttggctt cgtgtaatcc tcgttcacag cccggggctt tcgctgctcg     4620
   gaaggattgt gaatggttcc gtgtgtccag atggctggtt acattgcatg aaaacgggta     4680
<210> 30
   <211> 1437
   <212> DNA
   <213> Aspergillus niger
<400> 30
   atgtcgaagc ttggaaagta agcggaaact ccccaccccc aaaaaaccag ggccacgaag      60
   cgacaaaaaa gaagaaaaag aaagaaagaa aatctgacca ggaccttttt ccactttcca     120
   gccgcgccga ctgggccgac gacgaggagt tcgacgaccc ctccgctctc cccccgcagc     180
   aaatcacgac caacaaagat ggcacgaaga cgatcgtctc ctaccgattc aacgacgaag     240
   gcaagaaggt gaaggtgacc cgccggatca aaacgaccgt tgtgcgcgaa catgtcaacc     300
   cgcaggtcgc ggagcggagg tcctgggcca agttcggtct ggagaagggc aacgcgcccg     360
   gaccttcgtt cgatacgacc tccgtgggtg agaacattgt tttccggccc agcgtcaact     420
   ggaagcttca ggcggctgag gcggagaaga acggtggcga gaagggcagt gtgaaggatc     480
   agctgaagga caagaaggtc aagtgtcgta tttgcagtgg cgagcacttt actgctcgct     540
   gtcccttcaa ggatactatg gctcctgtcg acgagcccac tgctggtgga gaggctggtg     600
   atgaggattc tccggctgct ggcgctttgg gtgctggtac ttctagctac gtgccccctc     660
   atctgcggaa gggtgctgct ggtggcggag agagaatggc tggcaagtat gagaaggatg     720
   atttggcgac tctgagagtt acgaacgtga gttttcccat cccctccttg ttttgattta     780
   ctgttctgtg tttggctatt tgctggtctc cccggagggg ttgggggagg ggtcgaggga     840
   caggggatgc tagttttcct ctttctgtct ctttctctcc tcctttccct tgcctgctct     900
   gccctccctt tgccctctaa acactcctat tggtgtcgtt cagagggaga aagaaagagg     960
   cagcagccct ttcgggcaga cacatggatg tcatgagaac agcaatcagc ttcctctaat    1020
   ttccttgctt ctattgcatt tgcttcccac ggacacatca ctgactgaca ataaacccaa    1080
   acaggtgagc gagttggcag aggaaggaga actgcgggat ctgttcgaac gcttcggtcg    1140
   tgtcaccaga gtcttccttg ccagagacag agaaacccag agagccaagg gcttcgcttt    1200
   catcagcttt gcggatcgga gcgatgctgc acgtgcttgc gagaagatgg atggctgtaa    1260
   gttatccctc ccggttacgt ttccattttt cgtcatattc tgttttgttt ttttccgcca    1320
   tcgttgcaat gtcagcagca aagcaagtta tcggaatcgc aactaacatt tcctcttcat    1380
   ctagtcggtt accgtcacct tatcctgcgc gtcgaattcg ccaagcgtgc cacttag       1437
<210> 31
   <211> 867
   <212> DNA
   <213> Aspergillus niger
<400> 31
   atgtcgaagc ttggaaaccg cgccgactgg gccgacgacg aggagttcga cgacccctcc      60
   gctctccccc cgcagcaaat cacgaccaac aaagatggca cgaagacgat cgtctcctac     120
   cgattcaacg acgaaggcaa gaaggtgaag gtgacccgcc ggatcaaaac gaccgttgtg     180
   cgcgaacatg tcaacccgca ggtcgcggag cggaggtcct gggccaagtt cggtctggag     240
   aagggcaacg cgcccggacc ttcgttcgat acgacctccg tgggtgagaa cattgttttc     300
   cggcccagcg tcaactggaa gcttcaggcg gctgaggcgg agaagaacgg tggcgagaag     360
   ggcagtgtga aggatcagct gaaggacaag aaggtcaagt gtcgtatttg cagtggcgag     420
   cactttactg ctcgctgtcc cttcaaggat actatggctc ctgtcgacga gcccactgct     480
   ggtggagagg ctggtgatga ggattctccg gctgctggcg ctttgggtgc tggtacttct     540
   agctacgtgc cccctcatct gcggaagggt gctgctggtg gcggagagag aatggctggc     600
   aagtatgaga aggatgattt ggcgactctg agagttacga acgtgagcga gttggcagag     660
   gaaggagaac tgcgggatct gttcgaacgc ttcggtcgtg tcaccagagt cttccttgcc     720
   agagacagag aaacccagag agccaagggc ttcgctttca tcagctttgc ggatcggagc     780
   gatgctgcac gtgcttgcga gaagatggat ggcttcggtt accgtcacct tatcctgcgc     840
   gtcgaattcg ccaagcgtgc cacttag                                         867
<210> 32
   <211> 288
   <212> PRT
   <213> Aspergillus niger
<400> 32
   Met Ser Lys Leu Gly Asn Arg Ala Asp Trp Ala Asp Asp Glu Glu Phe
   1               5                   10                  15
   Asp Asp Pro Ser Ala Leu Pro Pro Gln Gln Ile Thr Thr Asn Lys Asp
               20                  25                  30
   Gly Thr Lys Thr Ile Val Ser Tyr Arg Phe Asn Asp Glu Gly Lys Lys
           35                  40                  45
   Val Lys Val Thr Arg Arg Ile Lys Thr Thr Val Val Arg Glu His Val
       50                  55                  60
   Asn Pro Gln Val Ala Glu Arg Arg Ser Trp Ala Lys Phe Gly Leu Glu
   65                  70                  75                  80
   Lys Gly Asn Ala Pro Gly Pro Ser Phe Asp Thr Thr Ser Val Gly Glu
                   85                  90                  95
   Asn Ile Val Phe Arg Pro Ser Val Asn Trp Lys Leu Gln Ala Ala Glu
               100                 105                 110
   Ala Glu Lys Asn Gly Gly Glu Lys Gly Ser Val Lys Asp Gln Leu Lys
           115                 120                 125
   Asp Lys Lys Val Lys Cys Arg Ile Cys Ser Gly Glu His Phe Thr Ala
       130                 135                 140
   Arg Cys Pro Phe Lys Asp Thr Met Ala Pro Val Asp Glu Pro Thr Ala
   145                 150                 155                 160
   Gly Gly Glu Ala Gly Asp Glu Asp Ser Pro Ala Ala Gly Ala Leu Gly
                   165                 170                 175
   Ala Gly Thr Ser Ser Tyr Val Pro Pro His Leu Arg Lys Gly Ala Ala
               180                 185                 190
   Gly Gly Gly Glu Arg Met Ala Gly Lys Tyr Glu Lys Asp Asp Leu Ala
           195                 200                 205
   Thr Leu Arg Val Thr Asn Val Ser Glu Leu Ala Glu Glu Gly Glu Leu
       210                 215                 220
   Arg Asp Leu Phe Glu Arg Phe Gly Arg Val Thr Arg Val Phe Leu Ala
   225                 230                 235                 240
   Arg Asp Arg Glu Thr Gln Arg Ala Lys Gly Phe Ala Phe Ile Ser Phe
                   245                 250                 255
   Ala Asp Arg Ser Asp Ala Ala Arg Ala Cys Glu Lys Met Asp Gly Phe
               260                 265                 270
   Gly Tyr Arg His Leu Ile Leu Arg Val Glu Phe Ala Lys Arg Ala Thr
           275                 280                 285
<210> 33
   <211> 6182
   <212> DNA
   <213> Aspergillus niger
<400> 33
   cggccgcggg ggcactgtcg atcttatcac attcacaatc atcgagctat cgcctaatat 60
   gcgtctaaag gaggaagcac ccggtactgg atctctatgt ggaagcacct ttgtcaacag 120
   acgctttgaa gagatgctaa atgaccgcct ctcctccctc cctggctggg acagggatac 180
   actggatgaa gcaatgcatc gatttgaaac tgtcgctaaa agaactttca gtggaaatac 240
   agacgattac ttcatgttcc ctgttccagg tatagcagac agccaggaag tcggggttcg 300
   tcgcggccga ttccgagtta ctggccaaga gatgcagcaa ctgtttttgc ctatcctacg 360
   agacattgag gaccttgtcc gagagcagat cgagacctct gacgctcagg taaaagcaat 420
   tttcctggtt ggagggtttg gacagagccc ataccttcgc acatatcttc gcgactgctt 480
   ctctcctgaa gtcgaagtga tagcaccagt tgacggctgg actgctgttg tcagaggcgc 540
   gttgacgaag actcttgggg aggtttccga cacagagata aaaacatacg tcgattcccg 600
   aaaggcaagg gaaaactatg gaatgatttg ttcgactaga ttcattgata aagtgcatga 660
   tgcaaagaag aagtaagtgg cgactgcctg ctcaatgtgt tccagcatcc gtcttccatt 720
   tcttcgtccc agtggcctaa taattccagg tactggaatg ccaaagaagg aaagttctat 780
   attgatgtta tgcattggtt tgtttccaag gtatggaccg atattcgaac acacttctgg 840
   tctttgataa caagttcagg gggacgatat cgaagaagcg aaggccatta agacgaactg 900
   gtctcagcat aagcttgcca aggacggcac attcgactca atccgcgtca atctctacag 960
   gctcgatact cctatgggtg agaagccacc gttgtacttc aatcgccgtg agtactctcg 1020
   tgtagctgat tatggatgat gattatgagt tattgacatt tcgttcttag acgtgaagca 1080
   acatgccaag ttgaatccaa tccttaatca gattgaaaag aatcgcatcc caatctgcca 1140
   tggtgcagat aacgagctct actacacgat cggatttcaa atccatgcgg tgtactattc 1200
   cgctcactgt gaatacatgt tctggtatga aggctgcaat catggaagcg tcaaagccga 1260
   atacgtttga ccgttaacct ttatgagctt tcctttggac attatcctac caccatatct 1320
   cagtgacaaa aactagtcaa tcgtttgcta aggagcttag aagtaggacg aaaagaagaa 1380
   tggttaccca ttgaaggacg aacacttgcg ttaggcgttg tcgacattct gatctatttt 1440
   agcagcgaga aatctagata accattgagt caattctcca actaatatgc tgtctaatat 1500
   gtatctaggc gcgccctcgc acgcatgggt tgagtggtat ggggccatcc agagtcacct 1560
   gtggcagcat gagactgcac tcgaagcagc catcaaccca gccaatattc tgggctttcc 1620
   atccttagat cacatttgag atataaccca tttggtgaga gacacttgtg ccgttatacg 1680
   tgtctagact ggaaacgcaa ccctgaaggg attcttcctt tgagagatgg aagcgtgtca 1740
   tatctcttcg gttctacggc aggttttttt ctgctctttc gtagcatggc atggtcactt 1800
   cagcgcttat ttacagttgc tggtattgat ttcttgtgca aattgctatc tgacacttat 1860
   tagctatgga gtcaccacat ttcccagcaa cttccccact tcctctgcaa tcgccaacgt 1920
   cctctcttca ctgagtctcc gtccgataac ctgcactgca accggtgccc catggtacgc 1980
   ctccggatca tactcttcct gcacgagggc atcaagctca ctaaccgcct tgaaactctc 2040
   attcttctta tcgatgttct tatccgcaaa ggtaaccgga acaaccacgc tcgtgaaatc 2100
   cagcaggttg atcacagagg catacccata gtaccggaac tggtcatgcc gtaccgcagc 2160
   ggtaggcgta atcggcgcga tgatggcgtc cagttccttc ccggcctttt cttcagcctc 2220
   ccgccatttc tcaaggtact ccatctggta attccacttc tggagatgcg tgtcccagag 2280
   ctcgttcatg ttaacagctt tgatgttcgg gttcagtagg tctttgatat ttggaatcgc 2340
   cggctcgctg gatgcactga tatcgcgcat tacgtcggcg ctgccgtcag ccgcgtagat 2400
   atgggagatg agatcgtggc cgaaatcgtg cttgtatggc gtccacgagg tcacggtgtg 2460
   accggctttg gcgagtgcgg cgacggtggt ttccacgccg cgcaggatag gagggtgtgg 2520
   aaggacattg ccgtcgaagt tgtagtagcc gatattgagc ccgccgttct tgatcttgga 2580
   ggcaataatg tccgactcgg actggcgcca gggcatgggg atgaccttgg agtcgtattt 2640
   ccatggctcc tgaccgagga cggatttggt gaagaggcgg aggtctaaca tacttcatca 2700
   gtgactgccg gtctcgtata tagtataaaa agcaagaaag gaggacagtg gaggcctggt 2760
   atagagcagg aaaagaagga agaggcgaag gactcaccct caacagagtg cgtaatcggc 2820
   ccgacaacgc tgtgcaccgt ctcctgaccc tccatgctgt tcgccatctt tgcatacggc 2880
   agccgcccat gactcggcct tagaccgtac aggaagttga acgcggccgg cactcgaatc 2940
   gagccaccga tatccgttcc tacaccgatg acgccaccac gaatcccaac gatcgcaccc 3000
   tcaccaccag aactgccgcc gcacgaccag ttcttgttgc gtgggttgac ggtgcgcccg 3060
   atgatgttgt tgactgtctc gcagaccatc agggtctgcg ggacagaggt cttgacgtag 3120
   aagacggcac cggctttgcg gagcatggtt gtcagaaccg agtccccttc gtcgtacttg 3180
   tttagccatg agatgtagcc cattgatgtt tcgtagccct ggtggcatat gttagctgac 3240
   aaaaagggac atctaacgac ttaggggcaa cggtgtacct tgactcgaag ctggtctttg 3300
   agagagatgg ggaggccatg gagtggacca acgggtctct tgtgctttgc gtagtattca 3360
   tcgagttccc ttgcctgcgc gagagcggcg tcagggaaga actcgtgggc gcagtttgtc 3420
   tgcacagaag ccagcgtcag cttgatagtc ccataaggtg gcgttgttac atctccctga 3480
   gaggtagagg ggaccctact aactgctggg cgattgctgc ccgtttacag aatgctagcg 3540
   taacttccac cgaggtcaac tctccggccg ccagcttgga cacaagatct gcagcggagg 3600
   cctctgtgat cttcagttcg gcctctgaaa ggatccccga tttctttggg aaatcaataa 3660
   cgctgtcttc cgcaggcagc gtctggactt tccattcatc agggatggtt tttgcgaggc 3720
   gggcgcgctt atcagcggcc agttcttccc aggattgagg cattgtgatg tctgctcaag 3780
   cggggtagct gttagtcaag ctgcgatgaa gtgggaaagc tcgaactgaa aggttcaaag 3840
   gaataaggga tgggaaggat ggagtatgga tgtagcaaag tacttactta ggggaaataa 3900
   aggttcttgg atgggaagat gaatatactg aagatgggaa aagaaagaga aaagaaaaga 3960
   gcagctggtg gggagagcag gaaaatatgg caacaaatgt tggactgacg caacgacctt 4020
   gtcaaccccg ccgacacacc gggcggacag acggggcaaa gctgcctacc agggactgag 4080
   ggacctcagc aggtcgagtg cagagcaccg gatgggtcga ctgccagctt gtgttcccgg 4140
   tctgcgccgc tggccagctc ctgagcggcc tttccggttt catacaccgg gcaaagcagg 4200
   agaggcacga tatttggacg ccctacagat gccggatggg ccaattaggg agcttacgcg 4260
   ccgggtactc gctctaccta cttcggagaa ggtactatct cgtgaatctt ttaccagatc 4320
   ggaagcaatt ggacttctgt acctaggtta atggcatgct atttcgccga cggctataca 4380
   cccctggctt cacattctcc ttcgcttact gccggtgatt cgatgaagct ccatattctc 4440
   cgatgatgca atagattctt ggtcaacgag ggcacaccca gcctttccac ttcggggcgg 4500
   aggggcggcc ggtcccggat taataatcat ccactgcacc tcagagccgc cagagctgtc 4560
   tggcgcagtg gcgcttatta ctcagccctt ctctctgcgt ccgtccgtct ctccgcatgc 4620
   cagaaagagt caccggtcac tgtacagagc tcgaattgga cgctgaggcc ggcctagatt 4680
   tctccaattt ccaattccca ttttttcttc ccattttatt ctcctcctta ttaacttctt 4740
   cttttgcttc ttggttttcc gtacgtatca tgacatgaca cccttgggtc tcttttcgtc 4800
   tggcaggcgg gttctccatc catccacact ttccatggct gaagaagatc ttcatctcat 4860
   ctattgacta cttttctttg tactgtatga tccgatcgat aatgatgatt actgaagaaa 4920
   aaaaatgggg gaggacatcc caatgtgtgt gcgtgtgtcg tcttctcttt gctttttggg 4980
   tcgatttgat acgaagaaaa gcagcgtggt ctatttgtct tattcacgca ctactctttt 5040
   ccggttctat gtgtctattt atcatgctga gttgcaagtc aaggcaaggc aaggcaagct 5100
   tgcttgcaag ccctcttttc cttgtttact tttccgtgca ttgatcgatc gcgggtggaa 5160
   gtatacttag atcgagggag aaggttcaac cccaaaaaac aaaagtattt caatttgctc 5220
   gttcgagtca cactacatgg agtagagtag ttaggtaaag gttcggtcct gcttacttgg 5280
   gaacctagta gtgctgtcca ggttctagga cggggaggaa caggttttat tggctattgg 5340
   ggtaatgctc cgtattatcg tccgttcagt actgacagcc tggctggggt ctacttttca 5400
   atcatatggt ggatgttgta gagcctcttg cttggtatat ctcgggtaat tggttattag 5460
   atccttgcta agatgactcg atgcgtatta gctgacaagc tcctctgatt tggcggcatg 5520
   tgtcaggaag gcctggattt gagccacccg ggacgcgaca aggcagcatt gactgtgctg 5580
   atcaggcttt gcagagacga gatgcatggt ggataatgac catgggtctc cagagtgcgg 5640
   accaacgagc caagatcacg gtcagtgtgt aggtggttcg ggggtcaaca gctagctgtc 5700
   tagggacgtc agggatgccg ggacactgat catgacaata ccgaagggtg cctgtgtagt 5760
   cgtttggtaa agatggaagg ctggctagta gcgtttttgt atgagcttcg tcatgagtgt 5820
   catcggcaag gcagctctgc atgtgtgtct ggctagggaa gaaaatcggc atagcaagcg 5880
   ggaacgacgc attgctcgag tagacgactc aaccttgtat ggagagcatg accagatgtg 5940
   agatcgatat cgagccgccc atgacctccg ggctagagga cggcttctcc tagcatacag 6000
   atggatgcac gggactaatc agagcgtggc gccaggccag tggtttcgat ctccagtcaa 6060
   aatcaatttg gcttcgtgta atcctcgttc acagcccggg gctttcgctg ctcggaagga 6120
   ttgtgaatgg ttccgtgtgt ccagatggct ggttacattg catgaaaacg ggtagcggcc 6180
   gc 6182
<210> 34
   <211> 714
   <212> DNA
   <213> Discosoma sp.
<400> 34
   atggcctcct ccgaggacgt catcaaggag ttcatgcgct tcaaggtgcg catggagggc        60
   tccgtgaacg gccacgagtt cgagatcgag ggcgagggcg agggccgccc ctacgagggc       120
   acccagaccg ccaagctgaa ggtgaccaag ggcggccccc tgcccttcgc ctgggacatc       180
   ctgtcccccc agttccagta cggctccaag gtgtacgtga agcaccccgc cgacatcccc       240
   gactacaaga agctgtcctt ccccgagggc ttcaagtggg agcgcgtgat gaacttcgag       300
   gacggcggcg tggtgaccgt gacccaggac tcctccctgc aggacggctc cttcatctac       360
   aaggtgaagt tcatcggcgt gaacttcccc tccgacggcc ccgtaatgca gaagaagact       420
   atgggctggg aggcctccac cgagcgcctg tacccccgcg acggcgtgct gaagggcgag       480
   atccacaagg ccctgaagct gaaggacggc ggccactacc tggtggagtt caagtccatc       540
   tacatggcca agaagcccgt gcagctgccc ggctactact acgtggactc caagctggac       600
   atcacctccc acaacgagga ctacaccatc gtggagcagt acgagcgcgc cgagggccgc       660
   caccacctgt tcctgactca cggtatggac gaattgtaca agtcgaagct gtaa             714
<210> 35
   <211> 237
   <212> PRT
   <213> Discosoma sp.
<400> 35
   Met Ala Ser Ser Glu Asp Val Ile Lys Glu Phe Met Arg Phe Lys Val
   1               5                   10                  15
   Arg Met Glu Gly Ser Val Asn Gly His Glu Phe Glu Ile Glu Gly Glu
               20                  25                  30
   Gly Glu Gly Arg Pro Tyr Glu Gly Thr Gln Thr Ala Lys Leu Lys Val
           35                  40                  45
   Thr Lys Gly Gly Pro Leu Pro Phe Ala Trp Asp Ile Leu Ser Pro Gln
       50                  55                  60
   Phe Gln Tyr Gly Ser Lys Val Tyr Val Lys His Pro Ala Asp Ile Pro
   65                  70                  75                  80
   Asp Tyr Lys Lys Leu Ser Phe Pro Glu Gly Phe Lys Trp Glu Arg Val
                   85                  90                  95
   Met Asn Phe Glu Asp Gly Gly Val Val Thr Val Thr Gln Asp Ser Ser
               100                 105                 110
   Leu Gln Asp Gly Ser Phe Ile Tyr Lys Val Lys Phe Ile Gly Val Asn
           115                 120                 125
   Phe Pro Ser Asp Gly Pro Val Met Gln Lys Lys Thr Met Gly Trp Glu
       130                 135                 140
   Ala Ser Thr Glu Arg Leu Tyr Pro Arg Asp Gly Val Leu Lys Gly Glu
   145                 150                 155                 160
   Ile His Lys Ala Leu Lys Leu Lys Asp Gly Gly His Tyr Leu Val Glu
                   165                 170                 175
   Phe Lys Ser Ile Tyr Met Ala Lys Lys Pro Val Gln Leu Pro Gly Tyr
               180                 185                 190
   Tyr Tyr Val Asp Ser Lys Leu Asp Ile Thr Ser His Asn Glu Asp Tyr
           195                 200                 205
   Thr Ile Val Glu Gln Tyr Glu Arg Ala Glu Gly Arg His His Leu Phe
       210                 215                 220
   Leu Thr His Gly Met Asp Glu Leu Tyr Lys Ser Lys Leu
   225                 230                 235
<210> 36
   <211> 2968
   <212> DNA
   <213> Discosoma sp.
<400> 36
   gagttataga cggtccggca taggtaagga gagattagat atttcgactt cccgattcct      60
   tagtctcctt agtctggaaa ctgagaaagg cctgaactcg ttcaatctat tagctctaat     120
   cacgtagtct caactagaat cctgaacagg cgccggaacc agtccaaatg cgtggagtac     180
   agcaagtcca gtcaaatcac gattagaatg gccatatata agcaaaaaac ccttggacaa     240
   tacgtccgat cgagaccaaa gcaatctggt acatcaccct tcttatatac agggtagccg     300
   gaattgtatt gactctatac gaagccgcag gtttaccagc acagaggtgc gatagctaca     360
   cgaatgtaga gtgaaggcat tacaaccgat tgctaagtgt catagaggct aagcttcacg     420
   gctcaaggag tctgtggagg agacctgata taagaattga gagttatact ccggaggtta     480
   tgttagtacc tcaaaaatgt gaagtacaac gaaactcgaa cttcgaatga caatagcttg     540
   gctggctgtt agtcaactga ggtacaacgt catcttgcaa ttagttatgt tctttgctag     600
   atagtgagct gctgtaaact tgtagttctt agaaacatgt ctgaagtcta gagtcgagac     660
   ttgtgattgg tgtaaattag tgggggtccg gagcggaata acaatcgagg tcagcctcct     720
   gggtctgtca gtaagctaac cacgaaagga caagcgtaga cgaggtcacg gggtactggc     780
   ctagccggct tcggctagcg cccacgaaaa tagggcaacg tcgaagcgag agacacacac     840
   ggattccacg atcgccgacg acaagcactc aactccacga caacccacga caacccttgc     900
   cgctgcgttc ttggtgaagg gctgcaggat tagaggtgag tattttattt tctggctatt     960
   gtgatctttt tcccccccgg tttccgctgg ttctatcttg tcgagcgagt cggtgcgcct    1020
   tcgatcggtc tgtcaaattt ccatccgtcc actcgttcac ccggtatcga tacaagaggg    1080
   aatggagcat tacgacaggg gagcagaggc agaactctcg atgttgctac tttttttgct    1140
   tggaacaact tccttgaaag attcttttct gaatgcggct gggaaactgc gaattggata    1200
   tccagcgaaa ggttaccgac agactcacgg ccatcgatac cactcagcgg gatagaattg    1260
   aaggcattcg gacattagat gatcagcttc gcttgtttcg actcgtgcta atcaattatc 1320
   cctggacagt cccccacata gacagcaaac ggatccatgg cctcctccga ggacgtcatc 1380
   aaggagttca tgcgcttcaa ggtgcgcatg gagggctccg tgaacggcca cgagttcgag 1440
   atcgagggcg agggcgaggg ccgcccctac gagggcaccc agaccgccaa gctgaaggtg 1500
   accaagggcg gccccctgcc cttcgcctgg gacatcctgt ccccccagtt ccagtacggc 1560
   tccaaggtgt acgtgaagca ccccgccgac atccccgact acaagaagct gtccttcccc 1620
   gagggcttca agtgggagcg cgtgatgaac ttcgaggacg gcggcgtggt gaccgtgacc 1680
   caggactcct ccctgcagga cggctccttc atctacaagg tgaagttcat cggcgtgaac 1740
   ttcccctccg acggccccgt aatgcagaag aagactatgg gctgggaggc ctccaccgag 1800
   cgcctgtacc cccgcgacgg cgtgctgaag ggcgagatcc acaaggccct gaagctgaag 1860
   gacggcggcc actacctggt ggagttcaag tccatctaca tggccaagaa gcccgtgcag 1920
   ctgcccggct actactacgt ggactccaag ctggacatca cctcccacaa cgaggactac 1980
   accatcgtgg agcagtacga gcgcgccgag ggccgccacc acctgttcct gactcacggt 2040
   atggacgaat tgtacaagtc gaagctgtaa gtcgactcta gaggatcgat ccccggggtg 2100
   cttctaaggt atgagtcgca aaattgtttt ttatttttgg tcttgagtct aatatgctcg 2160
   cagctcttgc gttgtatatg gtcgttgtcg cgtattttct gttgtattaa aagatcaaac 2220
   gagatcaagg gatggctcgc gggctgtctc tcgcactagg aggaagaatg cctgaaaaag 2280
   gaactttgat tttagctgtg gaatagagat ggcttgtttg aggacgcttg tcgcttggcg 2340
   cagggacttg aatggcagct tgtggaaacc gaaggcgaga aaagtcgacg gatactgtac 2400
   gtggttctat tgccagtgcg gtggaagctt ccttctcata tagttcaatc cttctttgaa 2460
   tctgtttgtt tcatatttgg actgtttcat tctctgcttg cgcattctca tcttcgagaa 2520
   cgactgcagg gattgttggt tctgtggagc tgatgagcgc gccttgacca cccttgttct 2580
   tgttttgctc ttttgttctc atttaacccg tttctccctt ccaacccttt gaccttgcaa 2640
   cattgtctcc cagcgcgttg ccaaagcgaa cttgatatca gtatagtatg accaagtagt 2700
   ctaccaaaat aaattttagt acagtattgc tagttataca agataaattt tgtacaacaa 2760
   tgattatgca gagctggtat tctgtaagga gtaaattcat gcaggtacag agcgtccacg 2820
   tcaggcacac gcgctttagt gctgggtaca cattatgcta cctttggggc aaggtgctat 2880
   tctaggtagg gtatgcctag caatgccatg atcttatgac ctcagtaatc tgaccttgga 2940
   tttaagatcg gagttctcaa accctgcg 2968
<210> 37
   <211> 3009
   <212> DNA
   <213> Penicillium chrysogenum
<400> 37
   gaggaagacg tgatcagagt aagccctatg ccaacagaag gggaggatgg gatcagagta     60
   agacccatgt cagattctgc aaacgaagga cggccgaaat tacttgccgc ccccccccgg     120
   tgtatgtggc ctaagagaag acacagtgtc ttagacaatc atattcccag ctcgtccatc    180
   atctcatcga catccttgat tggagcatac ataccggaat ccaatcagtg aattatgaat    240
   gacacaacca ctcagtttcc cacgattcat ggtgtcggca atcatctccc gggcgcaatc    300
   tcgccatggg atactggatg gagagataac cgagattcgg ccggcttcac ttcaacttgc    360
   atggctctga tgcaagatgt gtgcggatca gatctcctag ccaaaggccc gcacattcaa    420
   cgtcgtgact ggcgtggcgc gaaggctatc aaccttgttg accagatccc ctcttgtcat    480
   cgagaccggc agagaaggga gcgtctcttg tcgaacaatc cggtcatggc attgagagcc    540
   agaagacata ggggttgaat ggcccctaag ttgaccaaaa atgtgagtgt atcgatgttt    600
   cagacagcaa tgccgattcg tcaagctgaa gaacccttgg aagcttcaag gacctgtcag    660
   gttgcatctc tctcggacgt gacccagtct cagtcatcgt tagaatagga agaagttcag    720
   ccctcaggtc aaggatggtt aatgcagtca ggtctcgttg gcctaaatga tagttatgta    780
   cgccacggta ttcttgtagc tggccttgaa gcttcagaag actgcccacg aaattagcag    840
   tatcaagttc ggcgcccgga cttcccattt gggttctgtg ataatttctt tgatacgaca    900
   gtcgttagga gccgattttt tcttcctatt ctttttcccc tgctgtgtag gttggatgtt    960
   ttcgtgaggg atgttggcct gaaagagttg aatgtcaaat caatcactat cgttcgactg   1020
   ttcacccatg atgtcctcca gctcgcccat ccggcgcgtc ttcgaaacgg tgtgcttctg   1080
   agcggccctc tgagaggccg atgtacttag aaacatctcc caataatctg gacctttaac   1140
   cgtgaatgtt tcacaacctt gatcagttta aatgataaat agtgacacaa ggtgaagtac   1200
   gagcccggag atcagggcga atgactactc tgcccgctat aagtattggg tttggtccga   1260
   aggaactgtg ttcggggcct ttaaagttaa atatttttga aagcagcgct tgcagagagt   1320
   gttaatattc ggaccccgtt gaatgtcatg tttatcattg ggcaaacagt ggagtggtgt   1380
   caatcttgta tcagtctagt agaaacgagg ttaaagaacc ttggaactac tttagatggg   1440
   gctatacaca ccgcgccacg tgattcccgc caccagccac aactgtgcca cagtaaatta   1500
   ttaaatccac aacggattcg cctttgaaag aaatccagaa attcagcagc cgtaataaca   1560
   ccatcactta caccatgtct ccaaccggga agtgagtatt tgcagttcca gtcctacaag   1620
   aaacacgcgc ctaacaaaac tttctatttt acagccgcgc cgactgggcc gacgacgaag   1680
   atttcgatga cccctccgtg ctcccgccgc aagaagtgat cacgaacaag gacggcacca   1740
   agaccgtcat ttcctaccgc tacaatgatg acaacaagaa agtgaaggtc acccgccgaa   1800
   ttaagaccac cgttgtccgc gaacatgtca acccccaagt cgccgaacgc cgcaaatggg 1860
   agaagttcgg tctcgagaag ggccacgccg ccggtccctc gttcgacacc acctccgtcg 1920
   gcgagaacat catcttccgc cccagcgtca actggaaggc taatgctaag gaggccgaga 1980
   aggagggtgg cgaaaagggc agcatgaagg accagttgaa ggacaagaag gtcaagtgtc 2040
   gtatttgcag tggagagcat ttcacggctc gctgtccctt caaggacacc atggctcccg 2100
   tcgaggaggg cactgctgct gctcctggtg tcgaggcaga ggaggatgca ggtggtcttg 2160
   gtgctggaaa gtccagctac gttcctcctc acatgcggaa gggcggtgct ggtggcggcg 2220
   agaagatggg cggtcgtttc gagaaggacg atttggcgac tctcagagtt acaaacgtat 2280
   gttctgccat tttctgcttt tctacgggct ctacgggcgt cagggtcgtt tattttggtt 2340
   cggtattgac ttttcaaaca ggtcagcgag ttggcagagg agaacgagtt gcgggatctc 2400
   ttcgagcgtt tcggtcgtgt caccagagtc ttccttgcac gggatcggga aacccagaga 2460
   gccaagggct ttgctttcat cagctatgcg gaccgtggcg acgcagcact tgcttgcgag 2520
   aaggtggatg gctgtatgtc ttcaataccc catttcgtgc tttcctttct accagttcct 2580
   caagtactaa cccgtctttc tgctagtcgg ttaccgccac cttattctcc gcgtcgagtt 2640
   cgccaagcgc actacttaaa cttctttatc ggttctctct tacgactttt tgaatggaac 2700
   gtttccttct tctcaggcgg gcctatcttt gggccgaagc tcttttcctt gtactgtagg 2760
   acctggttga taatgattcc caaaaagaca tccagcatgt cagttacttg cattcgtcag 2820
   tctatacaaa agcaatggtt tagagaaatt ttgaacttta tacatggttt tatttgttgc 2880
   ttcacggccg taccttctgg aaatccacgg taggagtgtc aatttgcgtt tttgataatc 2940
   cttccaaggt tcttctcgaa gtagttgttc tataattgct tcacagctac catggaacat 3000
   cccaagcac 3009
<210> 38
   <211> 1085
   <212> DNA
   <213> Penicillium chrysogenum
<400> 38
   atgtctccaa ccgggaagtg agtatttgca gttccagtcc tacaagaaac acgcgcctaa      60
   caaaactttc tattttacag ccgcgccgac tgggccgacg acgaagattt cgatgacccc     120
   tccgtgctcc cgccgcaaga agtgatcacg aacaaggacg gcaccaagac cgtcatttcc     180
   taccgctaca atgatgacaa caagaaagtg aaggtcaccc gccgaattaa gaccaccgtt     240
   gtccgcgaac atgtcaaccc ccaagtcgcc gaacgccgca aatgggagaa gttcggtctc     300
   gagaagggcc acgccgccgg tccctcgttc gacaccacct ccgtcggcga gaacatcatc     360
   ttccgcccca gcgtcaactg gaaggctaat gctaaggagg ccgagaagga gggtggcgaa     420
   aagggcagca tgaaggacca gttgaaggac aagaaggtca agtgtcgtat ttgcagtgga     480
   gagcatttca cggctcgctg tcccttcaag gacaccatgg ctcccgtcga ggagggcact     540
   gctgctgctc ctggtgtcga ggcagaggag gatgcaggtg gtcttggtgc tggaaagtcc     600
   agctacgttc ctcctcacat gcggaagggc ggtgctggtg gcggcgagaa gatgggcggt     660
   cgtttcgaga aggacgattt ggcgactctc agagttacaa acgtatgttc tgccattttt     720
   ctgctttcta cgggctctac gggcgtcagg gtcgtttatt ttggttcggt attgactttt     780
   caaacaggtc agcgagttgg cagaggagaa cgagttgcgg gatctcttcg agcgtttcgg     840
   tcgtgtcacc agagtcttcc ttgcacggga tcgggaaacc cagagagcca agggctttgc     900
   tttcatcagc tatgcggacc gtggcgacgc agcacttgct tgcgagaagg tggatggctg     960
   tatgtcttca ataccccatt tcgtgctttc ctttctacca gttcctcaag tactaacccg    1020
   tctttctgct agtcggttac cgccacctta ttctccgcgt cgagttcgcc aagcgcacta    1080
   cttaa                                                                1085
<210> 39
   <211> 864
   <212> DNA
   <213> Penicillium chrysogenum
<400> 39
   atgtctccaa ccgggaaccg cgccgactgg gccgacgacg aagatttcga tgacccctcc      60
   gtgctcccgc cgcaagaagt gatcacgaac aaggacggca ccaagaccgt catttcctac     120
   cgctacaatg atgacaacaa gaaagtgaag gtcacccgcc gaattaagac caccgttgtc     180
   cgcgaacatg tcaaccccca agtcgccgaa cgccgcaaat gggagaagtt cggtctcgag    240
   aagggccacg ccgccggtcc ctcgttcgac accacctccg tcggcgagaa catcatcttc     300
   cgccccagcg tcaactggaa ggctaatgct aaggaggccg agaaggaggg tggcgaaaag     360
   ggcagcatga aggaccagtt gaaggacaag aaggtcaagt gtcgtatttg cagtggagag     420
   catttcacgg ctcgctgtcc cttcaaggac accatggctc ccgtcgagga gggcactgct     480
   gctgctcctg gtgtcgaggc agaggaggat gcaggtggtc ttggtgctgg aaagtccagc     540
   tacgttcctc ctcacatgcg gaagggcggt gctggtggcg gcgagaagat gggcggtcgt     600
   ttcgagaagg acgatttggc gactctcaga gttacaaacg tcagcgagtt ggcagaggag     660
   aacgagttgc gggatctctt cgagcgtttc ggtcgtgtca ccagagtctt ccttgcacgg     720
   gatcgggaaa cccagagagc caagggcttt gctttcatca gctatgcgga ccgtggcgac     780
   gcagcacttg cttgcgagaa ggtggatggc ttcggttacc gccaccttat tctccgcgtc     840
   gagttcgcca agcgcactac ttaa                                            864
<210> 40
   <211> 287
   <212> PRT
   <213> Penicillium chrysogenum
<400> 40
   Met Ser Pro Thr Gly Asn Arg Ala Asp Trp Ala Asp Asp Glu Asp Phe
   1               5                   10                  15
   Asp Asp Pro Ser Val Leu Pro Pro Gln Glu Val Ile Thr Asn Lys Asp
               20                  25                  30
   Gly Thr Lys Thr Val Ile Ser Tyr Arg Tyr Asn Asp Asp Asn Lys Lys
           35                  40                  45
   Val Lys Val Thr Arg Arg Ile Lys Thr Thr Val Val Arg Glu His Val
       50                  55                  60
   Asn Pro Gln Val Ala Glu Arg Arg Lys Trp Glu Lys Phe Gly Leu Glu
   65                  70                  75                  80
   Lys Gly His Ala Ala Gly Pro Ser Phe Asp Thr Thr Ser Val Gly Glu
                   85                  90                  95
   Asn Ile Ile Phe Arg Pro Ser Val Asn Trp Lys Ala Asn Ala Lys Glu
               100                 105                 110
   Ala Glu Lys Glu Gly Gly Glu Lys Gly Ser Met Lys Asp Gln Leu Lys
           115                 120                 125
   Asp Lys Lys Val Lys Cys Arg Ile Cys Ser Gly Glu His Phe Thr Ala
       130                 135                 140
   Arg Cys Pro Phe Lys Asp Thr Met Ala Pro Val Glu Glu Gly Thr Ala
   145                 150                 155                 160
   Ala Ala Pro Gly Val Glu Ala Glu Glu Asp Ala Gly Gly Leu Gly Ala
                   165                 170                 175
   Gly Lys Ser Ser Tyr Val Pro Pro His Met Arg Lys Gly Gly Ala Gly
               180                 185                 190
   Gly Gly Glu Lys Met Gly Gly Arg Phe Glu Lys Asp Asp Leu Ala Thr
           195                 200                 205
   Leu Arg Val Thr Asn Val Ser Glu Leu Ala Glu Glu Asn Glu Leu Arg
       210                 215                 220
   Asp Leu Phe Glu Arg Phe Gly Arg Val Thr Arg Val Phe Leu Ala Arg
   225                 230                 235                 240
   Asp Arg Glu Thr Gln Arg Ala Lys Gly Phe Ala Phe Ile Ser Tyr Ala
                   245                 250                 255
   Asp Arg Gly Asp Ala Ala Leu Ala Cys Glu Lys Val Asp Gly Phe Gly
               260                 265                 270
   Tyr Arg His Leu Ile Leu Arg Val Glu Phe Ala Lys Arg Thr Thr
           275                 280                 285
<210> 41
   <211> 7890
   <212> DNA
   <213> Rasamsonia emersonii
<400> 41
   ggagcctggc tcagcatgct cacggactgc aggatgaaca cgcgtctccg aaggtgcaac     60
   cggggagaag ataaccaccg tccttgaggg aagaacatct gtcatcatcg aaacagaagc    120
   agtctcagtc tcggtctcgg tctcagtccc gtcatctata gtcgtcacag ttgggatcgg    180
   aaacgtggcc gaccagtccc tggtcttgta ccaggtgtta ttctcgtatc gcggcggcgc    240
   gatcatgtcg agcgcctcac caatccttcc gaggacatag cccatcctga acccgccctg    300
   gaggcgctcg atgaagttgt tggcgggatg aacggcggtg aaagggatgg catggaagat    360
   gccgaggatg gccatggcca tggagaacat gaacaggccg aaggggtcca tggtgatgat    420
   gattcgatcg atatcagaca gctgaatatg tattagcttt ccaaatttct tctatcatga    480
   aattcgacag gaaatagaaa aagaaaggat gctggctttg attaaagaga gtagctgggt    540
   cttatcacaa gagattaaaa agtgtcttaa acaaagacaa gacaagacaa gcatccgtct    600
   gatgcatgag tttcgagata tatataatac aaaggatgga ttcactgaac agttcgttga    660
   cttctgaaga acaaaggctg gtctctgcat gcccgagaat agtaacaaca ataaagagca    720
   aatacaacgt tgtgtacagc aagcgaatga cctgccttga atgagtctgg cagatttatt    780
   cccccatcta ctaccttcaa gtacctcatc agatggccag cagaaggtgc aagtgggtat    840
   atcttctcat tcgaagaact tagtgtttag ttttctgagc agcaatatag ctagttgcaa    900
   gttagaaaag agtataaaga accgtttccg caacaccagc taccctcaaa gaaaggaatt    960
   gaacaaactg ggaactaccg attactacca agctggaggg cataggcaat gaacgccagg   1020
   aattggtaaa gactgaagat aggaaggtat ggtgaatact gcatagtgca atgtagacct   1080
   tcagtatcat aaggatcatc cattcattat agacgctaat ataaagtatt tctgaaaaaa   1140
   aatgttggag aggagatcaa gtctttttat tcaacggctt tcaaagacta aaagctggaa   1200
   aaggcaggct atcatcatga tacctagcat agcataccat gtttgcgttt atcacttcat   1260
   attcaggaca acctcctgcc caggaccacg ccataggcaa ctgtcgccag gagaccgccc 1320
   aggttgacca gcgtcgagat tccatgcagc ttggcaaact tcttgttgag ctcggtcatc 1380
   tccttggagt gaggaggcgg gtcataactc ttcttgccgt cgatcgattc tgccagagat 1440
   catatatatc agcccgtacc tatacagtgt actcgtgcgt actcggcagc agccaaaccg 1500
   gtaggtagta ggcaggtacc aaccttgctg ccacctctgc ttgataactc ccacaacctt 1560
   cggggtcaga tagagcaggt tggtcagtcc cgaaacgaag acgatcgaaa gcggcagcaa 1620
   caccgtcaga cgattctcct cgagaagcac ccccgcgagg ctagagggcc cagttcccag 1680
   gaccgttctg gctcccgggt aggtcagcgc tgcgacaacg gggagagcgc tctgcagggt 1740
   gaagtagatc gggaacaggc tattctggag cgtcgagaac tgctgacggg gaagcgtcct 1800
   gaacgcgacg gttccgccaa cgaaggtcta catatataac ggaatgttgt aggagctttt 1860
   gatgagttat ctatcctcct gaccaattga ccagaaacaa aactcaggat gtcaaacctg 1920
   atagatctcg actcccagaa gggtgccgta gctgattgaa gtgtgatgtc agccgatcta 1980
   ttagtagcag ccgtagtact ggtgacgcac cttagtatgt ggaaggggcc caggatagac 2040
   atcctgctct gttgtactga tatggaaaca cctcgtgact ggaacagaac tatatgggat 2100
   tatacttaga cagatacccc actgactggg aattcagagg gaagagtaag ttgtgttatg 2160
   ctacgggtag gttagagaag ctgtcaagct tgggtctccc gagctaacgc tagctgcatg 2220
   tggggcatgt tcttatctcc acggcccgct caaacctaga tctgcttcca acaaagcaca 2280
   aatatctata cacacggcct tttccgtaag gcccacgcac cttcccgacg tcatgtgcac 2340
   tcgcgtctgc cgcgcctcaa aaaggaaata tcacgcgtct gcctggaggc gctccttagt 2400
   catagaaaga aacgcatcta cgccatgcag tgatttattt atctgacatt tccttcctct 2460
   tcgttgcagc aggagggaca gctgacatct cttttgcaaa atggctgaca aggaggccac 2520
   cgtctacgtg atcgatgtgg gaaagtccat ggggaggcgc cgccatggac ggccggtatc 2580
   tgacctggaa tgggcaatgc aatatgtctg ggacaagatt acgacaaccg tatgctgaca 2640
   cttgatccgg tctcctggaa attaaattcc tgcgttgaga actgacatat cttctgttag 2700
   gttgccacgg ggcggaaaac ggctacaatt ggagtggtcg ggctgaggac agatggtgag 2760
   attttaccgt gcccgaatca ggtaaatatg atttactgat gtatctggac agaaacatcg 2820
   aacgacttgc aggatgatga cagctattcg cacatctctg tctttcagga aattggacag 2880
   tatgtgcctc agctgacact gatgactagt gacttttcct cgcatatact aaataaatca 2940
   ctgccagggt cctcatgcct gatctgcgaa aactgcgcga cctgatcaag cctagcaaca 3000
   ctgatgaagg agatggtgag ttttgcccgt atcttcggac tcatttgatt tgatattgag 3060
   acctatctac ctatagctat ctcctccctt gtcgtcgcga tccagatgat caccacttat 3120
   accaaaaagc tgaagtatcg acggaaaatc attctcgtga cgaacgggga aggatccatg 3180
   agtaccgatg gtcttgatga gatcgtgaaa aagctcaagt ccgatagcat tgaattggtg 3240
   gtcttgtatg tttttcactt ctctttgact tttcttgtgg ctggtatgca aaatggctaa 3300
   actggtttcg ttgcaggggt gttgactttg atgatcctga atttggtgtc aaagaggagg 3360
   acaagaatcc agcaaaagta ttcaatgttt tttttttagc aggttggaag agttgctgat 3420
   tcgatctgcc gcaggctgag aatgaagcgg tcctcagagg tctcgttgat tcctgcgacg 3480
   gagtctacgg gacattacaa caggccatat tggagctgga cacaccgcgt gtgaaggttg 3540
   ttcgtggaat accctccttt agaggagagc tccgactggg gaaccctgaa gagtattcgt 3600
   ctgcccttcg tatcccagtc gaaagatact accgaactta tgttgccaag ccgccgacag 3660
   cgagctcctt tgtcctacga tctgacgctg cagctggtca agagggtgca gagaatgcac 3720
   tgacaagcgt ccgaaacgca cggacatatc acgtcagtga tgagtccgca ccaggaggca 3780
   agagagacgt ggagcgagaa gatctcgcca agggctacga gtatgggaga accgcggtgc 3840
   acattagtga gtccgatgag aatatcacca aactccagac gaaccctggt ctggaaatca 3900
   tcggcttcat tcagagtgac catgtatgtt tctcgtcaag ggtatctcat ctgaaccgtg 3960
   attaacctag gatccagtac gaccgataca tgcacatgtc taccagcaat gtcataattg 4020
   cacagaaagc aaacgaaaag gcgatccttg ctctttcatc tttcattcac gccttgttcg 4080
   agttggactg ttatgctgtg gccagacttg ttaccaagga caacaagccc ccactcatcg 4140
   tattactggc accatctatt gaagcagact ttgaatgtct tctagaagtc cagctccctt 4200
   ttgctgaaga tgttcggtcg taccgtttcc ctcccttgga caaggtggtc actgtctctg 4260
   gaaagacagt caaagagcac cgacatctcc caagtgacga attgctgaat gcgatgagca 4320
   aatacgtcga cagcatggag ctcgtcgaca aggatgaaaa cgggtgagtc atcacaggga 4380
   aaccgtcatg ctgctcatct caagtatact gacaactcca cagagaacca gttgacagcc 4440
   tggctcccag actggaggat tcgtactctc cactgctgca caggatcgag caagctatcc 4500
   ggtggcgtgc catccatcca aacgagcctc ttccgccccc ttctgagaag ttgacgcagc 4560
   tgtcacgacc gccagcagat ctgcaagcgc gcgcgaagaa atacctggat cgggtcattg 4620
   ccgccgccga tgtgaagaaa ggtctgtcaa cttctacgct cccccagaat gcatctgact 4680
   aaaaaatgct gcacagttcc accaaaagca aaaggtcgca agcggaatcg cgaagccgac 4740
   aaacccctat cgggtcttga cgttgacgag ctccttcgtc gcgagaagcg cgccaagatc 4800
   tcagccaaca acgccatccc cgagttcaaa cagtcgctgg tcaacgccga gaccatcgac 4860
   gccgtccgtg acgcagtcag ccagatggaa agcatcatcg agaaccacat ccgaagcagc 4920
   tttggagacg ccaactacga ccgcgtgatc gaggagctgg gtgtcctccg cgaggagctg 4980
   atcgcctacg aagagccgga tctctacaac gacttcctgc ggaggctgaa ggacaagatc 5040
   ctcaatgagg agctgggcgg agacagacga gagctgtggt ggctcgtcag gaggcaacgg 5100
   gtcggtctga tagacaagaa ggcgtcggaa cgggttgaag ttactgaaca ggaagccagg 5160
   gaggtaagta agcagataca ttattccttt agttccatta aacgagctgc atgatgagct 5220
   gacttttgtt cactagttca tgacctcgaa ataaaatagt ccattattgc tatgtatgtc 5280
   aaggcgcctg gccgtagtag tcttaacatg ctgatgctgt gaatcaaagc gccagatgaa 5340
   caataataga aataatacca cttggtagct gtctccattc tcacagatag acaacgttaa 5400
   agaaaagaaa aacgtaaaaa gagggtatat gtggtctagt aacgccgcaa ggaaaaaaaa 5460
   actcatacgt tagtttcgaa cgcaaatctc aaaatcgagc acttcgagta aatactctgt 5520
   cgtatcgttt cgcctcagga tatcttcccg agccttctct ttccgatatc gattttccgt 5580
   tgtaatctag ttattattac tccagttagt aaatgcacga cgggcagtat tgtaaataat 5640
   gaaatcagca gcgagagtac gaacatgtcc acatcctcat cggctttccg gagcaactcg 5700
   ttctggatct ccagctcatt gttaatggcg atccccagct ccttctgtcg agcgacgatt 5760
   ttcatcaact cctccacgct ccggtcctgc tcttccatcg tctgcttctg cagctggagc 5820
   acgccctggt tgtcgagttc ccgcgtcttg tccgtttcct tgcccaggac tcgtccagaa 5880
   cggggtttgg cgctccccac cagggcgtcc ttgtcctgca tcgaagcgac agcgttgtcg 5940
   agcttgctct tcgtcaccat cgcgttgtgc agattctcca atccgtcctt ctctttcttg 6000
   gcgctcgcga tgagatcctt cctccgacgg atctctccct cgcctaacct gctgccgccc 6060
   catccagacg acttgtcgct caggttcttc agcccttcct caagagcgcc gatcatcgac 6120
   cctgctttca ccaagctgct tttggcctgt gccgagctct cgtgttgttt ctgtggagtc 6180
   gtggcctgat cacgtctcgt cagatgcagc ctcgtctcgt gtaagtgcgc cttcatctcc 6240
   cggtagcaat ccagccacag gactggatcc gtgatcggtc cgccgcctgg cgcgcccggt 6300
   tccgtgatgg acttgtgaag cttcgatgcg gcagagctat ccgacagggc ttgggagggg 6360
   aggtttagaa aggacctcca gacgcttgtt tgacgccatc gcgggtcctc gctctcgttg 6420
   atggcccgca ggtatctttc caggcctttg cgccgctctt cgcgcagagt ctcgttcgag 6480
   ttcgtgttgg aaaaccagga cttcccgggc agagcgacgg gtggttgggc gccaacctgg 6540
   cggactagtg cgtcatggaa cgatgcaaat tctgaatagc gtttctggac aacgaacgac 6600
   cgtagaggca gccggatggt gatgttgtat agcgtatacg gactgggagc gtccgcgatg 6660
   gtggctgtcg ggatggaaat ttcgacattc ggggccatga ttatagttca gacgggaaaa 6720
   agaacaaaac aaagagcagg cccttgttat cgaccaggaa gcataattcc cgccgcttct 6780
   cttgcggtat ctctgtcgtt gcagagttgg ttgcagagta gtggagtcgg ccggcgggtg 6840
   gaaactcccg caatgacgca ggcgccccat cttcttctgc caccgccgat ctgtggctta 6900
   gcttcttctt gtcaagactc gactccacca tcgcgactcc aggcagcacg aatcgcacga 6960
   ttgccgaaaa actacaccgt actaggggaa ggcctaatta atctattacc ctagctaaaa 7020
   atggggttgt caaacttatc atatagccgt gcgacccgcc cttggaggtc actagatcca 7080
   acctgcgcac ggcctggtta cggttgatgg gagctaaaat tagaacgaaa gatatactgg 7140
   cggtccgtcc ccgcgtctat ccacaatcca aaactcgtat gcagagttat ctacaggtcg 7200
   atccaatcat gagtcctttg tgacatgtcg ttgaatacat ggtctcaatc gagtctgccg 7260
   ttcttacatg accatcctca ccaagatcaa tgtcccgtga ttcgactgtc agccaagata 7320
   cgtctcacct ggccccatct ctactgtcga caacgtctgc ctatactgta ggtgatcaga 7380
   atacgcagtc ccggggagtc tactcgcgat ggggtggttc atacgtcggc tcctcgtcga 7440
   cgttgtctct gggtccgtcg gagagcgtca atatagacgg gagacgaaag ttgctcttga 7500
   tctatatcca tggcttcatg ggtgaagaag cgagcttcca caagttccct gctcatgtcc 7560
   ataaccttgt caccattgct ctggccgagt cgcacgttgt gtattcgaag gtatatcctc 7620
   gatacaaatc ccgccgagca atggacattg cacgtgatga tttcagtcga tggtgcgttt 7680
   gcagactggc atatctctct ttagagatca tcctagaaag aaacgcatga tactaagtgt 7740
   cgaataggct atcaccgcat gagtcggaag atacagatgt gatcctactc ggccacagcc 7800
   tgggtgggat cctagccgca gaggttgcgc tgctcccatc agcccctggg agcaaggaga 7860
   tcttcgagca tcgtatcctg ggactcatca 7890
<210> 42
   <211> 2145
   <212> DNA
   <213> Rasamsonia emersonii
<400> 42
   atggctgaca aggaggccac cgtctacgtg atcgatgtgg gaaagtccat ggggaggcgc      60
   cgccatggac ggccggtatc tgacctggaa tgggcaatgc aatatgtctg ggacaagatt     120
   acgacaaccg ttgccacggg gcggaaaacg gctacaattg gagtggtcgg gctgaggaca     180
   gatgaaacat cgaacgactt gcaggatgat gacagctatt cgcacatctc tgtctttcag     240
   gaaattggac aggtcctcat gcctgatctg cgaaaactgc gcgacctgat caagcctagc     300
   aacactgatg aaggagatgc tatctcctcc cttgtcgtcg cgatccagat gatcaccact     360
   tataccaaaa agctgaagta tcgacggaaa atcattctcg tgacgaacgg ggaaggatcc     420
   atgagtaccg atggtcttga tgagatcgtg aaaaagctca agtccgatag cattgaattg     480
   gtggtcttgg gtgttgactt tgatgatcct gaatttggtg tcaaagagga ggacaagaat     540
   ccagcaaaag ctgagaatga agcggtcctc agaggtctcg ttgattcctg cgacggagtc     600
   tacgggacat tacaacaggc catattggag ctggacacac cgcgtgtgaa ggttgttcgt     660
   ggaataccct cctttagagg agagctccga ctggggaacc ctgaagagta ttcgtctgcc     720
   cttcgtatcc cagtcgaaag atactaccga acttatgttg ccaagccgcc gacagcgagc 780
   tcctttgtcc tacgatctga cgctgcagct ggtcaagagg gtgcagagaa tgcactgaca 840
   agcgtccgaa acgcacggac atatcacgtc agtgatgagt ccgcaccagg aggcaagaga 900
   gacgtggagc gagaagatct cgccaagggc tacgagtatg ggagaaccgc ggtgcacatt 960
   agtgagtccg atgagaatat caccaaactc cagacgaacc ctggtctgga aatcatcggc 1020
   ttcattcaga gtgaccatta cgaccgatac atgcacatgt ctaccagcaa tgtcataatt 1080
   gcacagaaag caaacgaaaa ggcgatcctt gctctttcat ctttcattca cgccttgttc 1140
   gagttggact gttatgctgt ggccagactt gttaccaagg acaacaagcc cccactcatc 1200
   gtattactgg caccatctat tgaagcagac tttgaatgtc ttctagaagt ccagctccct 1260
   tttgctgaag atgttcggtc gtaccgtttc cctcccttgg acaaggtggt cactgtctct 1320
   ggaaagacag tcaaagagca ccgacatctc ccaagtgacg aattgctgaa tgcgatgagc 1380
   aaatacgtcg acagcatgga gctcgtcgac aaggatgaaa acggagaacc agttgacagc 1440
   ctggctccca gactggagga ttcgtactct ccactgctgc acaggatcga gcaagctatc 1500
   cggtggcgtg ccatccatcc aaacgagcct cttccgcccc cttctgagaa gttgacgcag 1560
   ctgtcacgac cgccagcaga tctgcaagcg cgcgcgaaga aatacctgga tcgggtcatt 1620
   gccgccgccg atgtgaagaa agttccacca aaagcaaaag gtcgcaagcg gaatcgcgaa 1680
   gccgacaaac ccctatcggg tcttgacgtt gacgagctcc ttcgtcgcga gaagcgcgcc 1740
   aagatctcag ccaacaacgc catccccgag ttcaaacagt cgctggtcaa cgccgagacc 1800
   atcgacgccg tccgtgacgc agtcagccag atggaaagca tcatcgagaa ccacatccga 1860
   agcagctttg gagacgccaa ctacgaccgc gtgatcgagg agctgggtgt cctccgcgag 1920
   gagctgatcg cctacgaaga gccggatctc tacaacgact tcctgcggag gctgaaggac 1980
   aagatcctca atgaggagct gggcggagac agacgagagc tgtggtggct cgtcaggagg 2040
   caacgggtcg gtctgataga caagaaggcg tcggaacggg ttgaagttac tgaacaggaa 2100
   gccagggagt ccattattgc tatctgtctc cattctcaca gatag 2145
<210> 43
   <211> 714
   <212> PRT
   <213> Rasamsonia emersonii
<400> 43
   Met Ala Asp Lys Glu Ala Thr Val Tyr Val Ile Asp Val Gly Lys Ser
   1               5                   10                  15
   Met Gly Arg Arg Arg His Gly Arg Pro Val Ser Asp Leu Glu Trp Ala
               20                  25                  30
   Met Gln Tyr Val Trp Asp Lys Ile Thr Thr Thr Val Ala Thr Gly Arg
           35                  40                  45
   Lys Thr Ala Thr Ile Gly Val Val Gly Leu Arg Thr Asp Glu Thr Ser
       50                  55                  60
   Asn Asp Leu Gln Asp Asp Asp Ser Tyr Ser His Ile Ser Val Phe Gln
   65                  70                  75                  80
   Glu Ile Gly Gln Val Leu Met Pro Asp Leu Arg Lys Leu Arg Asp Leu
                   85                  90                  95
   Ile Lys Pro Ser Asn Thr Asp Glu Gly Asp Ala Ile Ser Ser Leu Val
               100                 105                 110
   Val Ala Ile Gln Met Ile Thr Thr Tyr Thr Lys Lys Leu Lys Tyr Arg
           115                 120                 125
   Arg Lys Ile Ile Leu Val Thr Asn Gly Glu Gly Ser Met Ser Thr Asp
       130                 135                 140
   Gly Leu Asp Glu Ile Val Lys Lys Leu Lys Ser Asp Ser Ile Glu Leu
   145                 150                 155                 160
   Val Val Leu Gly Val Asp Phe Asp Asp Pro Glu Phe Gly Val Lys Glu
                   165                 170                 175
   Glu Asp Lys Asn Pro Ala Lys Ala Glu Asn Glu Ala Val Leu Arg Gly
               180                 185                 190
   Leu Val Asp Ser Cys Asp Gly Val Tyr Gly Thr Leu Gln Gln Ala Ile
           195                 200                 205
   Leu Glu Leu Asp Thr Pro Arg Val Lys Val Val Arg Gly Ile Pro Ser
       210                 215                 220
   Phe Arg Gly Glu Leu Arg Leu Gly Asn Pro Glu Glu Tyr Ser Ser Ala
   225                 230                 235                 240
   Leu Arg Ile Pro Val Glu Arg Tyr Tyr Arg Thr Tyr Val Ala Lys Pro
                   245                 250                 255
   Pro Thr Ala Ser Ser Phe Val Leu Arg Ser Asp Ala Ala Ala Gly Gln
               260                 265                 270
   Glu Gly Ala Glu Asn Ala Leu Thr Ser Val Arg Asn Ala Arg Thr Tyr
   275 280 285
   His Val Ser Asp Glu Ser Ala Pro Gly Gly Lys Arg Asp Val Glu Arg
   290 295 300
   Glu Asp Leu Ala Lys Gly Tyr Glu Tyr Gly Arg Thr Ala Val His Ile
   305 310 315 320
   Ser Glu Ser Asp Glu Asn Ile Thr Lys Leu Gln Thr Asn Pro Gly Leu
   325 330 335
   Glu Ile Ile Gly Phe Ile Gln Ser Asp His Tyr Asp Arg Tyr Met His
   340 345 350
   Met Ser Thr Ser Asn Val Ile Ile Ala Gln Lys Ala Asn Glu Lys Ala
   355 360 365
   Ile Leu Ala Leu Ser Ser Phe Ile His Ala Leu Phe Glu Leu Asp Cys
   370 375 380
   Tyr Ala Val Ala Arg Leu Val Thr Lys Asp Asn Lys Pro Pro Leu Ile
   385 390 395 400
   Val Leu Leu Ala Pro Ser Ile Glu Ala Asp Phe Glu Cys Leu Leu Glu
   405 410 415
   Val Gln Leu Pro Phe Ala Glu Asp Val Arg Ser Tyr Arg Phe Pro Pro
   420 425 430
   Leu Asp Lys Val Val Thr Val Ser Gly Lys Thr Val Lys Glu His Arg
   435 440 445
   His Leu Pro Ser Asp Glu Leu Leu Asn Ala Met Ser Lys Tyr Val Asp
   450 455 460
   Ser Met Glu Leu Val Asp Lys Asp Glu Asn Gly Glu Pro Val Asp Ser
   465 470 475 480
   Leu Ala Pro Arg Leu Glu Asp Ser Tyr Ser Pro Leu Leu His Arg Ile
   485 490 495
   Glu Gln Ala Ile Arg Trp Arg Ala Ile His Pro Asn Glu Pro Leu Pro
   500 505 510
   Pro Pro Ser Glu Lys Leu Thr Gln Leu Ser Arg Pro Pro Ala Asp Leu
   515 520 525
   Gln Ala Arg Ala Lys Lys Tyr Leu Asp Arg Val Ile Ala Ala Ala Asp
       530                 535                 540
   Val Lys Lys Val Pro Pro Lys Ala Lys Gly Arg Lys Arg Asn Arg Glu
   545                 550                 555                 560
   Ala Asp Lys Pro Leu Ser Gly Leu Asp Val Asp Glu Leu Leu Arg Arg
                   565                 570                 575
   Glu Lys Arg Ala Lys Ile Ser Ala Asn Asn Ala Ile Pro Glu Phe Lys
               580                 585                 590
   Gln Ser Leu Val Asn Ala Glu Thr Ile Asp Ala Val Arg Asp Ala Val
           595                 600                 605
   Ser Gln Met Glu Ser Ile Ile Glu Asn His Ile Arg Ser Ser Phe Gly
       610                 615                 620
   Asp Ala Asn Tyr Asp Arg Val Ile Glu Glu Leu Gly Val Leu Arg Glu
   625                 630                 635                 640
   Glu Leu Ile Ala Tyr Glu Glu Pro Asp Leu Tyr Asn Asp Phe Leu Arg
                   645                 650                 655
   Arg Leu Lys Asp Lys Ile Leu Asn Glu Glu Leu Gly Gly Asp Arg Arg
               660                 665                 670
   Glu Leu Trp Trp Leu Val Arg Arg Gln Arg Val Gly Leu Ile Asp Lys
           675                 680                 685
   Lys Ala Ser Glu Arg Val Glu Val Thr Glu Gln Glu Ala Arg Glu Ser
       690                 695                 700
   Ile Ile Ala Ile Cys Leu His Ser His Arg
   705                 710
<210> 44
   <211> 4063
   <212> DNA
   <213> Rasamsonia emersonii
<400> 44
   ctcggagctg ggaggcgtgc tctcgttcgg atccacggtt tatggcttcg cgactggctg       60
   gaccagctat gcagcagatt acaccgtcta tcagcccgct aaccgcagtc gccgcaaggt      120
   gtttttggcg acatggcttg gtatcatcac tcctctcctc ttcacggaga tgctgggcgt      180
   ggctgtcatg accgccacca gccttaatga cggcaacaat gcctaccagg acggctataa      240
   cgcttctggc accggtggct tgctggctgc tgtcctcttc cccaagctgg gtggctttgg      300
   caagttctgc gtcgtcatcc tcgcactgtc aatcattgcc aacaactgcc ccaacattta 360
   ctcggtgtcc cttaccctct tagttctggg ccgttggact cgtctcatac cgcgcttcat 420
   ctggactctt gtagccaccg gggtctacgt cgccattgct atccccggct attcgcactt 480
   cggcggtgtt ggagaatttc atgaacttca tcgcttactg gctggcgatc tatgagggga 540
   ttgccgtgac cgatcacttt gtcttcaagc gtggtttctc agggtaccga cccgaaatct 600
   acgatcggcc cgacgaacta cccccgggca ttgctgcggt gggtgcgttc tgctgtggtg 660
   ttgcgggaat gatcaccgga atgagccagc agtggtgggt cggtcccatt gctctgcacg 720
   ctggcgaagc gccgtttggc ggcgacgtcg gctttgagct tggctttgca ttcgctgctg 780
   tcggctattt aatcttgaga ccgatcgagc tgaggatctt caagcgatag ggttgtttgg 840
   atggcattgt gagggaaatt ggtgtcttcg tgtttttttt ttgatgaggt tatatgttta 900
   tatatattca tctttcgtca tcttttagcc gtcatggagt gtaattatgt cgataccatc 960
   aatgcatgta tattgagagg catttccttt ctgggaagta tagacctagt acacaatata 1020
   gcgagtcgaa cctgatatca gaggatctat cggtatatat catcagacgt tattcacata 1080
   gtcagggaat atcatcccag catcaaagga cattcaagtt ttctcagtag gttgtgatca 1140
   ggtcctgcta acctggatat gtagagtaca tatactttga gagttgagtt caatctcctc 1200
   ctcttttctc ttttgaaagc caaattgagg tgtgctgcta aattgtctaa taaatagaat 1260
   cacacgacaa gggagcactg caagtacaac agttaaagag aatgcaaatg attaccatgc 1320
   gtcataggac aagagtatag atctgcttgg tcacgtgact tgttcgctat gtctagccac 1380
   acggctgacc tgcgccaccg attgaatcca tctaaaagaa atccacaacg gattcgcctc 1440
   cgctccgaaa tcaagaaaat tgatcacaat caagaaacca caatattatc accacaaaga 1500
   tgtcgagact ggcaaagtaa gtacattatc aaatattccg gacgtgtgct ggacataatt 1560
   ttgctaacat tgtcttcttt catctacagc cgtgccgact gggccgacga cgaggagttt 1620
   gatgacccct ccgccctccc cgcacagcaa gtcacaacca acaaggacgg aacgaaaact 1680
   gtcgtgtcgt atcgcttcaa cgatgatggc aaaaaagtga aagtgacgcg gcggatccgc 1740
   accaccgtcg tgaaggagca cgtcaaccct cgagtcgcgg aacggagaac atgggccaag 1800
   ttcggtctgg agaagggcca tgctcccggc ccatcgtttg acaccacctc ggtcggcgag 1860
   aacattgtct tccgtcccag cgtcaactgg aaggcgcagg ccaaggaggc ggagaaggaa 1920
   ggcgagaagg gcggcatcaa ggaccagctg aaggacaaga aggtcaagtg ccggatttgc 1980
   agcggagagc acttcacggc tcgctgtccg ttcaaggaca cgatggctcc gatcgacgag 2040
   cctgctcacg gccccggagt ggatttggac gacgacgaca ggcccacggg cgctctgggc 2100
   ggtggtggag gaagctatgt gcctcctcac ctgcgcaaag gcggtgctgc ggctggcgag 2160
   aagatgggtg gcaagtacga gagagacgat cttgcgaccc tgagagtgac aaacgtaagt 2220
   gctatttgcc agccttttct agatgagata tgagctgaca cggattgctt ctactcaggt 2280
   gagcgagttg gcagaagagc aggaactccg ggatctattc gaacggtttg gacgggtcac 2340
   ccgagtcttt ttggcccggg accgcgacac gcagaaagcc aagggctttg catttatcag 2400
   cttcgcagac cggaacgacg ctgctcgtgc ttgcgagaag atggatggct gtaagtgtcc 2460
   ccacgatggt atcatgagtc tcggtcatat gctgacatta actcctccag tcggttaccg 2520
   ccaccttatc ctgcgcgtgg aattcgccaa gagggcttct taaaagaact tccttccctt 2580
   ttcttttact tcttcgcctc acccgtcatg gcttatttca tttcatggtt gaagattgtt 2640
   tgtactgtac gacgaatcga acgacctttc gaattcggta ggatagcttg gtaatgatga 2700
   tgcttttttt tcatgttgaa cgagggatca gataagcaaa tagcatcttt tgattcttct 2760
   ctctcgtctt gctgctcgag cgtacataca tagaatgaat tgatatatcc ttatgtccgc 2820
   catctctctc tccctgctgt tgactatgga ataacaaaca gaaaaagctg attcagatat 2880
   caaaaggcta tatgtattga aatggaccac tgaacgggac atgatatccc acccaaaaaa 2940
   agtgttcctt tcgatcgtcg cccttctctc gacccgattt cctttcctta cccttacctt 3000
   cttgatccaa ggagttcaaa aagctcaaaa gctcaagtga aaagaaaaga gttatttcta 3060
   ctcaaactgt actgtacata tagagtagct tttattcctt gtttcttttt ttgtcttgtg 3120
   tgcgtttaca tcagatatat gtacatacat acatatacat gcatatacac gaatcctcgc 3180
   tatactatag ttgatacgga tgtatatcct atcctatctt cttacttaca tacataggaa 3240
   agggaagtga aaagagatac cagaatacag aataaacaac agagaatgaa tgagtgagtg 3300
   ggtgagtaag tgagtgaatt tctacgaact gatcctcgtc aagtacagta agacgagatg 3360
   taaatgaata tcaatctact tactccatga gtcagtcagt ctattaagta tcacctctac 3420
   tatcacctct actataggta aaaacattaa tttccgtttt tgtatactgt catgtgcctc 3480
   tctaaatatc taaatattag ggaagggata tatagctaca gtagagttag ggtaaaaaag 3540
   agattacagc aggggatata gttagttagt aagtccggct agagagtaga tgatgacaag 3600
   gactaactac taaaatatga gaaataataa taataataat atgtatatag tgaggcccgc 3660
   ctgggaggtg gattgcaaag tccttggcat aaccaggtat acctattttc tcttcaaatc 3720
   agtcgagcaa aatgagacct ggaaggcttg tgtattatat tataggtaat caacagagat 3780
   tcattttgta tttgtttcat tttattccgt ctcatgcacg acagacaagc aacttttgct 3840
   tgctacctac ctactctgta aagaaaactc catacatgac ttcatgatat atgtggtatg 3900
   cctgtcctgt cctgtccaga tctatatcta gatgagatga gatatatggt tagtatgtac 3960
   atacatacat gtactccgta ggtaactact aaagtaacta cctaggtata ctgtaagtcg 4020
   gagggcctgc agcctgccct ggcggattgt tcatgaatgc agt 4063
<210> 45
   <211> 927
   <212> DNA
   <213> Rasamsonia emersonii
<400> 45
   atgtcgagac tggcaaaccg tgccgactgg gccgacgacg aggagtttga tgacccctcc      60
   gccctccccg cacagcaagt cacaaccaac aaggacggaa cgaaaactgt cgtgtcgtat     120
   cgcttcaacg atgatggcaa aaaagtgaaa gtgacgcggc ggatccgcac caccgtcgtg     180
   aaggagcacg tcaaccctcg agtcgcggaa cggagaacat gggccaagtt cggtctggag     240
   aagggccatg ctcccggccc atcgtttgac accacctcgg tcggcgagaa cattgtcttc     300
   cgtcccagcg tcaactggaa ggcgcaggcc aaggaggcgg agaaggaagg cgagaagggc     360
   ggcatcaagg accagctgaa ggacaagaag gtcaagtgcc ggatttgcag cggagagcac     420
   ttcacggctc gctgtccgtt caaggacacg atggctccga tcgacgagcc tgctcacggc     480
   cccggagtgg atttggacga cgacgacagg cccacgggcg ctctgggcgg tggtggagga     540
   agctatgtgc ctcctcacct gcgcaaaggc ggtgctgcgg ctggcgagaa gatgggtggc     600
   aagtacgaga gagacgatct tgcgaccctg agagtgacaa acgtgagcga gttggcagaa     660
   gagcaggaac tccgggatct attcgaacgg tttggacggg tcacccgagt ctttttggcc     720
   cgggaccgcg acacgcagaa agccaagggc tttgcattta tcagcttcgc agaccggaac     780
   gacgctgctc gtgcttgcga gaagatggat ggctgtaagt gtccccacga tggtatcatg     840
   agtctcggtc atatgctgac attaactcct ccagtcggtt accgccacct tatcctgcgc     900
   gtggaattcg ccaagagggc ttcttaa                                         927
<210> 46
   <211> 308
   <212> PRT
   <213> Rasamsonia emersonii
<400> 46
   Met Ser Arg Leu Ala Asn Arg Ala Asp Trp Ala Asp Asp Glu Glu Phe
   1               5                   10                  15
   Asp Asp Pro Ser Ala Leu Pro Ala Gln Gln Val Thr Thr Asn Lys Asp
               20                  25                  30
   Gly Thr Lys Thr Val Val Ser Tyr Arg Phe Asn Asp Asp Gly Lys Lys
           35                  40                  45
   Val Lys Val Thr Arg Arg Ile Arg Thr Thr Val Val Lys Glu His Val
       50                  55                  60
   Asn Pro Arg Val Ala Glu Arg Arg Thr Trp Ala Lys Phe Gly Leu Glu
   65                  70                  75                  80
   Lys Gly His Ala Pro Gly Pro Ser Phe Asp Thr Thr Ser Val Gly Glu
                   85                  90                  95
   Asn Ile Val Phe Arg Pro Ser Val Asn Trp Lys Ala Gln Ala Lys Glu
               100                 105                 110
   Ala Glu Lys Glu Gly Glu Lys Gly Gly Ile Lys Asp Gln Leu Lys Asp
           115                 120                 125
   Lys Lys Val Lys Cys Arg Ile Cys Ser Gly Glu His Phe Thr Ala Arg
       130                 135                 140
   Cys Pro Phe Lys Asp Thr Met Ala Pro Ile Asp Glu Pro Ala His Gly
   145                 150                 155                 160
   Pro Gly Val Asp Leu Asp Asp Asp Asp Arg Pro Thr Gly Ala Leu Gly
                   165                 170                 175
   Gly Gly Gly Gly Ser Tyr Val Pro Pro His Leu Arg Lys Gly Gly Ala
               180                 185                 190
   Ala Ala Gly Glu Lys Met Gly Gly Lys Tyr Glu Arg Asp Asp Leu Ala
           195                 200                 205
   Thr Leu Arg Val Thr Asn Val Ser Glu Leu Ala Glu Glu Gln Glu Leu
       210                 215                 220
   Arg Asp Leu Phe Glu Arg Phe Gly Arg Val Thr Arg Val Phe Leu Ala
   225                 230                 235                 240
   Arg Asp Arg Asp Thr Gln Lys Ala Lys Gly Phe Ala Phe Ile Ser Phe
                   245                 250                 255
   Ala Asp Arg Asn Asp Ala Ala Arg Ala Cys Glu Lys Met Asp Gly Cys
               260                 265                 270
   Lys Cys Pro His Asp Gly Ile Met Ser Leu Gly His Met Leu Thr Leu
           275                 280                 285
   Thr Pro Pro Val Gly Tyr Arg His Leu Ile Leu Arg Val Glu Phe Ala
       290                 295                 300
   Lys Arg Ala Ser
   305
<210> 47
   <211> 55
   <212> DNA
   <213> Penicillium chrysogenum
<400> 47
   ggggacaact ttgtatagaa aagttgggcc caacgcatgt gtacgagagt caagg 55
<210> 48
   <211> 50
   <212> DNA
   <213> Penicillium chrysogenum
<400> 48
   ggggactgct tttttgtaca aacttgagac ggaaggagat cgcgtaacag 50
<210> 49
   <211> 50
   <212> DNA
   <213> Penicillium chrysogenum
<400> 49
   ggggacagct ttcttgtaca aagtggggcg cagtccattc ttgcatctac 50
<210> 50
   <211> 53
   <212> DNA
   <213> Penicillium chrysogenum
<400> 50
   ggggacaact ttgtataata aagttgggcc cagccacttc ttgtatcacg gat 53
<210> 51
   <211> 35
   <212> DNA
   <213> Aspergillus nidulans
<400> 51
   agagaggatc cgagttggcc agttgacaac ctgag 35
<210> 52
   <211> 37
   <212> DNA
   <213> Aspergillus nidulans
<400> 52
   agagagcggc cgcgagtatg agcgatcgac acgaatg 37
<210> 53
   <211> 3438
   <212> DNA
   <213> Aspergillus nidulans
<400> 53
   agagaggatc cgagttggcc agttgacaac ctgagcatct cggagttttt ttcacgggag 60
   cttgggcctt gtggatgtcc gatttccgag ctgaggccga ggccgccttc aacagctgag 120
   ctgatgtcag gccatcctga tattgttgaa tgcgcttccg cggttaccgg tcgtatcctg 180
   atggcgacaa ttttcgactc ggtagcgccg gcttgaatgg tagaatgcag ccgctcgaaa 240
   aaaagatggc tgggaatcgt tctttgttag atactcaacg tctcaccata tcgatagctc 300
   tagtcgagta tccaggagct ggtaggagct gggtggtgaa ctagactgca agttaaatga 360
   agacagtcag gggactgggt cttggcggta gggacccatt tggttggcca gcgccagcag 420
   atctgtggct tccggttggc tacttgtaac caactgatgg tcagatggat ctgccgtctg 480
   ttttgatttg aattttccct gctcattctg attctgtgag aggctgcatt cattatcaca 540
   tctcataccc ggcgcctgcg acttcggtca cctctgcggt ctggcggtta gcggggtgcg 600
   tctgagactc gtcagtcagc attcgagtat gcgaactctg actttgctca cctaagagtt 660
   tgcacgagat gccgaaatcc tcctcgagta gagtttgcaa ggcttgaacc ttggtccttg 720
   aagcccgaaa gtggctcagt agtgggatcg atagtctggt tgttgaagat tttctcctcc 780
   accttaccta tggccgctgg ccttctccac ctttcaggct ttcaggcacc ctcggctcgg 840
   attctgtatc gtccggtacc gaagctagtc ctagctagtc aaagctagtc caagctagtc 900
   tcgtcaaggt ttggcgcagc gcggttccgt gtaaagtaca aatttgaaat acgaatacgc 960
   agtactcgca gccggcactt ccgctcagcc caggctcaga ggctaagggt gttggcgctt 1020
   cctcatcatc ttcttctcgt cgaccttttc ctctttctct ccctatcggt gcttctctcc 1080
   aacctcattc tcagtcgttc gcccatcagg tttatactcc ggctccgtgg ccatctgcct 1140
   ccctcacgac ctcctcgttc caggttttcc tctcgactgc tgcgcccttg cacttcgcct 1200
   tgcatcagtg aaaccccctg caacgtgacg gctcaaagac atcctcgttt ggccgctgga 1260
   gaccggagcg tgcgcttcgt ttcgtcttct tcgaaccgat ctcaatttcc ccgctcgggt 1320
   tgacgccgtc agcaccctgc tcgttgccta acggcttgtt attcaagacc ccttttctgc 1380
   cgcttccgcg accgatttat tcgtcgcctt ccaactcttg tacaatcggg gggaaagaaa 1440
   gcagacggag ttcgatctgg aggaattata gctgagtctt gcccgcaaga ctcgccgcaa 1500
   ccatgaatca aacacttccc acgtggaagg accgcacgca gaaccagttt ggaaagcttc 1560
   agatccaggt tccatggcgg tccatccaac tgctcgtccc gcatcgcatg cggcggaagt 1620
   taaggtccaa attgcgcagt agagcgtctc ctacctcgtc aatagcctct ttacagacgt 1680
   cgttatcgcc tgcagacaca ctacgatcgc tccaaagcca ccgatggacg gtttacgact 1740
   tccaatatct gcttctgttg atcgtgggca tcttctcttt gaccgttatc gagtcgcccg 1800
   ggcctttggg caaaacggcc attttctcca tgctcctatt ctctctcctg atccctatga 1860
   cccgccagtt cttcctcccg tttctgccga ttgccggatg gcttctgttt ttctacgcct 1920
   gccagtgagt taaaaacaac ccgctaccag accccgtgca gcagttactc acatatgcag 1980
   gttcatccca agcgattggc gccctgcgat ttgggttcgt gtcttgcctg cactggagaa 2040
   tattctctac ggcgcaaaca tcagcaacat cctatccgct caccagaacg ttgtgcttga 2100
   cgtgctggcg tggctaccct acggtatctg ccactatggc gctccgtttg tgtgctcgtt 2160
   gatcatgttc atcttcggtc cgcccggcac tgttcccctt ttcgcgcgca ctttcggcta 2220
   tatcagtatg actgcggtta ctattcagct gtttttccct tgctctccac cttggtatga 2280
   gaatcgctat ggtctagctc cggcagacta ctccatccaa ggtgatcccg cagggcttgc 2340
   ccgcattgac aagcttttcg gcatcgacct ttacacgtct ggtttccatc agtcgcctgt 2400
   tgtgttcggc gcttttccgt cgctgcatgc tgccgactca accctggccg cacttttcat 2460
   gagtcatgtt ttcccccgca tgaagcccgt cttcgtgacc tatactctat ggatgtggtg 2520
   ggcaacaatg tacctctcac atcactatgc ggtcgatttg gttgcgggtg gtctcctggc 2580
   cgccattgct ttctacttcg ccaagacccg attccttccc cgtgtccagc tcgacaagac 2640
   cttccgttgg gactacgact atgtggaatt cggcgagtct gccctggagt atgggtatgg 2700
   tgcagctggc tatgatggag acttcaatct cgacagcgat gaatggactg ttggttcttc 2760
   atcctccgtc tcctcaggct ccttgagtcc cgttgacgat cattactcat gggaaaccga 2820
   ggcactgacc tccccacata ctgatattga gtccggcagg catactttca gcccttgagt 2880
   agccacaaac caaactcgat acctgcatat agcgatctcg ctcctcctcc actgcatcta 2940
   tttacgagac ggcgttagaa catttcacga cattctggct ttattgcatc gagcacattt 3000
   cgacacatat atctttaata ccctttcttc ggtgtcccag atcatcggtt cgaccttaat 3060
   gtacctcggt ccgaatccgc ctgggatact gtttctcttt ccgccgcact tcactgtaca 3120
   ttgcttgaca ttgcgaaacc gggttgggct cgaacgtggg atgggttatc gctcatcgct 3180
   acacgccgtt gctccatcat aatgttaatg gacacaatgg ggctacgcat cctggtgttt 3240
   agtcctggaa gaccatccga taacccccgt cggtaacact cgcttgtctc gtgtccaccc 3300
   agacactact tcaattctca cttctatcgt ccgctattac cttgacctgg tcgaacccat 3360
   ccttattatt cgtttcgact atgctatata tttattttta ccattcgtgt cgatcgctca 3420
   tactcgcggc cgctctct 3438
<210> 54
   <211> 24
   <212> DNA
   <213> Penicillium chrysogenum
<400> 54
   agctttgacg ctagattgga gatg 24
<210> 55
   <211> 24
   <212> DNA
   <213> Penicillium chrysogenum
<400> 55
   caagcaagcc atctcaacaa gtgc 24
<210> 56
   <211> 70
   <212> DNA
   <213> Saccharomyces cerevisiae
<400> 56
   tactcgctgt attgaaagga tcaaaagacc aaagaccacc aggaataatg ccagctgaag 60
   cttcgtacgc 70
<210> 57
   <211> 73
   <212> DNA
   <213> Saccharomyces cerevisiae
<400> 57
   atgagaagag taacattaga aaacaagtgc agagcatatt ctgtgcatct agcataggcc 60
   actagtggat ctg 73
<210> 58
   <211> 1715
   <212> DNA
   <213> Artificial sequence
<220>
   <223> tif35::loxP-KanMX4-loxP deletion cassette
<400> 58
   tactcgctgt attgaaagga tcaaaagacc aaagaccacc aggaataatg ccagctgaag 60
   cttcgtacgc tgcaggtcga caacccttaa tataacttcg tataatgtat gctatacgaa 120
   gttattaggt ctagagatct gtttagcttg cctcgtcccc gccgggtcac ccggccagcg 180
   acatggaggc ccagaatacc ctccttgaca gtcttgacgt gcgcagctca ggggcatgat 240
   gtgactgtcg cccgtacatt tagcccatac atccccatgt ataatcattt gcatccatac 300
   attttgatgg ccgcacggcg cgaagcaaaa attacggctc ctcgctgcag acctgcgagc 360
   agggaaacgc tcccctcaca gacgcgttga attgtcccca cgccgcgccc ctgtagagaa 420
   atataaaagg ttaggatttg ccactgaggt tcttctttca tatacttcct tttaaaatct 480
   tgctaggata cagttctcac atcacatccg aacataaaca accatgggta aggaaaagac 540
   tcacgtttcg aggccgcgat taaattccaa catggatgct gatttatatg ggtataaatg 600
   ggctcgcgat aatgtcgggc aatcaggtgc gacaatctat cgattgtatg ggaagcccga 660
   tgcgccagag ttgtttctga aacatggcaa aggtagcgtt gccaatgatg ttacagatga 720
   gatggtcaga ctaaactggc tgacggaatt tatgcctctt ccgaccatca agcattttat 780
   ccgtactcct gatgatgcat ggttactcac cactgcgatc cccggcaaaa cagcattcca 840
   ggtattagaa gaatatcctg attcaggtga aaatattgtt gatgcgctgg cagtgttcct 900
   gcgccggttg cattcgattc ctgtttgtaa ttgtcctttt aacagcgatc gcgtatttcg 960
   tctcgctcag gcgcaatcac gaatgaataa cggtttggtt gatgcgagtg attttgatga 1020
   cgagcgtaat ggctggcctg ttgaacaagt ctggaaagaa atgcataagc ttttgccatt 1080
   ctcaccggat tcagtcgtca ctcatggtga tttctcactt gataacctta tttttgacga 1140
   ggggaaatta ataggttgta ttgatgttgg acgagtcgga atcgcagacc gataccagga 1200
   tcttgccatc ctatggaact gcctcggtga gttttctcct tcattacaga aacggctttt 1260
   tcaaaaatat ggtattgata atcctgatat gaataaattg cagtttcatt tgatgctcga 1320
   tgagtttttc taatcagtac tgacaataaa aagattcttg ttttcaagaa cttgtcattt 1380
   gtatagtttt tttatattgt agttgttcta ttttaatcaa atgttagcgt gatttatatt 1440
   ttttttcgcc tcgacatcat ctgcccagat gcgaagttaa gtgcgcagaa agtaatatca 1500
   tgcgtcaatc gtatgtgaat gctggtcgct atactgctgt cgattcgata ctaacgccgc 1560
   catccagtgt cgaaaacgag ctctcgagaa cccttaatat aacttcgtat aatgtatgct 1620
   atacgaagtt attaggtgat atcagatcca ctagtggcct atgctagatg cacagaatat 1680
   gctctgcact tgttttctaa tgttactctt ctcat 1715
<210> 59
   <211> 40
   <212> DNA
   <213> Saccharomyces cerevisiae
<400> 59
   gaataaaaaa gagctcacgc tttttcagtt cgagtttatc 40
<210> 60
   <211> 41
   <212> DNA
   <213> Saccharomyces cerevisiae
<400> 60
   gttaatagca actctaacca tggtttgttt gtttatgtgt g 41
<210> 61
   <211> 26
   <212> DNA
   <213> Saccharomyces cerevisiae
<400> 61
   gcttatggtg gtggtgcttc ttatag 26
<210> 62
   <211> 35
   <212> DNA
   <213> Saccharomyces cerevisiae
<400> 62
   agagggtacc tgtgcatcta ttccttaacc ttagg 35
<210> 63
   <211> 24
   <212> DNA
   <213> Saccharomyces cerevisiae
<400> 63
   agtacggtca ttggacctgg aatc 24
<210> 64
   <211> 24
   <212> DNA
   <213> Saccharomyces cerevisiae
<400> 64
   cgttccatgc acctccatga atgt 24
<210> 65
   <211> 20
   <212> DNA
   <213> Penicillium chrysogenum
<400> 65
   ccaccgttgt ccgcgaacat 20
<210> 66
   <211> 20
   <212> DNA
   <213> Penicillium chrysogenum
<400> 66
   tccttctcgg cctccttagc 20
<210> 67
   <211> 20
   <212> DNA
   <213> Penicillium chrysogenum
<400> 67
   ctggcggtat ccacgtcacc 20
<210> 68
   <211> 20
   <212> DNA
   <213> Penicillium chrysogenum
<400> 68
   aggccagaat ggatccaccg 20
<210> 69
   <211> 20
   <212> DNA
   <213> Aspergillus nidulans
<400> 69
   ggctggctgt tagtcaactg 20
<210> 70
   <211> 20
   <212> DNA
   <213> Aspergillus nidulans
<400> 70
   aggaggctga cctcgattgt 20

## Description

### Field of the invention

The present invention relates to a vector-host system, a host cell, a method for the production of a vector-host system, a method for the production of a biological compound of interest, a method for screening for a polynucleotide encoding a biological compound of interest and a method for use of the host cell and of the vector-host system.

### Background of the invention

In prokaryotes plasmids, circular DNA molecules that replicate autonomously independent from the host genome, have been the workhorses in both fundamental and biotechnological studies aimed to understand cellular processes, or to produce commercially interesting products such as enzymes, metabolites etc. However, unlike some naturally occurring plasmids, constructed plasmids in bacteria are inherently unstable and require selection (e.g. an auxotrophic marker, a dominant growth marker or an antibiotic resistance marker) to be retained in the cell at a satisfactory level. Consequently, the presence of a plasmid in the cell dictates the medium composition or requires continuous use of (expensive) antibiotics. This feature is not only true for prokaryotes, but also for the limited number of eukaryotes, mainly yeast species and a few filamentous fungi, where autonomously replicating plasmids have so far been utilized with some success. In the budding yeast *Saccharomyces cerevisiae* plasmid vectors either contain the replicon of the naturally occurring 2 µM plasmid or an autonomously replicating sequence (ARS) isolated from chromosomal DNA. Naturally occurring plasmid origins and organism-specific ARS sequences have also been used as plasmid replicons in some other yeast species. However, in filamentous fungi the approaches used to develop autonomously replicating plasmid vectors in yeast have met with little success, as exemplified by the observation that the 2 µM replicon does not function in these organisms. Consequently, only limited use is made of replicating plasmids in filamentous fungi.

The most utilized plasmid replicon in the ascomycete *Aspergillus nidulans* that has been shown to function also in other Aspergilli, various *Penicillium* species as well as in *Gibberella fujikuroi* and *Trichoderma reesei,* is the *AMA1* replicon (Gems et al., 1991 Gene. 98(1):61-7). This replicon represents a rearranged chromosomal *A. nidulans* DNA fragment that enables plasmids carrying an auxotrophic marker (like *pyrG -* uracil) or a dominant marker (like *amdS-*N-source or *ble*^{R} *-* phleomycine) to replicate at multiple copies per chromosome (Fierro et al., 1996 Curr Genet. 29(5):482-9). However, similar to bacterial plasmids, *AMA1*-based vectors are mitotically notoriously unstable, and growth on rich media (in case of *pyrG* and *amdS*) or growth on antibiotic-free medium (in case of *ble*^{R}) results in rapid loss of the plasmid from the cells. In many cases, industrial fermentations exploit non-selective complex media that do not allow the use of such mitotically unstable plasmids. As a consequence, in most production systems employing filamentous fungi, expression cassettes have routinely been ectopically integrated into the host genome, or were occasionally inserted via targeted integration (e.g. at the *niaD* locus allowing selection on chlorate resistance). The recent availability of mutants deficient in orthologs of the mammalian KU70 and KU80 proteins involved in non-homologous end joining (NHEJ) has enabled highly efficient targeted integration strategies in many filamentous fungi (Ninomiya et al., 2004; Proc Natl Acad Sci USA. 101:12248-53). However, ultimate expression remains locus-dependent and the identification of loci that allow efficient expression time-consuming.

Hagg et al.(Journal of Biotechnology, 2004, 111(1): pages 17-30) disclose a host plasmid system for stable plasmid maintenance in *Escherichia coli.* The plasmid carries the essential *infA* gene, which is deleted form the chromosome of the host strain, and an origin of replication. Increased stability was tested using a model system wherein the *bla* gene was introduced in the plasmid.

WO03/000881 A2 discloses that in bacteria a selection marker on a plasmid is replaced by an essential gene in order to complement the essential gene missing from the genome of the bacterium.

Hanachi (Journal of Biological Chemistry, 1999, 274(13): pages 8546-8553) discloses cloning and characterisation of *tif35* gene encoding the p33 subunit of Eukaryotic Translation Initiation Factor-3 from S. *cerevisiae.*

Carvalho et al. (Applied Microbiology and Biotechnology 2010, 87(4): pages 1463-1473) disclose autonomously replicating vectors useful in filamentous fungi containing either the *pyrG* allele or a hygromycin resistance gene as selectable markers.

WO2008138835 A1 discloses a method for producing a compound of interest in filamentous fungi wherein a library of genes introduced in fungal host cell has been screened for a gene of interest, wherein the gene is located in a plasmid having a fungal replication initiator sequence and promoter allowing expression of the protein of interest, wherein the host cell has a decreased homologous recombination efficiency.

It would thus be very advantageous if stabilization of autonomously replicating plasmids in industrial host cells could be enhanced such that non-selective, antibiotic-free media can be used.

### Summary of the invention

The present invention is defined by the appended claims.

According to the present specification, there is disclosed a host cell deficient in an essential gene, comprising a vector, said vector comprising at least said essential gene and an autonomous replication sequence, wherein the host cell is a filamentous fungal cell.

The specification also discloses a host cell deficient in an essential gene, comprising a vector, said vector comprising at least said essential gene and an autonomous replication sequence, wherein the host cell comprises a recombinant polynucleotide construct comprising a polynucleotide encoding a biological compound of interest or a compound involved in the synthesis of a biological compound of interest.

A vector-host system comprising a host cell as defined herewith is also disclosed.

The specification further discloses a method for the production of a host cell or of a vector-host system, which method comprises:
a. providing a host cell and a vector, which comprises at least a gene essential for said host cell and an autonomous replication sequence,
b. co-transforming the host cell with the vector and a disruption construct for said essential gene to render the host cell deficient in the essential gene.

The invention provides:
- a method for the production of a biological compound of interest comprising culturing a filamentous fungal host cell deficient in an essential gene, comprising a vector, said vector comprising at least said essential gene and an autonomous replication sequence, wherein the host cell comprises a recombinant polynucleotide construct comprising a polynucleotide encoding a biological compound of interest or a compound involved in the synthesis of a biological compound of interest under conditions conducive to the production of the biological compound of interest and isolating the compound of interest from the culture broth; wherein a recombinant polynucleotide construct encoding the biological compound of interest or a compound involved in the synthesis of the biological compound of interest is located on said vector.

The invention also provides:
- a method for the production of a biological compound of interest comprising producing a filamentous fungal host cell according to a method which comprises:
   a. providing a filamentous fungal host cell and a vector which comprises at least a gene essential for said host cell and an autonomous replication sequence, wherein a recombinant polynucleotide construct encoding the compound of interest or a compound involved in the synthesis of the biological compound of interest is located on said vector; and
   b. co-transforming the host cell with the vector and a disruption construct for said essential gene to render the host cell deficient in the essential gene;
culturing such a host cell under conditions conducive to the production of the biological compound of interest and isolating the compound of interest from the culture broth.

The specification further discloses:
- a method for the production of a biological compound of interest comprising:
   a. providing a host cell, said host cell being deficient in an essential gene, said host cell comprising a vector, said vector comprising at least said essential gene and an autonomous replication sequence, wherein the host cell is (i) a filamentous fungal cell or (ii) a host cell which comprises a recombinant polynucleotide construct comprising a polynucleotide encoding a biological compound of interest or a compound involved in the synthesis of a biological compound of interest,
   b. optionally providing said host cell with a recombinant polynucleotide construct comprising a polynucleotide encoding a biological compound of interest or compound involved in the synthesis of a biological compound of interest,
   c. culturing the host cell under conditions conducive to the production of the biological compound of interest, and optionally
   d. isolating the biological compound of interest from the culture broth.

The present specification discloses:
- a method for screening for a polynucleotide encoding a biological compound of interest comprising:
   a. providing a library of polynucleotides possibly containing a polynucleotide encoding a biological compound of interest,
   b. providing a multiplicity of individual host cells, said host cell being deficient in an essential gene and comprising a vector, said vector comprising at least said essential gene and an autonomous replication sequence, wherein said host cell is (i) a filamentous fungal cell or (ii) a host cell which comprises a recombinant polynucleotide construct comprising a polynucleotide encoding a biological compound of interest or a compound involved in the synthesis of a biological compound of interest,
   c. screening the transformants for expression of the biological compound of interest;
- a method for screening for a polynucleotide encoding a biological compound of interest comprising:
   a. providing a library of polynucleotides possibly containing a polynucleotide encoding a biological compound of interest,
   b. providing a multiplicity of individual host cells, said host cell being deficient in an essential gene and comprising a vector, said vector comprising at least said essential gene and an autonomous replication sequence, wherein said host cell is prepared according to the method of the invention,
   c. screening the transformants for expression of the biological compound of interest.

The present specification discloses:
- use of the vector-host system according to the specification for the production of a biological compound of interest;
- use of the vector-host system according to the specification for expression of a polynucleotide encoding a compound of interest;
- use of a host cell deficient in an essential gene to stabilize a vector, said vector comprising at least said essential gene and an autonomous replication sequence; and
- use of a host cell to stabilize a vector-host system, said host cell being a host cell prepared according to the method disclosed herein.

### Brief description of the Figures

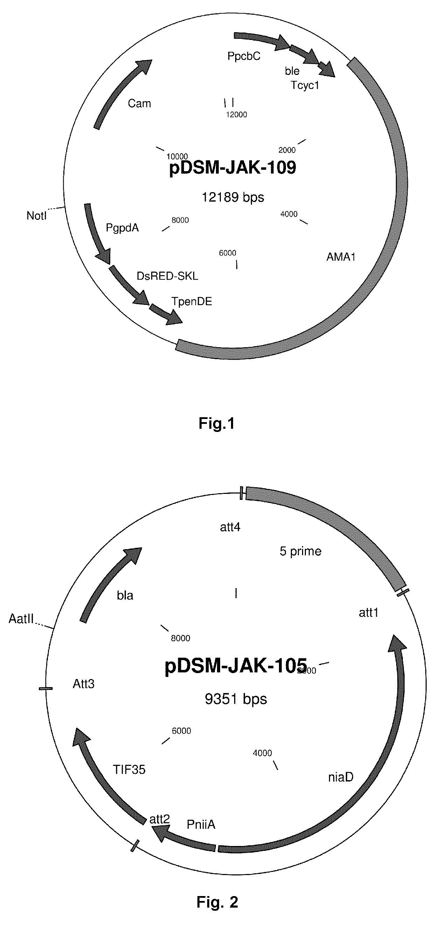

Fig.1 *P. chrysogenum* / *E. coli* shuttle plasmid pDSM-JAK-109, comprising the *AMA1* region as derived from pAMPF21*, a constitutively expressed *DsRed.SKL* cassette and a phleomycin-resistance cassette.
Fig. 2 plasmid pDSM-JAK-105, comprising a recombination cassette that enables replacement of the promoter of the *P. chrysogenum tif35* gene by the nitrate-inducible P. *chrysogenum niiA* promoter as well as the *P. chrysogenum niaD* gene that is used as selection marker.

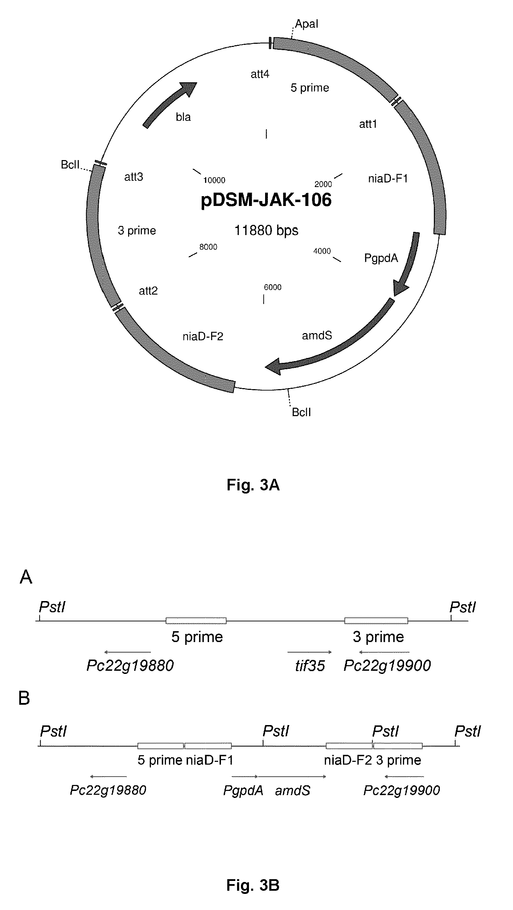

Fig. 3 A) pDSM-JAK-106, comprising a recombination cassette that enables to replace the genomic *P. chrysogenum tif35* gene including its regulatory regions by an *amdS* expression cassette. The *niaD-F1* and *niaD-F2* regions flanking the *amdS* expression cassette represent a direct repeat that can be used to remove the marker from the genome again using fluoroacetamide counterselection.
B) the *P. chrysogenum tif35* deletion cassette of plasmid pDSM-JAK-106.

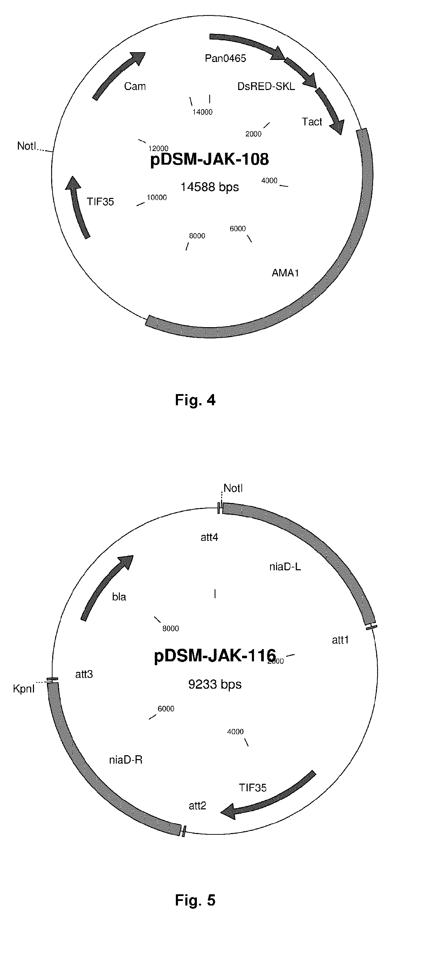

Fig. 4 plasmid pDSM-JAK-108 comprising the *AMA1* region as derived from pAMPF21*, the *DsRed.SKL* expression cassette and the *P. chrysogenum tif35* expression cassette. Note that pDSM-JAK-108 comprises no significant homology to *A*. *niger* and *P*. *chrysogenum* (when lacking the *tif35* gene) chromosomal DNA sequences.
Fig. 5 plasmid pDSM-JAK-116, comprising a recombination cassette that enables integration of the *P*. *chrysogenum tif35* gene with its own regulatory sequences at the *niaD* locus in the *P*. *chrysogenum* genome. Selection is based on absence of nitrate reductase activity that causes resistance towards chlorate.

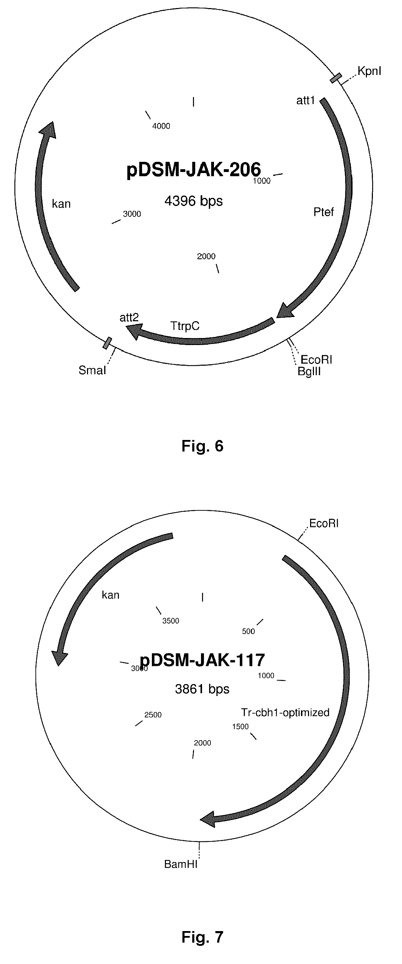

Fig. 6 plasmid pDSM-JAK-206, pDONR221 derivative containing the *A*. *nidulans tef* promoter and the *A*. *nidulans trpC* terminator regions.
Fig. 7 plasmid pDSM-JAK-117, pMK-RQ derivative containing the synthetic codon pair-optimized *T. reesei cbh1* cDNA.

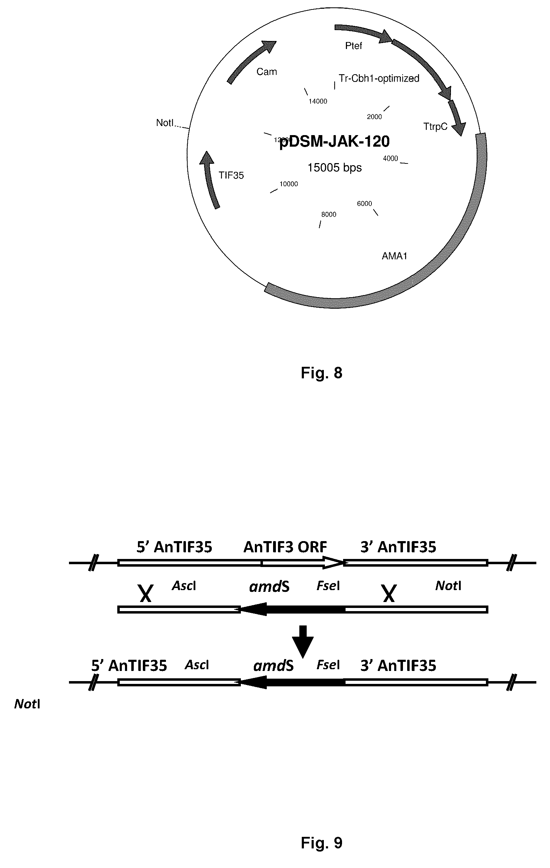

Fig. 8 plasmid pDSM-JAK-120 comprising the *AMA1* region of plasmid pAMPF21*, the *P*. *chrysogenum tif35* expression cassette and the codon pair-optimized *T. reesei cbh1* expression cassette.
Fig. 9 the *A*. *niger tif35* deletion cassette. Upon double homologous recombination (illustrated by x) between the fragment and the genomic DNA, the *Anig.tif35* gene (including part of the upstream promoter region) was effectively replaced by the *amdS* selection marker.

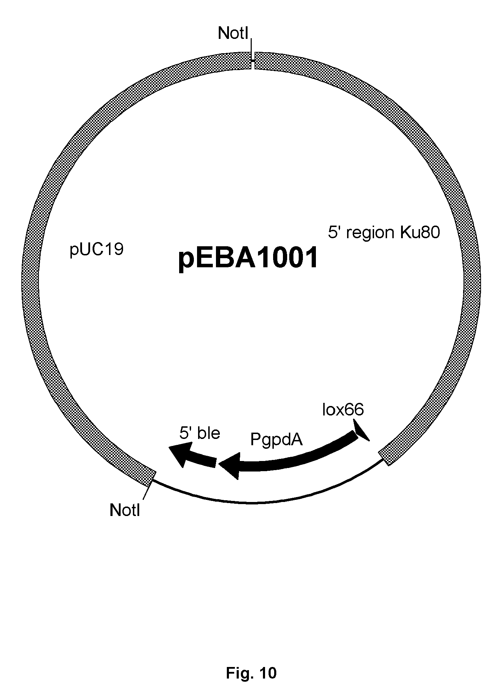

Fig. 10 depicts the pEBA1001 vector. Part of the vector fragment was used in bipartite gene-targeting method in combination with the pEBA1002 vector with the goal to delete the *ReKu80* ORF in *Rasamsonia emersonii.* The vector comprises a 2500 bp 5' upstream flanking region, a Iox66 site, the 5' part of the ble coding sequence driven by the *A.nidulans gpdA* promoter and the backbone of pUC19 (Invitrogen, Breda, The Netherlands). The *E. coli* DNA was removed by digestion with restriction enzyme Notl, prior to transformation of the *R. emersonii* strains.

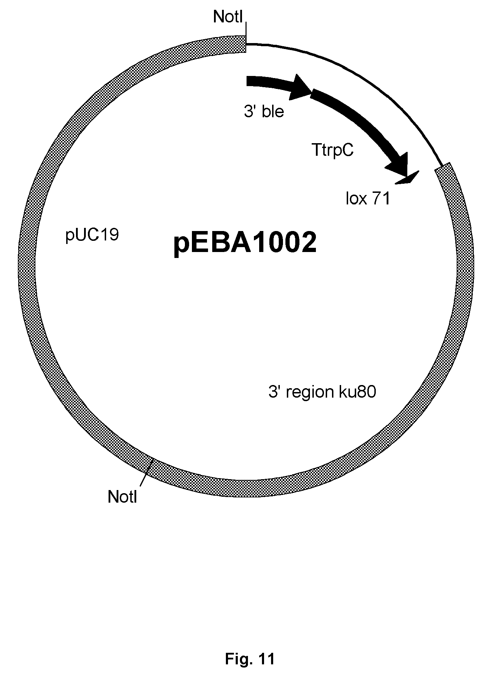

Fig. 11 depicts the pEBA1002 vector. Part of the vector fragment was used in bipartite gene-targeting method in combination with the pEBA1001 vector with the goal to delete the *ReKu80* ORF in *Rasamsonia emersonii.* The vector comprises the 3' part of the ble coding region, the *A.nidulans trp*C terminator, a Iox71 site, a 2500 bp 3' downstream flanking region of the *ReKu80* ORF, and the backbone of pUC19 (Invitrogen, Breda, The Netherlands). The *E. coli* DNA was removed by digestion with restriction enzyme Notl, prior to transformation of the *R. emersonii* strains.

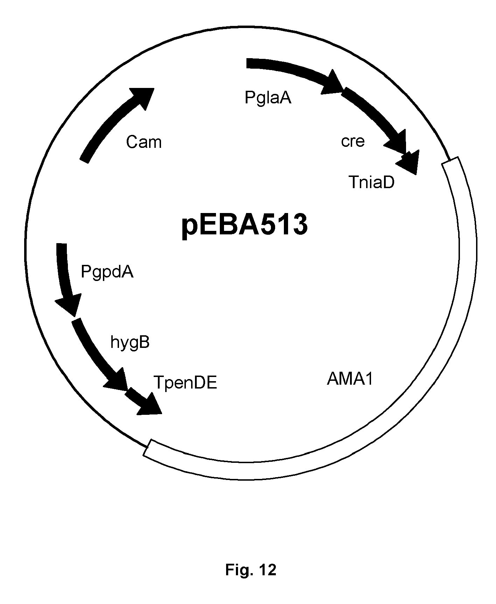

Fig. 12 depicts a map of pEBA513 for transient expression of cre recombinase in fungi. pEBA513 is a pAMPF21 derived vector containing the *AMA*1 region and the CAT chloramphenicol resistance gene. Depicted are the cre recombinase gene (cre) expression cassette, containing the *A.niger* glaA promoter (Pgla), cre recombinase coding region, and niaD terminator. In addition, the hygromycin resistance cassette consisting of the *A. nidulans* gpdA promoter (PgpdA), hygB coding region and the *P*. *chrysogenum* penDE terminator is indicated.

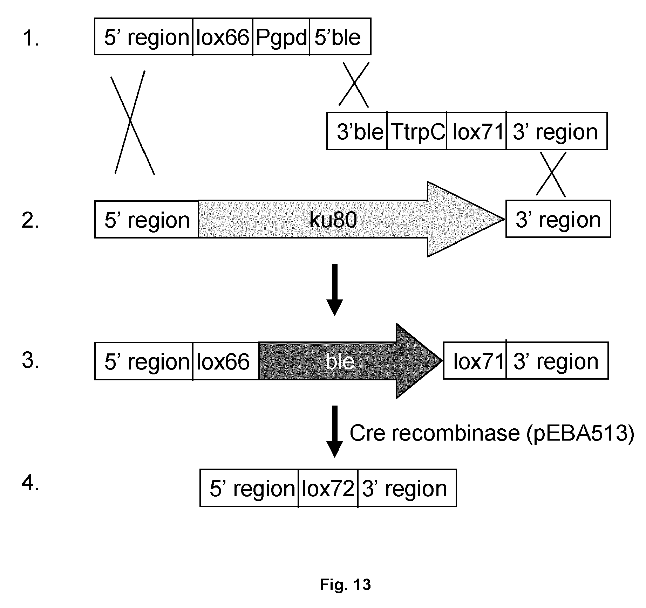

Fig. 13 depicts the strategy used to delete the *ReKu80* gene of *R. emersonii.* The vectors for deletion of *ReKu80* comprise the overlapping non-functional *ble* selection marker fragments (split marker) flanked by IoxP sites and 5' and 3' homologous regions of the *ReKu80* gene for targeting (1). The constructs integrate through triple homologous recombination (X) at the genomic *ReKu80* locus and at the overlapping homologous non-functional ble selection marker fragment (2) and replaces the genomic *ReKu80* gene copy (3). Subsequently, the selection marker is removed by transient expression of cre recombinase leading to recombination between the Iox66 and Iox71 sites resulting in the deletion of the ble gene with a remainder double-mutant Iox72 site left within the genome (4). Using this overall strategy, the *ReKu80* ORF is removed from the genome.

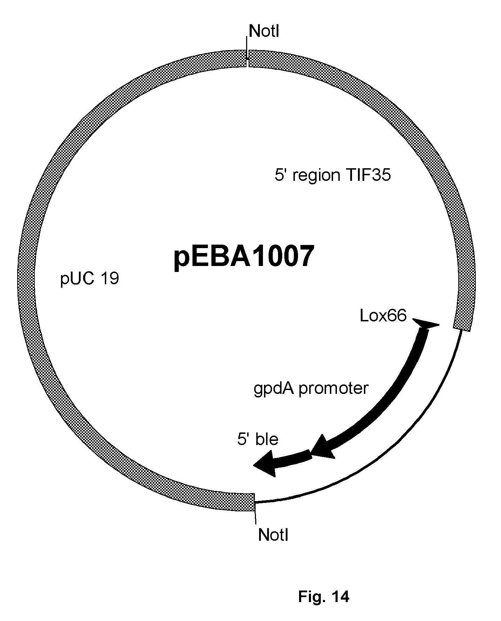

Fig. 14 depicts the pEBA1007 vector. Part of the vector fragment was used in bipartite gene-targeting method in combination with the pEBA1008 vector with the goal to delete the *ReTif35* ORF in *Rasamsonia emersonii.* The vector comprises a 1500 bp 5' upstream flanking region of the *ReTif35* ORF, a Iox66 site, the 5' part of the ble coding region driven by the *A.nidulans gpdA* promoter and the backbone of pUC19 (Invitrogen, Breda, The Netherlands). The *E. coli* DNA was removed by digestion with restriction enzyme Notl, prior to transformation of the *R. emersonii* strains.

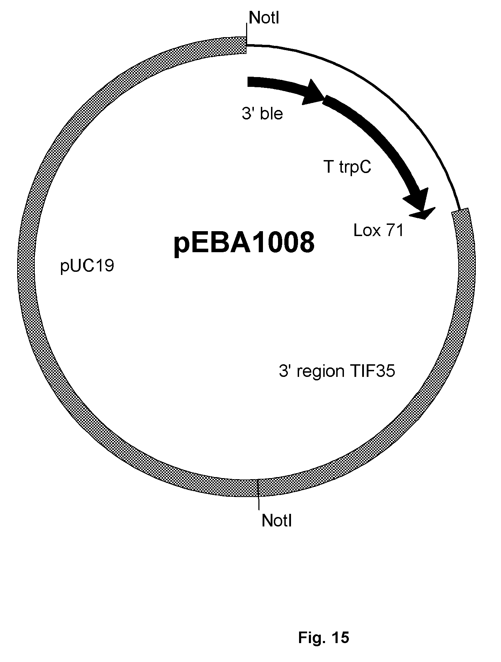

Fig. 15 depicts the pEBA1008 vector. Part of the vector fragment was used in bipartite gene-targeting method in combination with the pEBA1007 vector with the goal to delete the *ReTif35* ORF in Rasamsonia *emersonii.* The vector comprises the 3' part of the ble coding region, the *A.nidulans trp*C terminator, a Iox71 site, a 1500 bp 3' downstream flanking region of the *ReTif35* ORF, and the backbone of pUC19 (Invitrogen, Breda, The Netherlands). The *E. coli* DNA was removed by digestion with restriction enzyme Notl, prior to transformation of the *R. emersonii* strains.

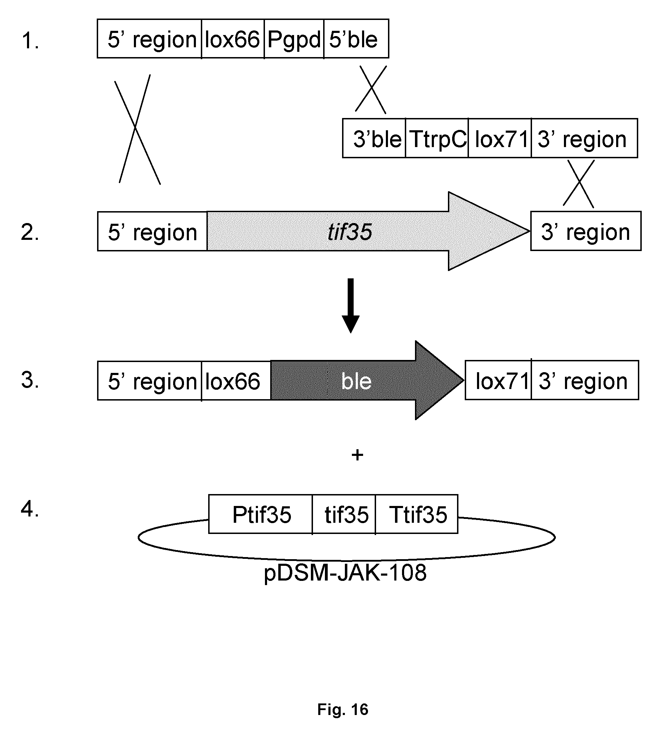

Fig. 16 depicts the strategy used to delete the *ReTif35* gene of *R. emersonii.* The vectors for deletion of *ReTif35* comprise the overlapping non-functional *ble* selection marker fragments (split marker) flanked by IoxP sites and 5' and 3' homologous regions of the *ReTif35* gene for targeting (1). The constructs integrate through triple homologous recombination **(X)** at the genomic *ReTif35* locus and at the overlapping homologous non-functional ble selection marker fragment (2) and replaces the genomic *ReTif35* gene copy (3). Essential gene *Pchr•tif35* is expressed from pDSM-JAK-108 (4).

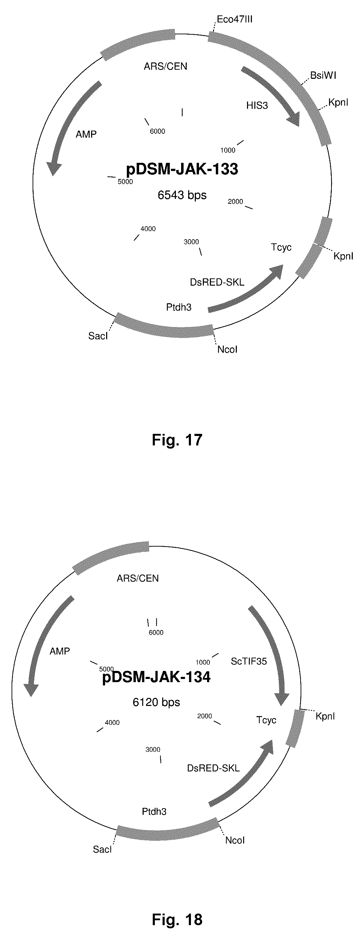

Fig. 17 depicts the pDSM-JAK-133 vector, an *E. coli* / *S*. *cerevisiae* shuttle vector, which contains the *Scer•HIS3* auxotrophic marker, the *ARS*/*CEN* replicon and the *DsRed*.*SKL* gene expressed by the *Scer•TDH3 promoter.*
Fig. 18 depicts the pDSM-JAK-134 vector, a derivative of pDSM-JAK-133 in which the entire *Scer•HIS3* auxotrophic marker was replaced by the *Scer•TIF35 gene.*

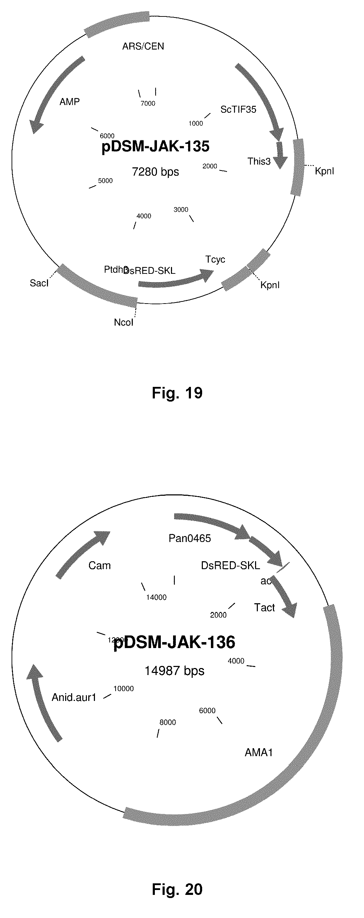

Fig. 19 depicts the pDSM-JAK-135 vector, a derivative of pDSM-JAK-133 in which part of the *Scer•HIS3* auxotrophic marker was replaced by the *Scer•TIF35 gene.*
Fig. 20 depicts the pDSM-JAK-136 vector comprising the *AMA1* region as derived from pAMPF21*, the *DsRed.SKL* expression cassette and the *A. nidulans tif35* gene with its own regulatory sequences. Note that pDSM-JAK-136 comprises no significant homology to *A. niger, P. chrysogenum* and *R. emersonii* chromosomal DNA sequences (except *aur1*).

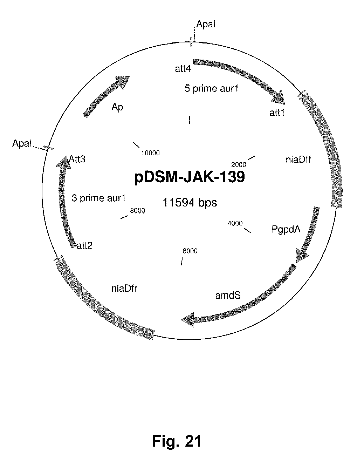

Fig. 21 depicts the pDSM-JAK-139 vector comprising a recombination cassette that enables to replace the genomic *P. chrysogenum aur1* gene by an *amdS* expression cassette. The *niaD-F1* and *niaD-F2* regions flanking the *amdS* expression cassette represent a direct repeat that can be used to remove the marker from the genome again using fluoroacetamide counterselection.

### Detailed description of the invention

It has now surprisingly been demonstrated that a vector-host system can be produced where the stability of the vector comprising an autonomous replication sequence is substantially increased.

The present invention relates to a method for the production of a biological compound of interest comprising culturing
a filamentous fungal host cell deficient in an essential gene, comprising a vector, said vector comprising at least said essential gene and an autonomous replication sequence, wherein the host cell comprises a recombinant polynucleotide construct comprising a polynucleotide encoding a biological compound of interest or a compound involved in the synthesis of a biological compound of interest
under conditions conducive to the production of the biological compound of interest and
isolating the compound of interest from the culture broth,
wherein a recombinant polynucleotide construct encoding the biological compound of interest or a compound involved in the synthesis of the biological compound of interest is located on said vector.

The present invention also relates to a method for the production of a biological compound of interest comprising
producing a filamentous fungal host cell according to a method which comprises:
a. providing a filamentous fungal host cell and a vector which comprises at least a gene essential for said host cell and an autonomous replication sequence, wherein a recombinant polynucleotide construct encoding the compound of interest or a compound involved in the synthesis of the biological compound of interest is located on said vector; and
b. co-transforming the host cell with the vector and a disruption construct for said essential gene to render the host cell deficient in the essential gene;
culturing such a host cell under conditions conducive to the production of the biological compound of interest and
isolating the compound of interest from the culture broth.

Accordingly, the present specification discloses a vector-host system wherein the host is deficient in an essential gene and wherein the vector comprises at least said essential gene and an autonomous replication sequence.

One great advantage of the system is that it provides for a reliable and versatile system which has increased stability compared to state of the art vector host systems under all relevant conditions, i.e. during sporulation, germination and industrial production processes. Another advantage is that it is possible to work with one vector in various different hosts. Yet another advantage is that it can be used with all kinds of culture media, including complex (also referred to as undefined) medium. All these advantages make it a very versatile system.

The vector may be any vector (e.g. a plasmid or a virus), which can be conveniently subjected to recombinant DNA procedures. The choice of the vector will typically depend on the compatibility of the vector with the host cell into which the vector is to be introduced. Preferably, the vector is a plasmid. The vector may be a linear or a closed circular plasmid. The vector may further comprise a, preferably non-selective, marker that allows for easy determination of the vector in the host cell. Suitable markers include GFP and DsRed. The chance of gene conversion or integration of the vector into the host genome must be minimized. The person skilled in the art knows how to construct a vector with minimal chance of integration into the genome. In one embodiment, the vector lacks significant similarity with the genome of the host to minimize the chance of integration into the host genome. This may be achieved by using control sequences, such as promoters and terminators, which originate from another species than the host species. In one embodiment, control sequences from *A. nidulans* are used for a vector which is used in a vector-host system in fungi, in particular a filamentous fungus other than *A. nidulans.* A suitable example of such a vector is plasmid pDSM-JAK-108 as presented in Fig. 4 of this application. The vectors of the vector-host system of the present invention are also encompassed herewith.

Deficiency of a host cell is herein defined as a phenotypic feature, wherein the cell produces less of the product encoded by the essential gene, has a reduced expression level of the mRNA transcribed from the essential gene or has a decreased specific (protein) activity of the product encoded by the essential gene and combinations of these possibilities, as compared to the parent cell which is not deficient in the essential gene. The host cell is typically deficient in view of a genetic lesion in an essential gene resulting in partial or complete non-functionality of the essential gene

Clearly, a host cell with a complete deficiency in an essential gene cannot exist. Thus, a host cell as disclosed herewith having a complete deficiency in an essential gene is one which can exist only when it harbours a vector of the invention.

A host cell as disclosed herewith thus carries a modification in its genome such that the host cell is partially or completely non-functional for an essential gene and a vector comprising a functional essential gene and autonomous replication sequence. That is to say, the invention discloses a host cell being changed in its genome or endogenous genetic traits/DNA resulting reduced- or non-functionality of an essential gene and a vector comprising a functional essential gene and autonomous replication sequence.

Deficiency can be measured using any assay available to the skilled person, such as transcriptional profiling, Northern blotting, Southern blotting and Western blotting.

Deficiency of the host cell deficient in the essential gene is preferably measured relative to the parent cell that is not deficient in the essential gene. Preferably, the deficiency of the host cell, wherein said host cell produces at least 10% less of the product encoded by the essential gene, has an at least 10% reduced expression level of the mRNA transcribed from the essential gene or has an at least 10% decreased specific (protein) activity of the product encoded by the essential gene as compared to the parent cell which is not deficient in the essential gene. More preferably, the deficiency is at least 20%, even more preferably at least 30%, even more preferably at least 40%, even more preferably at least 50%, even more preferably at least 60%, even more preferably at least 70%, even more preferably at least 75%, even more preferably at least 80%, even more preferably at least 85%, even more preferably at least 90%, even more preferably at least 95%, even more preferably at least 96%, even more preferably at least 97%, even more preferably at least 98%, even more preferably at least 99%, even more preferably at least 99.5%, even more preferably at least 99.9% and most preferably the deficiency is complete, i.e. 100%.

The vector-host system disclosed herewith has increased stability of the vector comprising an autonomous replication sequence. The stability is preferably measured relative to a vector-host system, wherein the vector is identical but the host is not deficient in the essential gene. The stability is preferably determined comparing the loss of the vector in subsequent cycles of sporulation and single colony isolation on plates with non-selective solid medium. The higher the stability, the longer it will take before the vector is lost from the host cell, in particular on non-selective solid medium, such as complex or undefined medium. In the system, the vector is maintained for at least four subsequent cycles of sporulation. Preferably, the vector is maintained for at least five, at least six, at least seven, at least eight, at least nine or at least ten subsequent cycles of sporulation. More preferably, the vector is maintained for at least 15, at least 20, at least 25, at least 30, at least 40, at least 50, at least 60 or at least 70 subsequent cyles of sporulation. If the vector comprises a non-selective colour marker such as GFP or DsRed, presence of the vector in the host can easily be observed by presence of the colour of the marker in the colonies.

Preferably, the increase in stability of the vector-host system disclosed herewith compared to a vector-host system wherein the vector is identical but the host cell is not deficient in the essential gene is at least a two-fold increase, more preferably at least a three-fold increase, more preferably at least a five-fold increase, more preferably at least a ten-fold increase, more preferably at least a twenty-fold increase, more preferably at least a fifty-fold increase, more preferably at least a hundred-fold increase, more preferably at least a two-hundred-fold increase, more preferably at least a five hundred-fold increase and most preferably at least a thousand-fold increase.

The deficient host cell is preferably obtained by modification of the essential gene in the genome of the parent host cell. That is to say, deficient host cell disclosed herewith is a cell which is modified in its genome so that is is partially or completely non-functional for an essential gene.

Said modification of the essential gene in the genome of the host cell is herein defined as any event resulting in a change or addition in the polynucleotide sequence in the cell in the genome of the cell. A modification is construed as one or more modifications. Modification may be accomplished by the introduction (insertion), substitution or removal (deletion) of one or more nucleotides in a nucleotide sequence. This modification may for example be in a coding sequence or a regulatory element required for the transcription or translation of the polynucleotide. For example, nucleotides may be inserted or removed so as to result in the introduction of a stop codon, the removal of a start codon or a change or a frame-shift of the open reading frame of a coding sequence. The modification of a coding sequence or a regulatory element thereof may be accomplished by site-directed or random mutagenesis, DNA shuffling methods, DNA reassembly methods, gene synthesis (see for example Young and Dong, (2004), Nucleic Acids Research 32*,* Gupta et al. (1968), Proc. Natl. Acad. Sci USA, 60: 1338-1344*;* Scarpulla et al. (1982), Anal. Biochem. 121: 356-365; Stemmer et al. (1995), Gene 164: 49-53), or PCR generated mutagenesis in accordance with methods known in the art. Examples of random mutagenesis procedures are well known in the art, such as for example chemical (NTG for example) mutagenesis or physical (UV for example) mutagenesis. Examples of directed mutagenesis procedures are the QuickChange™ site-directed mutagenesis kit (Stratagene Cloning Systems, La Jolla, CA), the 'The Altered Sites^{®} II in vitro Mutagenesis Systems' (Promega Corporation) or by overlap extension using PCR as described in Gene. 1989 Apr 15;77(1):51-9. (Ho SN, Hunt HD, Horton RM, Pullen JK, Pease LR "Site-directed mutagenesis by overlap extension using the polymerase chain reaction") or using PCR as described in Molecular Biology: Current Innovations and Future Trends. (Eds. A.M. Griffin and H.G.Griffin. ISBN 1-898486-01-8;1995 Horizon Scientific Press, PO Box 1, Wymondham, Norfolk, U.K.).

A modification in the genome can be determined by Southern blotting or by comparing the DNA sequence of the modified cell to the sequence of the non-modified cell. Sequencing of DNA and genome sequencing can be done using standard methods known to the person skilled in the art, for example using Sanger sequencing technology and/or next generation sequencing technologies such as Illumina GA2, Roche 454, and the like, as reviewed in Elaine R. Mardis (2008), Next-Generation DNA Sequencing Methods, Annual Review of Genomics and Human Genetics 9: 387-402.

Preferred methods of modification are based on techniques of gene replacement, gene deletion, or gene disruption.

For example, in case of replacement of a polynucleotide, nucleic acid construct or expression cassette, an appropriate DNA sequence may be introduced at the target locus to be replaced. The appropriate DNA sequence is preferably present on a cloning vector. Preferred integrative cloning vectors comprise a DNA fragment, which is homologous to the polynucleotide or has homology to the polynucleotides flanking the locus to be replaced for targeting the integration of the cloning vector to this pre-determined locus. In order to promote targeted integration, the cloning vector is preferably linearized prior to transformation of the cell. Preferably, linearization is performed such that at least one but preferably either end of the cloning vector is flanked by sequences homologous to the DNA sequence (or flanking sequences) to be replaced. This process is called homologous recombination and this technique may also be used in order to achieve (partial) gene deletion or gene disruption.

For example, for gene disruption, a polynucleotide corresponding to the endogenous polynucleotide may be replaced by a defective polynucleotide, that is a polynucleotide that fails to produce a (fully functional) protein. By homologous recombination, the defective polynucleotide replaces the endogenous polynucleotide. It may be desirable that the defective polynucleotide also encodes a marker, which may be used for selection of transformants in which the nucleic acid sequence has been modified.

Alternatively or in combination with other mentioned techniques, a technique based on *in vivo* recombination of cosmids in *E. coli* can be used, as described in: A rapid method for efficient gene replacement in the filamentous fungus Aspergillus nidulans (2000) Chaveroche, M-K., Ghico, J-M. and d'Enfert C; Nucleic acids Research, vol 28, no 22*.* Other techniques which may be used to inactivate an essential gene are known to the skilled person and may be employed, including gene silencing, for example by RNA interference (RNAi) or antisense strategies, in which mRNA is degraded.

In order to increase the efficiency of inactivation of the essential gene when transforming the vector as disclosed herewith simultaneously with the disruption construct for the essential gene, the essential gene is preferably placed in the genome between IoxP sites. The Cre/LoxP system has been shown to be functional in various host cells. When Cre is introduced simultaneously with the vector according to the invention, the essential gene will be excised by the Cre/IoxP system. Cre may e.g. be introduced as the encoding polynucleotide on a separate vehicle, as active protein, or may be present as encoding sequence on the vector according to the invention. The IoxP sites flanking the essential gene may be introduced by methods known in the art, such as gene replacement. For convenient gene replacement, together with the essential gene a selectable marker may be placed between IoxP sites in the genome. Such marker will be excised together with the essential gene upon activation of Cre.

Another way by which the efficiency of transformation may be increased is by placing the essential gene in the host genome under the control of an inducible promoter. The inducible promoter may be any inducible promoter suitable for the purpose, be it a chemically or physically induced promoter (such as by temperature or light). The person skilled in the art knows how to select such promoter. In one embodiment, the *niiA* promoter from *Penicillium chrysogenum* is used. This promoter is induced by nitrate but is repressed by ammonium. When culturing on ammonium as the sole N-source in the medium, the host is deficient for the essential gene. When culturing on nitrate as the sole N-source in the medium, the host cell is not deficient in the essential gene. In another embodiment, the *xlnA* promoter from *Aspergillus niger* is used. This promoter is induced by xylose but is repressed by glucose. When culturing on glucose medium, the host is deficient for the essential gene. When culturing on xylose medium, the host cell is not deficient in the essential gene.

High transformation frequency is a particularly useful improvement when large pools of transformants are required. An example of such an application is the construction of expression libraries. A time consuming and labour intensive technique in fungi as much less transformants are obtained compared to well known expression systems such as *E. coli.* Advantages of preparing expression libraries in the intended production organism is the more reliable up scaling and more relevant (post) translational modifications of the host cell. WO2008/000715 provides an efficient method for high throughput transfection of filamentous fungi which method can be used in combination with the methods of the present invention.

The autonomous replication sequence may be any suitable sequence available to the person skilled in the art that it confers to the plasmid replication that is independent of chromosomal replication. Preferably, the autonomous replication sequence is the *AMA1* replicon (Gems et al., 1991 Gene. 98(1):61-7). Telomeric repeats may also result in autonomous replication (In vivo linearization and autonomous replication of plasmids containing human telomeric DNA in Aspergillus nidulans, Aleksenko et al. Molecular and General Genetics MGG, 1998 - Volume 260, Numbers 2-3, 159-164, DOI: 10.1007/s004380050881). CEN/ARS sequences from yeast may also be suitable.

The essential gene is preferably a gene that has not been shown to be non-essential. More preferably, the essential gene is a gene whose deficiency renders the host cell non-viable. More preferably, the essential gene is a gene whose deficiency renders the host cell non-viable under all conditions and on any medium, in particular complex (undefined) medium. An essential gene in the context of the present invention may be a gene that renders the host cell non-viable when another (non-essential) gene has been rendered deficient. Preferably, the essential gene is an essential gene in other host cells as well. In one embodiment, the essential gene is a gene which is essential in fungi. Preferably, the essential gene is essential in filamentous fungi, more preferably, the essential gene is essential in the filamentous fungi belonging to *Penicillium, Aspergillus* and *Rasamsonia*/*Talaromyces.* Suitable examples of classes of essential genes include, but are not limited to, genes involved in DNA synthesis & modification, RNA synthesis & modification, protein synthesis & modification, proteasome function, the secretory pathway, cell wall biogenesis and cell division. In the context of the present application, the essential gene is not a auxotrophic marker (such as *pyrG*), dominant growth marker (such as *nia*D and *amd*S) and dominant resistance marker (such as *ble*). A preferred essential gene is the *tif35* gene encoding the g subunit of translation initiation factor 3, which has an ortholog in all eukaryotes. In one embodiment, the *tif35* gene encoding the g subunit of translation initiation factor 3 from *P. chrysogenum* is used as the essential gene. A further preferred essential gene is the *A. nidulans aur1* gene encoding the enzyme phosphatidylinositol:ceramide phosphoinositol transferase, which is required for sphingolipid synthesis. Thus, in one embodiment, the *aur1* gene encoding the enzyme phosphatidylinositol:ceramide phosphoinositol transferase from *A. nidulans* is used as the essential gene.

Eukaryotic cells have at least two separate pathways (one via homologous recombination (HR) and one via non-homologous recombination (NHR)) through which nucleic acids (in particular DNA) can be integrated into the host genome. The yeast *Saccharomyces cerevisiae* is an organism with a preference for homologous recombination (HR). The ratio of non-homologous to homologous recombination (NHR/HR) of this organism may vary from about 0.07 to 0.007.

WO 02/052026 discloses mutants of S. *cerevisiae* having an improved targeting efficiency of DNA sequences into its genome. Such mutant strains are deficient in a gene involved in NHR (KU70).

Contrary to S. *cerevisiae,* most higher eukaryotes such as filamentous fungal cells up to mammalian cells have a preference for NHR. Among filamentous fungi, the NHR/HR ratio ranges between 1 and more than 100. In such organisms, targeted integration frequency is rather low.

To improve the efficiency of targeted deletion of the essential gene in the genome, it is preferred that the efficiency of homologous recombination (HR) is enhanced in the host cell of the vector-host system according to the invention.
Accordingly, preferably in the vector-host system described herewith, the host cell is, preferably inducibly, increased in its efficiency of homologous recombination (HR). The host cell is preferably decreased in its efficiency of non-homologous recombination (NHR). The ratio of non-homologous recombination / homologous recombination (NHR/HR) will typically be decreased in a preferred host cell of the invention.

Since the NHR and HR pathways are interlinked, the efficiency of HR can be increased by modulation of either one or both pathways. Increase of expression of HR components will increase the efficiency of HR and decrease the ratio of NHR/HR. Decrease of expression of NHR components will also decrease the ratio of NHR/HR The increase in efficiency of HR in the host cell of the vector-host system according to the present specification is preferably depicted as a decrease in ratio of NHR/HR and is preferably calculated relative to a parent host cell wherein the HR and/or NHR pathways are not modulated. The efficiency of both HR and NHR can be measured by various methods available to the person skilled in the art. A preferred method comprises determining the efficiency of targeted integration and ectopic integration of a single vector construct in both parent and modulated host cell. The ratio of NHR/HR can then be calculated for both cell types. Subsequently, the decrease in NHR/HR ration can be calculated. In WO2005/095624, this preferred method is extensively described.

Host cells having a decreased NHR/HR ratio as compared to a parent cell may be obtained by modifying the parent eukaryotic cell by increasing the efficiency of the HR pathway and/or by decreasing the efficiency of the NHR pathway. Preferably, the NHR/HR ratio thereby is decreased at least twice, preferably at least 4 times, more preferably at least 10 times. Preferably, the NHR/HR ratio is decreased in the host cell of the vector-host system according to the specification as compared to a parent host cell by at least 5%, more preferably at least 10%, even more preferably at least 20%, even more preferably at least 30%, even more preferably at least 40%, even more preferably at least 50%, even more preferably at least 60%, even more preferably at least 70%, even more preferably at least 80%, even more preferably at least 90% and most preferably by at least 100%.

According to one embodiment, the ratio of NHR/HR is decreased by increasing the expression level of an HR component. HR components are well-known to the person skilled in the art. HR components are herein defined as all genes and elements being involved in the control of the targeted integration of polynucleotides into the genome of a host, said polynucleotides having a certain homology with a certain pre-determined site of the genome of a host wherein the integration is targeted.

According to an embodiment, the ratio of NHR/HR is decreased by decreasing the expression level of an NHR component. NHR components are herein defined as all genes and elements being involved in the control of the integration of polynucleotides into the genome of a host, irrespective of the degree of homology of said polynucleotides with the genome sequence of the host. NHR components are well-known to the person skilled in the art. Preferred NHR components are a component selected from the group consisting of the homolog or ortholog for the host cell of the vector-host system according to the invention of the yeast genes involved in the NHR pathway: KU70, KU80, RAD50, MRE11, XRS2, LIG4, LIF1, NEJ1 and SIR4 (van den Bosch et al., 2002, Biol. Chem. 383: 873-892 and Allen et al., 2003, Mol. Cancer Res. 1:913-920). Most preferred are one of KU70, KU80, and LIG4 and both KU70 and KU80. The decrease in expression level of the NHR component can be achieved using the methods as described herein for obtaining the deficiency of the essential gene.

Since it is possible that decreasing the expression of components involved in NHR may result in adverse phenotypic effects, it is preferred that in the host cell of the vector-host system disclosed herewith, the increase in efficiency in homologous recombination is inducible. This can be achieved by methods known to the person skilled in the art, for example by either using an inducible process for an NHR component (e.g. by placing the NHR component behind an inducible promoter) or by using a transient disruption of the NHR component, or by placing the gene encoding the NHR component back into the genome.

In order to be able to further engineer the host cell of the vector-host system disclosed herewith, the deficiency of the host cell of the vector-host system may or may not be an inducible deficiency. This can be achieved by methods known to the person skilled in the art, for example by placing the essential gene in the genome of the host cell behind an inducible promoter or by using a transient disruption of the essential gene, or by placing the entire essential gene back into the genome. The inducible promoter may be any inducible promoter suitable for the purpose, be it a chemically or physically induced promoter (such as by temperature or light). The person skilled in the art knows how to select such promoter. In one embodiment, the *niiA* promoter from *Penicillium chrysogenum* is used. This promoter is induced by nitrate but is repressed by ammonium. When culturing on ammonium as the sole N-source in the medium, the host is deficient for the essential gene. When culturing on nitrate as the sole N-source in the medium, the host cell is not deficient in the essential gene. In another embodiment, the *xlnA* promoter from *Aspergillus niger* is used. This promoter is induced by xylose but is repressed by glucose. When culturing on glucose medium, the host is deficient for in essential gene. When culturing on xylose medium, the host cell is not deficient in the essential gene.

The host cell is a filamentous fungal cell.

"Filamentous fungi" include all filamentous forms of the subdivision *Eumycota* and *Oomycota* (as defined by Hawksworth et al., In, Ainsworth and Bisby's Dictionary of The Fungi, 8th edition, 1995, CAB International, University Press, Cambridge, UK). The filamentous fungi are characterized by a mycelial wall composed of chitin, cellulose, glucan, chitosan, mannan, and other complex polysaccharides. Vegetative growth is by hyphal elongation. Filamentous fungal strains include, but are not limited to, strains of *Acremonium, Agaricus, Aspergillus, Aureobasidium, Chrysosporium, Coprinus, Cryptococcus, Filibasidium, Fusarium, Geosmithia, Humicola, Magnaporthe, Mucor, Myceliophthora, Neocallimastix, Neurospora, Paecilomyces, Penicillium, Piromyces, Panerochaete, Pleurotus, Rasamsonia, Schizophyllum, Talaromyces, Thermoascus, Thermomyces, Thielavia, Tolypocladium,* and *Trichoderma.*

Preferred filamentous fungal cells belong to a species of an *Acremonium, Aspergillus, Chrysosporium, Myceliophthora, Penicillium, Rasamsonia, Talaromyces, Thielavia, Fusarium* or *Trichoderma* genus, and even more preferably a species of *Aspergillus niger, Acremonium alabamense, Acremonium chrysogenum, Aspergillus awamori, Aspergillus foetidus, Aspergillus sojae, Aspergillus fumigatus, Talaromyces emersonii, Talaromyces thermophilus, Thermomyces lanuginosus, Thermoascus thermophilus, Thermoascus aurantiacus, Thermoascus crustaceus, Rasamsonia emersonii, Rasamsonia byssochlamyoides, Rasamsonia argillacea, Rasamsonia brevistipitata, Rasamsonia cylindrospora, Aspergillus oryzae, Chrysosporium lucknowense, Fusarium oxysporum, Myceliophthora thermophila, Trichoderma reesei, , Thielavia terrestris* or *Penicillium chrysogenum.* Most preferred species are *Aspergillus niger* or *Penicillium chrysogenum.* When the host cell is an *Aspergillus* host cell, the host cell preferably is CBS 513.88, CBS124.903 or a derivative thereof. When the host cell is a *Penicillium* host cell, the host cell is preferably *Penicillium chrysogenum* strain NRRL 1951 and Wisconsin 54-1255 and all industrial derivatives, in particular *Penicillium chrysogenum* strains DS54465 and DS61187. When the host cell belongs to the genus *Rasamsonia* also known as *Talaromyces,* more preferably the host cell belongs to the species *Talaromyces emersonii* also known as *Rasamsonia emersonii.* When the host cell according to the invention is a *Talaromyces emersonii* also known as *Rasamsonia emersonii* host cell, the host cell preferably is CBS 124.902 or a derivative thereof.

The *Rasamsonia emersonii* (*R. emersonii*) strains used herein are derived from ATCC16479, which is used as wild-type strain. ATCC16479 was formerly also known as *Talaromyces emersonii* and *Penicillium geosmithia emersonii.* Upon the use of the name *Rasamsonia emersonii* also *Talaromyces emersonii* is meant. Other strain designations of *R. emersonii* ATCC16479 are CBS393.64, IFO31232 and IM1116815.

*Rasamsonia (Talaromyces) emersonii* strain TEC-142 is deposited at CENTRAAL BUREAU VOOR SCHIMMELCULTURES, Uppsalalaan 8, P.O. Box 85167, NL-3508 AD Utrecht, The Netherlands on 1st July 2009 having the Accession Number CBS 124902. TEC-142S is a single isolate of TEC-142.

Such strains are suitable for use in the invention.

Several strains of filamentous fungi are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), and Agricultural Research Service Patent Culture Collection, Northern Regional Research Center (NRRL) *Aspergillus niger* CBS 513.88, *Aspergillus oryzae* ATCC 20423, IFO 4177, ATCC 1011, ATCC 9576, ATCC14488-14491, ATCC 11601, ATCC12892, *P. chrysogenum* CBS 455.95, *Penicillium citrinum* ATCC 38065, *Penicillium chrysogenum* P2, *Talaromyces emersonii* CBS 124.902, *Acremonium chrysogenum* ATCC 36225 or ATCC 48272, *Trichoderma reesei* ATCC 26921 or ATCC 56765 or ATCC 26921, *Aspergillus sojae* ATCC11906, *Chrysosporium lucknowense* C1, Garg 27K, VKM-F 3500 D, ATCC44006 and derivatives thereof.

Preferably, the filamentous fungal host cell additionally comprises modifications in its genome such that it is deficient in at least one of glucoamylase *(glaA),* acid stable alpha-amylase *(amyA),* neutral alpha-amylase (*amyBI* and *amyBII*)*,* oxalic acid hydrolase (*oahA*), a toxin, such as ochratoxin and fumonisin, and protease transcriptional regulator PrtT. Preferably, the host cell additionally comprises a disruption of the *pepA* gene encoding the major extracellular aspartic protease PepA.

Preferably, the host cell additionally comprises a modification of Sec61. A preferred Sec61 modification is a modification which results in a one-way mutant of Sec61; i.e. a mutant wherein the de novo synthesized protein can enter the ER via Sec61, but the protein cannot leave the ER via Sec61. Such modifications are extensively described in WO2005/123763. Most preferably, the Sec 61 modification is the S376W mutation in which Serine 376 is replaced by Tryptophan. These and other possible host modifications are also described in WO2012/001169, WO2011/009700, WO2007/062936, WO2006/040312 or WO2004/070022.

The vector-host system disclosed herewith is conveniently used for the production of a biological compound of interest. The host cell may already be capable to produce the biological compound of interest. The host cell may also be provided with a recombinant homologous or heterologous polynucleotide construct that encodes a polypeptide involved in the production of the biological compound of interest.

Accordingly, the host cell of the vector-host system disclosed herewith preferably comprises a recombinant polynucleotide construct comprising a polynucleotide encoding a compound involved in the synthesis of a biological compound of interest. The polynucleotide may also directly encode a biological compound of interest.

The recombinant polynucleotide construct encoding a compound of interest or a polypeptide involved in the synthesis of a biological compound of interest may be located on the vector of the vector-host system according to the invention.
The biological compound of interest according to the invention can be any biological compound. The biological compound may be biomass or any biopolymer or metabolite. The biological compound may be encoded by a single polynucleotide or a series of polynucleotides composing a biosynthetic or metabolic pathway or may be the direct product of a single polynucleotide or may be products of a series of polynucleotides. The biological compound may be native to the host cell or heterologous. The biological compound may be modified according WO2010/102982.

The term "heterologous biological compound" is defined herein as a biological compound which is not native to the cell; or a native biological compound in which structural modifications have been made to alter the native biological compound.

The term "biopolymer" is defined herein as a chain (or polymer) of identical, similar, or dissimilar subunits (monomers). The biopolymer may be any biopolymer. The biopolymer may for example be, but is not limited to, a nucleic acid, polyamine, polyol, polypeptide (or polyamide), or polysaccharide.

The biopolymer may be a polypeptide. The polypeptide may be any polypeptide having a biological activity of interest. The term "polypeptide" is not meant herein to refer to a specific length of the encoded product and, therefore, encompasses peptides, oligopeptides, and proteins. Polypeptides further include naturally occurring allelic and engineered variations of the above-mentioned polypeptides and hybrid polypeptides. The polypeptides may be a modified polypeptide according WO2010/102982. The polypeptide may be native or may be heterologous to the host cell. The polypeptide may be a collagen or gelatin, or a variant or hybrid thereof. The polypeptide may be an antibody or parts thereof, an antigen, a clotting factor, an enzyme, a hormone or a hormone variant, a receptor or parts thereof, a regulatory protein, a structural protein, a reporter, or a transport protein, protein involved in secretion process, protein involved in folding process, chaperone, peptide amino acid transporter, glycosylation factor, transcription factor, synthetic peptide or oligopeptide, intracellular protein. The intracellular protein may be an enzyme such as, a protease, ceramidases, epoxide hydrolase, aminopeptidase, acylases, aldolase, hydroxylase, aminopeptidase, lipase, non-ribosomal synthetase or polyketide synthetase. The polypeptide may be an enzyme secreted extracellularly, such as an oxidoreductase, transferase, hydrolase, lyase, isomerase, catalase, cellulase, chitinase, cutinase, deoxyribonuclease, dextranase, esterase. The enzyme may be a carbohydrase, e.g. cellulases such as endoglucanases, β-glucanases, cellobiohydrolases or β-glucosidases, GH61-enzymes, hemicellulases or pectinolytic enzymes such as xylanases, xylosidases, mannanases, galactanases, galactosidases, pectin methyl esterases, pectin lyases, pectate lyases, endo polygalacturonases, exopolygalacturonases rhamnogalacturonases, arabanases, arabinofuranosidases, arabinoxylan hydrolases, galacturonases, lyases, or amylolytic enzymes; hydrolase, isomerase, or ligase, phosphatases such as phytases, esterases such as lipases, phospholipases, galactolipases, proteolytic enzymes, dairy enzymes and products (e.g. chymosin, casein), oxidoreductases such as oxidases, transferases, or isomerases. The enzyme may be a phytase. The enzyme may be an aminopeptidase, asparaginase, amylase, carbohydrase, carboxypeptidase, endo-protease, metallo-protease, serine-protease, catalase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, protein deaminase, invertase, laccase, lipase, mannosidase, mutanase, oxidase, pectinolytic enzyme, peroxidase, phospholipase, polyphenoloxidase, ribonuclease, transglutaminase, or glucose oxidase, hexose oxidase, monooxygenase.

According to the present invention, a polypeptide can also be a fused or hybrid polypeptide to which another polypeptide is fused at the N-terminus or the C-terminus of the polypeptide or fragment thereof. A fused polypeptide is produced by fusing a nucleic acid sequence (or a portion thereof) encoding one polypeptide to a nucleic acid sequence (or a portion thereof) encoding another polypeptide.

Techniques for producing fusion polypeptides are known in the art, and include, ligating the coding sequences encoding the polypeptides so that they are in frame and expression of the fused polypeptide is under control of the same promoter (s) and terminator. The hybrid polypeptides may comprise a combination of partial or complete polypeptide sequences obtained from at least two different polypeptides wherein one or more may be heterologous to the host cell.

The biopolymer may be a polysaccharide. The polysaccharide may be any polysaccharide, including, but not limited to, a mucopolysaccharide(e. g., heparin and hyaluronic acid) and nitrogen-containing polysaccharide (eg., chitin). In a more preferred option, the polysaccharide is hyaluronic acid.

The polynucleotide of interest according to the invention may encode an enzyme involved in the synthesis of a primary or secondary metabolite, such as organic acids, carotenoids, antibiotics, anti-cancer drug, pigments isoprenoids, alcohols, fatty acids and vitamins. Such metabolite may be considered as a biological compound according to the present invention.

The term "metabolite" encompasses both primary and secondary metabolites; the metabolite may be any metabolite. Preferred metabolites are citric acid, gluconic acid and succinic acid, antibiotics, bioactive drugs, biofuels and building blocks of biomaterials.

The metabolite may be encoded by one or more genes, such as in a biosynthetic or metabolic pathway. Primary metabolites are products of primary or general metabolism of a cell, which are concerned with energy metabolism, growth, and structure. Secondary metabolites are products of secondary metabolism (see, for example, R. B. Herbert, The Biosynthesis of Secondary Metabolites, Chapman and Hall, New York, 1981).

The primary metabolite may be, but is not limited to, an amino acid, carboxylic acid, fatty acid, nucleoside, nucleotide, sugar, triglyceride, or vitamin.

The compounds of interest may be an organic compound selected from glucaric acid, gluconic acid, glutaric acid, adipic acid, succinic acid, tartaric acid, oxalic acid, acetic acid, lactic acid, formic acid, malic acid, maleic acid, malonic acid, citric acid, fumaric acid, itaconic acid, levulinic acid, xylonic acid, aconitic acid, ascorbic acid, kojic acid, coumeric acid, a poly unsaturated fatty acid, ethanol, 1,3-propane-diol, ethylene, glycerol, xylitol, carotene, astaxanthin, lycopene and lutein.

The secondary metabolite may be, but is not limited to, an alkaloid, coumarin, flavonoid, polyketide, quinine, steroid, peptide, or terpene, a β-lactam antibiotic such as Penicillin G or Penicillin V and fermentative derivatives thereof, a cephalosporin, cyclosporin or lovastatin. The secondary metabolite may be an antibiotic, antifeedant, attractant, bacteriocide, fungicide, hormone, insecticide, or rodenticide. Preferred antibiotics are cephalosporins and beta-lactams.

The biological compound of interest may also be the product of a selectable marker. A selectable marker is a product of a polynucleotide of interest which product provides for biocide or viral resistance, resistance to heavy metals, prototrophy to auxotrophs, and the like. Selectable markers include, but are not limited to, amdS (acetamidase), argB (ornithinecarbamoyltransferase), bar (phosphinothricinacetyltransferase), hygB (hygromycin phosphotransferase), niaD (nitratereductase), pyrG (orotidine-5'-phosphate decarboxylase), sC (sulfate adenyltransferase), trpC (anthranilate synthase), ble (phleomycin resistance protein), as well as equivalents thereof.

According to one embodiment, the biological compound of interest is preferably a polypeptide as described herein. Preferably, said polypeptide is an enzyme as described herein.

According to another embodiment, the biological compound of interest is preferably a metabolite as described herein.

When the biological compound of interest is a biopolymer as defined earlier herein, the host cell may already be capable to produce the biopolymer. The host cell may also be provided with a recombinant homologous or heterologous polynucleotide construct that encodes a polypeptide involved in the production of the biological compound of interest. The person skilled in the art knows how to modify a microbial host cell such that it is capable of production of the compound involved in the production of the biological compound of interest.

The term "recombinant polynucleotide construct" is herein referred to as a nucleic acid molecule, either single-or double-stranded, which is isolated from a naturally occurring gene or which has been modified to contain segments of nucleic acid which are combined and juxtaposed in a manner which would not otherwise exist in nature. The term recombinant polynucleotide construct is synonymous with the term "expression cassette" when the nucleic acid construct contains all the control sequences required for expression of a coding sequence, wherein said control sequences are operably linked to said coding sequence.

The term "operably linked" is defined herein as a configuration in which a control sequence is appropriately placed at a position relative to the coding sequence of the DNA sequence such that the control sequence directs the production of an mRNA or a polypeptide.

The term "control sequences" is defined herein to include all components, which are necessary or advantageous for the production of mRNA or a polypeptide, either *in vitro* or in a host cell. Each control sequence may be native or foreign to the nucleic acid sequence encoding the polypeptide. Such control sequences include, but are not limited to, a leader, Shine-Delgarno sequence, optimal translation initiation sequences (as described in Kozak, 1991, J. Biol. Chem. 266:19867-19870), a polyadenylation sequence, a pro-peptide sequence, a pre-pro-peptide sequence, a promoter, a signal sequence, and a transcription terminator. At a minimum, the control sequences include a promoter, and a transcriptional stop signal as well as translational start and stop signals. Control sequences may be optimized to their specific purpose. Preferred optimized control sequences used in the present invention are those described in WO2006/077258.

The control sequences may be provided with linkers for the purpose of introducing specific restriction sites facilitating ligation of the control sequences with the coding region of the nucleic acid sequence encoding a polypeptide.

The control sequence may be an appropriate promoter sequence (promoter).

The control sequence may also be a suitable transcription terminator (terminator) sequence, a sequence recognized by a filamentous fungal cell to terminate transcription. The terminator sequence is operably linked to the 3'-terminus of the nucleic acid sequence encoding the polypeptide. Any terminator, which is functional in the cell, may be used in the present invention.

According to the present invention, control sequences will always be chosen in such a way that the chance of gene conversion or integration of the vector into the host genome is minimized. The person skilled in the art knows how to construct a vector with minimal chance of integration into the genome. In one embodiment, the vector lacks significant similarity with the genome of the host to minimize the chance of integration into the host genome. This may be achieved by using control sequences, such as promoters and terminators, which originate from another species than the host species. In one embodiment, control sequences from *A. nidulans* are used for a vector which is used in a vector-host system in fungi, in particular a filamentous fungus other than *A*. *nidulans.*

Depending on the host, suitable control sequences may be obtained from the polynucleotides encoding *A.nidulans trpC, A.nidulans gpdA, A. nidulans* ribosomal protein S8 (AN0465), *A. nidulans tef (AN4218) A. oryzae* TAKA amylase, *A. niger* glucoamylase (*glaA*), *A. nidulans* anthranilate synthase, *A. niger* alpha-glucosidase, *trpC* and *Fusarium oxysporum* trypsin-like protease.

The control sequence may also be a suitable leader sequence (leaders), a non-translated region of an mRNA which is important for translation by the filamentous fungal cell. The leader sequence is operably linked to the 5'-terminus of the nucleic acid sequence encoding the polypeptide. Any leader sequence, which is functional in the cell, may be used in the present invention.

Depending on the host, suitable leaders may be obtained from the polynucleotides encoding *A. oryzae* TAKA amylase and *A*. *nidulans* triose phosphate isomerase and *A*. *niger* GlaA and phytase.

Other control sequences may be isolated from the *Penicillium* IPNS gene, or *pcbC* gene, the beta tubulin gene. All the control sequences cited in WO 01/21779 are herewith incorporated by reference.

The control sequence may also be a polyadenylation sequence, a sequence which is operably linked to the 3'-terminus of the nucleic acid sequence and which, when transcribed, is recognized by the filamentous fungal cell as a signal to add polyadenosine residues to transcribed mRNA. Any polyadenylation sequence, which is functional in the cell, may be used in the present invention.

The term "promoter" is defined herein as a DNA sequence that binds RNA polymerase and directs the polymerase to the correct downstream transcriptional start site of a nucleic acid sequence encoding a biological compound to initiate transcription. RNA polymerase effectively catalyzes the assembly of messenger RNA complementary to the appropriate DNA strand of a coding region. The term "promoter" will also be understood to include the 5'-non-coding region (between promoter and translation start) for translation after transcription into mRNA, cis-acting transcription control elements such as enhancers, and other nucleotide sequences capable of interacting with transcription factors. The promoter may be any appropriate promoter sequence suitable for a eukaryotic or prokaryotic host cell, which shows transcriptional activity, including mutant, truncated, and hybrid promoters, and may be obtained from polynucleotides encoding extra-cellular or intracellular polypeptides either homologous (native) or heterologous (foreign) to the cell. The promoter may be a constitutive or inducible promoter.

Examples of inducible promoters that can be used are chemically and physically inducible promoters, including starch-, cellulose-, hemicellulose (such as xylan- and/or xylose-inducible), copper-, oleic acid, oxygen and nitrate- inducible promoters. The promoter may be selected from the group, which includes but is not limited to promoters obtained from the polynucleotides encoding *P. chrysogenum* nitrate or nitrite reductase, *A. oryzae* TAKA amylase, *Rhizomucor miehei* aspartic proteinase, A. *niger* neutral alpha-amylase, *A*. *niger* acid stable alpha-amylase, *A*. *niger* or *A*. *awamori* glucoamylase (glaA), *A*. *niger* or *A*. *awamori* endoxylanase (*xlnA)* or beta-xylosidase *(xlnD), R. miehei* lipase, *A*. *oryzae* alkaline protease, *A*. *oryzae* triose phosphate isomerase, *A*. *nidulans* acetamidase, *Fusarium venenatum* amyloglucosidase (WO 00/56900), *Fusarium venenatum* Dania (WO 00/56900), *Fusarium venenatum* Quinn (WO 00/56900), *Fusarium oxysporum* trypsin-like protease (WO 96/00787), *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase IV, *Trichoderma reesei* endoglucanase V, *Trichoderma reesei* xylanase I, *Trichoderma reesei* xylanase II, *Trichoderma reesei* beta-xylosidase, as well as the NA2-tpi promoter (a hybrid of the promoters from the polynucleotides encoding *A*. *niger* neutral alpha-amylase and *A*. *oryzae* triose phosphate isomerase), and mutant, truncated, and hybrid promoters thereof. Other examples of promoters are the promoters described in WO2006/092396 and WO2005/100573, which are herein incorporated by reference. Another example of the use of promoters is described in WO2008/098933. Other examples of inducible (heterologous) promoters are the alcohol inducible promoter *a*/*c*A, the tet system using the tetracycline-responsive promoter, the estrogen-responsive promoter (Pachlinger et al. (2005), Appl & Environmental Microbiol 672-678).

In order to facilitate expression, the polynucleotide encoding the polypeptide involved in the production of the compound of interest may be a synthetic polynucleotide. The synthetic polynucleotides may be optimized in codon use, preferably according to the methods described in WO2006/077258 or WO2008/000632. WO2008/000632 addresses codon-pair optimization. Codon-pair optimization is a method wherein the nucleotide sequences encoding a polypeptide have been modified with respect to their codon-usage, in particular the codon-pairs that are used, to obtain improved expression of the nucleotide sequence encoding the polypeptide and/or improved production of the encoded polypeptide. Codon pairs are defined as a set of two subsequent triplets (codons) in a coding sequence (CDS).

Furthermore, standard molecular cloning techniques such as DNA isolation, gel electrophoresis, enzymatic restriction modifications of nucleic acids, Southern analyses, transformation of cells, etc., are known to the skilled person and are for example described by Sambrook et al. (1989) "Molecular Cloning: a laboratory manual", Cold Spring Harbor Laboratories, Cold Spring Harbor, New York and Innis et al. (1990) "PCR protocols, a guide to methods and applications" Academic Press, San Diego.

A nucleic acid may be amplified using cDNA, mRNA or alternatively, genomic DNA, as a template and appropriate oligonucleotide primers according to standard PCR amplification techniques. The nucleic acid so amplified can be cloned into an appropriate vehicle and characterized by DNA sequence analysis.

### Host cell

The present specification further discloses a host cell deficient in an essential gene, comprising a vector, said vector comprising at least said essential gene and an autonomous replication sequence.

The host cell is preferably a host cell of the vector-host system as defined earlier herein in the section "vector-host system".

The essential gene is preferably an essential gene as defined earlier herein in the section "vector-host system".

The vector is preferably a vector as defined earlier herein in the section "vector-host system".

Deficiency is defined as earlier herein in the section "vector-host system".

Deficiency can be measured using any assay available to the skilled person, such as transcriptional profiling, Northern blotting, Southern blotting and Western blotting.
Deficiency of the host cell deficient in the essential gene is preferably measured relative to the parent cell that is not deficient in the essential gene. Preferably, the deficiency of the host cell, wherein said host cell produces at least 10% less of the product encoded by the essential gene and/or has an at least 10% reduced expression level of the mRNA transcribed from the essential gene and/or has an at least 10% decreased specific (protein) activity of the product encoded by the essential gene as compared to the parent cell which is not deficient in the essential gene. More preferably, the deficiency is at least 20%, even more preferably at least 30%, even more preferably at least 40%, even more preferably at least 50%, even more preferably at least 60%, even more preferably at least 70%, even more preferably at least 75%, even more preferably at least 80%, even more preferably at least 85%, even more preferably at least 90%, even more preferably at least 95%, even more preferably at least 96%, even more preferably at least 97%, even more preferably at least 98%, even more preferably at least 99%, even more preferably at least 99.5%, even more preferably at least 99.9% and most preferably the deficiency is complete, i.e. 100%.

The host cell has increased stability of the vector comprising an autonomous replication sequence. The stability is preferably measured relative to a host cell, wherein the vector is identical but the host is not deficient in the essential gene. The stability is preferably determined comparing the loss of the vector in subsequent cycles of sporulation and single colony isolation on plates with non-selective solid medium. The higher the stability, the longer it will take before the vector is lost from the host cell, in particular on non-selective solid medium, such as complex or undefined medium. In the system according to the invention, the vector is maintained for at least four subsequent cycles of sporulation. Preferably, the vector is maintained for at least five, at least six, at least seven, at least eight, at least nine or at least ten subsequent cycles of sporulation. More preferably, the vector is maintained for at least 15, at least 20, at least 25, at least 30, at least 40, at least 50, at least 60 or at least 70 subsequent cycles of sporulation. If the vector comprises a non-selective colour marker such as GFP or DsRed, presence of the vector in the host can easily be observed by presence of the colour of the marker in the colonies.

Preferably, the increase in stability of the host cell disclosed herewith compared to a host cell wherein the vector is identical but the host cell is not deficient in the essential gene is at least a two-fold increase, more preferably at least a three-fold increase, more preferably at least a five-fold increase, more preferably at least a ten-fold increase, more preferably at least a twenty-fold increase, more preferably at least a fifty-fold increase, more preferably at least a hundred-fold increase, more preferably at least a two-hundred-fold increase, more preferably at least a five hundred-fold increase and most preferably at least a thousand-fold increase.

The autonomous replication sequence is preferably one as defined earlier herein in the section "vector-host system".
In one embodiment, the host cell is, preferably inducibly, increased in its efficiency of homologous recombination (HR) as defined earlier herein in the section "vector-host system". The host cell is preferably decreased in its efficiency of non-homologous recombination (NHR). The ratio of non-homologous recombination / homologous recombination (NHR/HR) will typically be decreased in a preferred host cell.

Host cells having a decreased NHR/HR ratio as compared to a parent cell may be obtained by modifying the parent eukaryotic cell by increasing the efficiency of the HR pathway and/or by decreasing the efficiency of the NHR pathway. Preferably, the NHR/HR ratio thereby is decreased at least twice, preferably at least 4 times, more preferably at least 10 times. Preferably, the NHR/HR ratio is decreased in the host cell of the vector-host system according to the specification as compared to a parent host cell by at least 5%, more preferably at least 10%, even more preferably at least 20%, even more preferably at least 30%, even more preferably at least 40%, even more preferably at least 50%, even more preferably at least 60%, even more preferably at least 70%, even more preferably at least 80%, even more preferably at least 90% and most preferably by at least 100%.

In order to be able to further engineer the host cell disclosed herein, the deficiency of said host cell may or may not be an inducible deficiency. This can be achieved by methods known to the person skilled in the art, for example by placing the essential gene in the genome of the host cell behind an inducible promoter or by using a transient disruption of the essential gene, or by placing the entire essential gene back into the genome. The inducible promoter may be any inducible promoter suitable for the purpose, be it a chemically or physically induced promoter (such as by temperature or light). The person skilled in the art knows how to select such promoter. In one embodiment, the *niiA* promoter from *Penicillium chrysogenum* is used. This promoter is induced by nitrate but is repressed by ammonium. When culturing on ammonium as the sole N-source in the medium, the host is deficient for the essential gene. When culturing on nitrate as the sole N-source in the medium, the host cell is not deficient in the essential gene. In another embodiment, the *xlnA* promoter from *Aspergillus niger* is used. This promoter is induced by xylose but is repressed by glucose. When culturing on glucose medium, the host is deficient for the essential gene. When culturing on xylose medium, the host cell is not deficient in the essential gene.

The host cell disclosed herein is conveniently used for the production of a biological compound of interest.

Accordingly, the host cell preferably comprises a recombinant polynucleotide construct comprising a polynucleotide encoding a compound involved in the synthesis of a biological compound of interest. The polynucleotide may also directly encode a biological compound of interest.

Said recombinant polynucleotide construct encoding a compound of interest is located on the vector of the vector-host system disclosed herein, said recombinant polynucleotide construct may be located on the genome of the host of the vector-host system, or said recombinant polynucleotide construct may be located on a separate vehicle.

The biological compound of interest is preferably one as defined earlier herein in the section "vector-host system".

### Method for the production of a vector-host system

The present specification further discloses a method for the production of a vector-host system, said method comprising:
a. providing a host cell and a vector, which comprises at least a gene essential for said host cell and an autonomous replication sequence,
b. co-transforming the host cell with the vector and a disruption construct for said essential gene to render the host cell deficient in the essential gene.

If the host cell is inducibly deficient in the essential gene, a method for the production of a vector-host system comprising:
a. providing a host cell and a vector, which vector comprising at least a gene essential for said host cell and an autonomous replication sequence,
b. transforming the host cell with a disruption construct for said essential gene to render the host cell inducibly deficient in the essential gene,
c. transforming the host cell with the vector;
is also part of the disclosure. The skilled person will understand that the host cell produced in step b. cannot be in the 'deficient state' as long as the host has not been transformed with the vector in step c.

Additional variations for method of transformation, co-transformation and use of disruption constructs are described in WO2008/000715 (High throughput transfection), WO2009/150195 and WO2008/113847.

Host cell, vector, essential gene and autonomous replication sequence are preferably those as defined earlier herein in the section "vector-host system". Deficiency is defined as earlier herein in the section "vector-host system".

Deficiency of the host cell deficient in the essential gene is preferably measured relative to the parent cell that is not deficient in the essential gene. Preferably, the deficiency of the host cell, wherein said host cell produces at least 10% less of the product encoded by the essential gene and/or has an at least 10% reduced expression level of the mRNA transcribed from the essential gene and/or has an at least 10% decreased specific (protein) activity of the product encoded by the essential gene as compared to the parent cell which is not deficient in the essential gene. More preferably, the deficiency is at least 20%, even more preferably at least 30%, even more preferably at least 40%, even more preferably at least 50%, even more preferably at least 60%, even more preferably at least 70%, even more preferably at least 75%, even more preferably at least 80%, even more preferably at least 85%, even more preferably at least 90%, even more preferably at least 95%, even more preferably at least 96%, even more preferably at least 97%, even more preferably at least 98%, even more preferably at least 99%, even more preferably at least 99.5%, even more preferably at least 99.9% and most preferably the deficiency is complete, i.e. 100%.

The host cell has increased stability of the vector comprising an autonomous replication sequence. The stability is preferably measured relative to a host cell, wherein the vector is identical but the host is not deficient in the essential gene. The stability is preferably determined comparing the loss of the vector in subsequent cycles of sporulation and single colony isolation on plates with non-selective solid medium. The higher the stability, the longer it will take before the vector is lost from the host cell, in particular on non-selective solid medium, such as complex or undefined medium. In the system according to the invention, the vector is maintained for at least four subsequent cycles of sporulation. Preferably, the vector is maintained for at least five, at least six, at least seven, at least eight, at least nine or at least ten subsequent cycles of sporulation. More preferably, the vector is maintained for at least 15, at least 20, at least 25, at least 30, at least 40, at least 50, at least 60 or at least 70 subsequent cyles of sporulation. I If the vector comprises a non-selective colour marker such as GFP or DsRed, presence of the vector in the host can easily be observed by presence of the colour of the marker in the colonies.

Preferably, the increase in stability of the host cell compared to a host cell wherein the vector is identical but the host cell is not deficient in the essential gene is at least a two-fold increase, more preferably at least a three-fold increase, more preferably at least a five-fold increase, more preferably at least a ten-fold increase, more preferably at least a twenty-fold increase, more preferably at least a fifty-fold increase, more preferably at least a hundred-fold increase, more preferably at least a two-hundred-fold increase, more preferably at least a five hundred-fold increase and most preferably at least a thousand-fold increase.

Preferably, the host cell is, preferably inducibly, increased in its efficiency of homologous recombination (HR) as described earlier herein in the section "vector-host system". The host cell is preferably decreased in its efficiency of non-homologous recombination (NHR). The ratio of non-homologous recombination / homologous recombination (NHR/HR) will typically be decreased in a preferred host cell of the invention.

The host cell can be rendered deficient for the essential gene according to the methods described earlier herein in the section "vector-host system". In one embodiment, the host cell is transformed with a disruption construct. Such disruption construct preferably comprises a polynucleotide corresponding to the wild-type polynucleotide such that the wild-type polynucleotide is replaced by a defective polynucleotide, i.e. a polynucleotide that fails to produce a (fully functional) protein. By homologous recombination, the defective polynucleotide replaces the endogenous polynucleotide.

In one embodiment, the vector comprising at least the essential gene for said host cell and an autonomous replication sequence is transformed simultaneously with the disruption construct in order to simultaneously disrupt the genomic copy of the essential gene and introduce the complementing copy with the vector.

The vector comprising at least the essential gene for the host cell and an autonomous replication sequence may also comprise a recombinant polynucleotide construct encoding a compound involved in the synthesis of a biological compound of interest. The biological compound of interest is preferably one described earlier herein in the section "vector-host system". Said recombinant polynucleotide construct encoding a compound involved in the synthesis of a biological compound of interest may also be introduced into the host cell on a separate vehicle using a separate transformation event, either before or after disruption of the essential gene and either before or after introduction of the vector comprising at least the essential gene for the host cell and an autonomous replication sequence. In one embodiment, the cre/IoxP system as described earlier herein is used to inactivate the essential gene on the genome.

Transformation of a host cell by introduction of a polynucleotide, an expression vector or a polynucleotide construct into the cell is preferably performed by techniques well known in the art (see Sambrook & Russell; Ausubel, *supra*)*.* Transformation may involve a process consisting of protoplast formation, transformation of the protoplasts, and regeneration of the cell wall in a manner known *per se.* Suitable procedures for transformation of *Aspergillus, Penicillium* and *Rasamsonia* cells are described in EP 238 023 and Yelton et al., 1984, Proceedings of the National Academy of Sciences USA 81:1470-1474 and Cantoral et al., 1987; Bio/Technol. 5: 494-497. Suitable procedures for transformation of *Aspergillus* and other filamentous fungal host cells using *Agrobacterium tumefaciens* are described in e.g. De Groot et al., Agrobacterium tumefaciens-mediated transformation of filamentous fungi. Nat Biotechnol. 1998, 16:839-842. Erratum in: Nat Biotechnol 1998 16:1074. A suitable method of transforming *Fusarium* species is described by Malardier et al., 1989, Gene 78:147156 or in WO 96/00787. Other methods can be applied such as a method using biolistic transformation as described in: Christiansen et al., Biolistic transformation of the obligate plant pathogenic fungus, Erysiphe graminis f.sp. hordei. 1995, Curr Genet. 29:100-102. Yeast may be transformed using the procedures described by Becker and Guarente, In Abelson, J. N. and Simon, M. I., editors, Guide to Yeast Genetics and Molecular Biology, Methods in Enzymology, Volume 194, pp 182-187, Academic Press, Inc., New York; Ito et al., 1983, Journal of Bacteriology 153: 163; and Hinnen et al., 1978, Proceedings of the National Academy of Sciences USA 75: 1920.

### Method for the production of a biological compound of interest

The present invention relates to a method for the production of a biological compound of interest as depicted in the appended claims.
1. Method for the production of a biological compound of interest comprising culturing a filamentous fungal host cell deficient in an essential gene, comprising a vector, said vector comprising at least said essential gene and an autonomous replication sequence, wherein the host cell comprises a recombinant polynucleotide construct comprising a polynucleotide encoding a biological compound of interest or a compound involved in the synthesis of a biological compound of interest
   under conditions conducive to the production of the biological compound of interest and isolating the compound of interest from the culture broth, wherein a recombinant polynucleotide construct encoding the biological compound of interest or a compound involved in the synthesis of the biological compound of interest is located on said vector.
2. Method for the production of a biological compound of interest comprising producing a filamentous fungal host cell according to a method which comprises:
   a. providing a filamentous fungal host cell and a vector which comprises at least a gene essential for said host cell and an autonomous replication sequence, wherein a recombinant polynucleotide construct encoding the compound of interest or a compound involved in the synthesis of the biological compound of interest is located on said vector; and
   b. co-transforming the host cell with the vector and a disruption construct for said essential gene to render the host cell deficient in the essential gene;
   culturing such a host cell under conditions conducive to the production of the biological compound of interest and
   isolating the compound of interest from the culture broth.
3. Method according to claim 1 or 2, wherein the host cell is, preferably inducibly, increased in its efficiency of homologous recombination (HR), decreased in its efficiency of non-homologous recombination (NHR) or decreased in its ratio of non-homologous recombination/homologous recombination (NHR/HR).
4. Method according to any one of the preceding claims, wherein the host cell deficiency in the essential gene is inducible.
5. Method according to any one of the preceding claims, wherein the essential gene is an essential gene in fungi.
6. Method according to any one of the preceding claims, wherein the essential gene is the *tif35* or *aur1* gene.
7. Method according to any one of the preceding claims, wherein the host cell is an *Aspergillus, Chrysosporium, Penicillium, Rasamsonia, Talaromyces* or *Trichoderma.*
8. Method according to claim 7, wherein the host cell is *Aspergillus niger, Penicillium chrysogenum,* or *Rasamsonia emersonii.*
9. Method according to any one of the preceding claims, wherein the vector contains control sequences from a species other than the host species.
10. Method according to any one of the preceding claims, wherein the biological compound of interest is a biopolymer or a metabolite.
11. Method according to claim 10, wherein the biopolymer is a polypeptide.
12. Method according to claim 11, wherein the polypeptide is an antibody or parts thereof, an antigen, a clotting factor, an enzyme, a hormone or a hormone variant, a receptor or parts thereof, a regulatory protein, a structural protein, a reporter, or a transport protein, protein involved in secretion process, protein involved in folding process, chaperone, peptide amino acid transporter, glycosylation factor, transcription factor, synthetic peptide or oligopeptide, intracellular protein.
13. Method according to claim 12, wherein the enzyme is a protease, ceramidase, epoxide hydrolase, aminopeptidase, acylases, aldolase, hydroxylase, aminopeptidase, lipase, non-ribosomal synthetase, polyketide synthetase, oxidoreductase, transferase, hydrolase, lyase, isomerase, catalase, cellulase, chitinase, cutinase, deoxyribonuclease, dextranase, esterase, carbohydrase, e.g. cellulase such as endoglucanase, β-glucanase, cellobiohydrolase or β-glucosidase, GH61-enzyme, hemicellulose, pectinolytic enzyme such as xylanase, xylosidase, mannanase, galactanase, galactosidase, pectin methyl esterase, pectin lyase, pectate lyase, endo polygalacturonase, exopolygalacturonase, rhamnogalacturonase, arabanase, arabinofuranosidase, arabinoxylan hydrolase, galacturonase, lyase, or amylolytic enzyme; hydrolase, isomerase, or ligase, phosphatase such as phytase, esterase such as lipase, phospholipase, galactolipase, proteolytic enzyme, dairy enzyme such as chymosin, oxidoreductase such as oxidase, transferase, isomerase, phytase, aminopeptidase, asparaginase, amylase, carbohydrase, carboxypeptidase, endo-protease, metallo-protease, serine-protease, catalase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, protein deaminase, invertase, laccase, lipase, mannosidase, mutanase, oxidase, pectinolytic enzyme, peroxidase, phospholipase, polyphenoloxidase, ribonuclease, transglutaminase, or glucose oxidase, hexose oxidase, monooxygenase.
14. Method according to any one of the preceding claims wherein the host cell is already capable to produce the biological compound of interest.

Host cell, vector, essential gene and autonomous replication sequence are preferably those as defined earlier herein in the section "vector-host system".

Deficiency is defined as earlier herein in the section "vector-host system".

Deficiency of the host cell deficient in the essential gene is preferably measured relative to the parent cell that is not deficient in the essential gene. Preferably, the deficiency of the host cell, wherein said host cell produces at least 10% less of the product encoded by the essential gene and/or has an at least 10% reduced expression level of the mRNA transcribed from the essential gene and/or has an at least 10% decreased specific (protein) activity of the product encoded by the essential gene as compared to the parent cell which is not deficient in the essential gene. More preferably, the deficiency is at least 20%, even more preferably at least 30%, even more preferably at least 40%, even more preferably at least 50%, even more preferably at least 60%, even more preferably at least 70%, even more preferably at least 75%, even more preferably at least 80%, even more preferably at least 85%, even more preferably at least 90%, even more preferably at least 95%, even more preferably at least 96%, even more preferably at least 97%, even more preferably at least 98%, even more preferably at least 99%, even more preferably at least 99.5%, even more preferably at least 99.9% and most preferably the deficiency is complete, i.e. 100%.

The host cell has increased stability of the vector comprising an autonomous replication sequence. The stability is preferably measured relative to a host cell, wherein the vector is identical but the host is not deficient in the essential gene. The stability is preferably determined comparing the loss of the vector in subsequent cycles of sporulation and single colony isolation on plates with non-selective solid medium. The higher the stability, the longer it will take before the vector is lost from the host cell, in particular on non-selective solid medium, such as complex or undefined medium. In the system according to the invention, the vector is maintained for at least four subsequent cycles of sporulation. Preferably, the vector is maintained for at least five, at least six, at least seven, at least eight, at least nine or at least ten subsequent cycles of sporulation. More preferably, the vector is maintained for at least 15, at least 20, at least 25, at least 30, at least 40, at least 50, at least 60 or at least 70 subsequent cyles of sporulation. I If the vector comprises a non-selective colour marker such as GFP or DsRed, presence of the vector in the host can easily be observed by presence of the colour of the marker in the colonies.

Preferably, the increase in stability of the host cell compared to a host cell wherein the vector is identical but the host cell is not deficient in the essential gene is at least a two-fold increase, more preferably at least a three-fold increase, more preferably at least a five-fold increase, more preferably at least a ten-fold increase, more preferably at least a twenty-fold increase, more preferably at least a fifty-fold increase, more preferably at least a hundred-fold increase, more preferably at least a two-hundred-fold increase, more preferably at least a five hundred-fold increase and most preferably at least a thousand-fold increase.

Preferably, the host cell is, preferably inducibly, increased in its efficiency of homologous recombination (HR) as described earlier herein in the section "vector-host system". The host cell is preferably decreased in its efficiency of non-homologous recombination (NHR). The ratio of non-homologous recombination / homologous recombination (NHR/HR) will typically be decreased in a preferred host cell of the invention.

The biological compound of interest is preferably one described earlier herein in the section "vector-host system".

The host cell can be rendered deficient for the essential gene according to the methods described earlier herein in the section "vector-host system". In one embodiment, the host cell is transformed with a disruption construct. Such disruption construct preferably comprises a polynucleotide corresponding to the wild-type polynucleotide such that the wild-type polynucleotide is replaced by a defective polynucleotide, i.e. a polynucleotide that fails to produce a (fully functional) protein. By homologous recombination, the defective polynucleotide replaces the endogenous polynucleotide.

In one embodiment, the vector comprising at least the essential gene for said host cell and an autonomous replication sequence is transformed simultaneously with the disruption construct in order to simultaneously disrupt the genomic copy of the essential gene and introduce the complementing copy with the vector.

The host cell may already be capable to produce the biological compound of interest. The host cell may also be provided with a recombinant homologous or heterologous polynucleotide construct that encodes a polypeptide involved in the production of the biological compound of interest.

The vector comprising at least the essential gene for the host cell and an autonomous replication sequence may accordingly comprise a recombinant polynucleotide construct encoding a compound involved in the synthesis of a biological compound of interest. Said recombinant polynucleotide construct encoding a compound involved in the synthesis of a biological compound of interest may also be introduced into the host cell on a separate vehicle using a separate transformation event, either before or after disruption of the essential gene and either before or after introduction of the vector comprising at least the essential gene for the host cell and an autonomous replication sequence. The recombinant polynucleotide construct encoding a compound involved in the synthesis of a biological compound of interest and the vehicle comprising it, is preferably one as defined earlier herein in the section "vector-host system".

Culturing as used herein means that the microbial cells are cultivated in a nutrient medium suitable for production of the biological compound of interest using methods known in the art. For example, the host cells may be cultivated by shake flask cultivation, small-scale or large-scale fermentation (including continuous, batch, fed-batch, or solid state fermentations) in laboratory or industrial fermentors performed in a suitable medium and under conditions allowing the compound of interest to be produced and isolated. The cultivation takes place in a suitable nutrient medium comprising carbon and nitrogen sources and inorganic salts, using procedures known in the art (see, e. g., Bennett, J. W. and LaSure, L., eds., More Gene Manipulations in Fungi, Academic Press, CA, 1991*).* Suitable media are available from commercial suppliers or may be prepared using published compositions (e. g., in catalogues of the American Type Culture Collection). The system according to the present invention is stable and versatile enough to maintain the vector-host system on all kinds of media, including non-selective, complex media which are typically exploited in industrial fermentations. If the compound of interest is secreted into the nutrient medium, the compound can be isolated directly from the medium. If the compound of interest is not secreted, it can be isolated from cell lysates.

The biological compound of interest may be isolated by methods known in the art. For example, the biological compound of interest may be isolated from the nutrient medium by conventional procedures including, but not limited to, centrifugation, filtration, extraction, spray drying, evaporation, or precipitation. The isolated biological compound of interest may then be further purified by a variety of procedures known in the art including, but not limited to, chromatography (e. g., ion exchange, affinity, hydrophobic, chromatofocusing, and size exclusion), electrophoretic procedures (e.g., preparative isoelectric focusing, differential solubility (e. g., ammonium sulfate precipitation), or extraction (see, e.g., Protein Purification, J.-C. Janson and Lars Ryden, editors, VCH Publishers, New York, 1989). In some applications the biological compound of interest may be used without substantial isolation from the culture broth; separation of the culture medium from the biomass may be adequate.

### Method for screening for a polynucleotide encoding a biological compound of interest

The present specification discloses a method for screening for a polynucleotide encoding a biological compound of interest comprising:
a. providing a library of polynucleotides possibly containing an polynucleotide encoding a biological compound of interest,
b. providing a multiplicity of individual host cells, said host cell being deficient in an essential gene, comprising a vector, said vector comprising at least said essential gene and an autonomous replication sequence,
c. screening the transformants for expression of the biological compound of interest.

After identification of the transformed host cell comprising the polynucleotide encoding the biological compound of interest in step (c), the polynucleotide may optionally be isolated from the host cell identified. Subsequently, the isolated polynucleotide may be retransformed into a suitable host cell, e.g. for industrial production of the biological compound of interest.

Screening may be performed using detection methods known in the art that are specific for the biological compound of interest. These detection methods include, but are not limited to use of specific antibodies, high performance liquid chromatography, capillary chromatography, electrophoresis, formation of an enzyme product, or disappearance of an enzyme substrate.

Host cell, vector, essential gene and autonomous replication sequence are preferably those as defined earlier herein in the section "vector-host system".

Deficiency is defined as earlier herein in the section "vector-host system".

Deficiency of the host cell deficient in the essential gene is preferably measured relative to the parent cell that is not deficient in the essential gene. Preferably, the deficiency of the host cell, wherein said host cell produces at least 10% less of the product encoded by the essential gene and/or has an at least 10% reduced expression level of the mRNA transcribed from the essential gene and/or has an at least 10% decreased specific (protein) activity of the product encoded by the essential gene as compared to the parent cell which is not deficient in the essential gene. More preferably, the deficiency is at least 20%, even more preferably at least 30%, even more preferably at least 40%, even more preferably at least 50%, even more preferably at least 60%, even more preferably at least 70%, even more preferably at least 75%, even more preferably at least 80%, even more preferably at least 85%, even more preferably at least 90%, even more preferably at least 95%, even more preferably at least 96%, even more preferably at least 97%, even more preferably at least 98%, even more preferably at least 99%, even more preferably at least 99.5%, even more preferably at least 99.9% and most preferably the deficiency is complete, i.e. 100%.

The host cell has increased stability of the vector comprising an autonomous replication sequence. The stability is preferably measured relative to a host cell, wherein the vector is identical but the host is not deficient in the essential gene. The stability is preferably determined comparing the loss of the vector in subsequent cycles of sporulation and single colony isolation on plates with non-selective solid medium. The higher the stability, the longer it will take before the vector is lost from the host cell, in particular on non-selective solid medium, such as complex or undefined medium. In the system according to the invention, the vector is maintained for at least four subsequent cycles of sporulation. Preferably, the vector is maintained for at least five, at least six, at least seven, at least eight, at least nine or at least ten subsequent cycles of sporulation. More preferably, the vector is maintained for at least 15, at least 20, at least 25, at least 30, at least 40, at least 50, at least 60 or at least 70 subsequent cyles of sporulation. I If the vector comprises a non-selective colour marker such as GFP or DsRed, presence of the vector in the host can easily be observed by presence of the colour of the marker in the colonies.

Preferably, the increase in stability of the host cell compared to a host cell wherein the vector is identical but the host cell is not deficient in the essential gene is at least a two-fold increase, more preferably at least a three-fold increase, more preferably at least a five-fold increase, more preferably at least a ten-fold increase, more preferably at least a twenty-fold increase, more preferably at least a fifty-fold increase, more preferably at least a hundred-fold increase, more preferably at least a two-hundred-fold increase, more preferably at least a five hundred-fold increase and most preferably at least a thousand-fold increase.

Preferably, the host cell is, preferably inducibly, increased in its efficiency of homologous recombination (HR) as described earlier herein in the section "vector-host system".

The biological compound of interest is preferably one described earlier herein in the section "vector-host system".

The host cell can be rendered deficient for the essential gene according to the methods described earlier herein in the section "vector-host system". In one embodiment, the host cell is transformed with a disruption construct. Such disruption construct preferably comprises a polynucleotide corresponding to the wild-type polynucleotide such that the wild-type polynucleotide is replaced by a defective polynucleotide, i.e. a polynucleotide that fails to produce a (fully functional) protein. By homologous recombination, the defective polynucleotide replaces the endogenous polynucleotide.

In one embodiment, the vector comprising at least the essential gene for said host cell and an autonomous replication sequence is transformed simultaneously with the disruption construct in order to simultaneously disrupt the genomic copy of the essential gene and introduce the complementing copy with the vector.

Transformation is preferably performed as described earlier herein.

The library may encode a biological compound of interest that is native or heterologous to the host cell. The polynucleotide encoding the biological compound of interest may originate from any organism capable of producing the biological compound of interest, including multicellular organisms and microorganisms e.g. bacteria and fungi. The origin of the polynucleotide may also be synthetic meaning that the library could be comprised of e.g. codon optimized variants encoding the same polypeptide or the library could comprise variants obtained by shuffling techniques, or directed evolution techniques known in the art.

The vector-host system and the host cell disclosed herein can conveniently be used for the production of a biological compound of interest.

Accordingly, the present specification further discloses the use of the vector-host system or of the host cell as described earlier herein for the production of a biological compound of interest.

The vector-host system and host cell, are preferably those as defined earlier herein in the sections "vector-host system" and "host cell". The methods and host cells, vectors, biological compound of interest and other desired features are preferably those as defined earlier herein in the section "Method for the production of a biological compound of interest".

The vector-host system and the host cell can conveniently be used for screening for a polynucleotide encoding a biological compound of interest.

Accordingly, the present specification further discloses the use of the vector-host system or of the host cell defined earlier herein for screening for a polynucleotide encoding a compound of interest.

The vector-host system and host cell, are preferably those as defined earlier herein in the sections "vector-host system" and "host cell". The methods and host cells, vectors, biological compound of interest, library and other desired features are preferably those as defined earlier herein in the section "Method for screening for a polynucleotide encoding a biological compound of interest".

The invention described and claimed herein is not to be limited in scope by the specific embodiments herein disclosed, since these embodiments are intended as illustrations of several aspects of the invention. Any equivalent embodiments and/or combinations of preferred aspects of the invention are intended to be within the scope of this invention. Indeed, various modifications of the invention in addition to those shown and described herein will become apparent to those skilled in the art from the foregoing description. Such modifications are also intended to fall within the scope of the appended claims. In the case of conflict, the present disclosure including definitions will control.

### Examples

### Strains

*P. chrysogenum* DS17690, (deposited on 15 April 2008 at the Centraalbureau voor Schimmelcultures, Utrecht, The Netherlands with deposition number CBS122850), is a high penicillin producing strain.
*P. chrysogenum* DS54465, a derivative of DS17690 wherein the *P. chrysogenum* KU70 homologue has been deleted (Snoek et al. (2009) Fungal Genetics and Biology 46, 418-426).
*P. chrysogenum* DS58274, a derivative of DS54465 carrying an inactivated *niaD* locus with a *GFP.SKL* expression cassette allowing its use as both auxotropic marker as well as a transformation control.
*P. chrysogenum* DS61187, a derivative of the much used laboratory strain Wis54-1255 deficient in NHEJ.

*A. niger* WT 1: This *A. niger* strain is a CBS513.88 strain comprising a gene deletion of the *A.niger* KU70 homolog, designated as *hdf*A*.* The construction of deletion vector and genomic deletion of the *hdf*A gene has been described in detail in WO05/095624. The vectors pDEL-HDFA, described in WO05/095624, has been used according the "MARKER-GENE FREE" approach as described in EP 0 635 574 B1. The procedure described above resulted in an *hdf*A deficient recombinant *A*. *niger* CBS 513.88 strain, possessing finally no foreign DNA sequences at all. As such, WT1 has an increased efficiency of homologous recombination and thus a decrease ration of NHR/HR. *A*. *niger* strain CBS513.88 was deposited on 10 August 1988 at the Centraalbureau voor Schimmelcultures, Utrecht, The Netherlands.

*A. niger* WT 2 is a WT 1 strain comprising a deletion of the gene encoding glucoamylase (*gla*A). WT 2 was constructed by using the "MARKER-GENE FREE" approach as described in EP 0 635 574 B1. In this patent it is extensively described how to delete *gla*A specific DNA sequences in the genome of CBS 513.88. The procedure resulted in a MARKER-GENE FREE Δ*gla*A recombinant *A. niger* CBS 513.88 strain, possessing finally no heterologous DNA sequences at all.

The *Rasamsonia emersonii (R. emersonii*) strains used herein are derived from ATCC16479, which is used as wild-type strain. ATCC16479 was formerly also known as *Talaromyces emersonii* and *Penicillium geosmithia emersonii.* Upon the use of the name *Rasamsonia emersonii* also *Talaromyces emersonii* is meant. Other strain designations of *R. emersonii* ATCC16479 are CBS393.64, IFO31232 and IM1116815.

*Rasamsonia (Talaromyces) emersonii* strain TEC-142 is deposited at CENTRAAL BUREAU VOOR SCHIMMELCULTURES, Uppsalalaan 8, P.O. Box 85167, NL-3508 AD Utrecht, The Netherlands on 1st July 2009 having the Accession Number CBS 124902. TEC-142S is a single isolate of TEC-142.

### Media

(i) YGG medium containing 0.8 % KCI, 1.6 % glucose, 0.67 % Difco yeast nitrogen base (Becton, Dickinson & Co., Sparks, Md, USA), 0.15 % citric acid, 0.6 % K₂HPO₄, 0.2 % yeast extract, pH 6.2, with addition of 100 U/ml penicillin and 100 µg/ml streptomycin (Gibco, Invitrogen, Breda, The Netherlands). YGG-sucrose medium contained in addition 34.2 % of sucrose.

*P. chrysogenum* protoplasts and mycelia were grown on:
(i) phleomycin selection agar containing: 1 % Difco yeast nitrogen base), 0.225 % citric acid, 0.9 % K₂HPO₄, 0.1 % yeast extract (Becton, Dickinson & Co.), 2 % glucose, 28.7 % sucrose and 2 % agar, and 1 ml/L of a trace element solution pH 7, supplemented with 100 U/ml penicillin, 100 µg/ml streptomycin and 50 µg/ml phleomycin (Invivogen, San Diego, USA).
(ii) Nitrogen source selection agar contained 0.3 % NaCl, 0.05 % MgSO₄.7H₂O, 0.001 % FeSO₄.7H₂O, 1 % glucose, 10 mM potassiumphosphate buffer pH 6.8 and 2 % agar and 1 ml/L of a trace element solution and was supplemented with 0.1 % acetamide and 15 mM CsCl₂ (acetamide selection agar), 0.1 % (NH₄)₂SO₄ (ammonium selection plates) or 0.1 % NaNO₃ (nitrate selection plates). For protoplast regeneration 34.2 % sucrose was added.
(iii) fluoroacetamide selection agar contained 0.3 % NaCl, 0.05 % MgSO₄.7H₂O, 0.001 % FeSO₄.7H₂O, 1 % glucose, 0.1 % fluoroacetamide, 5 mM urea, 10 mM potassiumphosphate buffer pH 6.8 and 2 % agar.
(iv) chlorate selection agar contained 0.3 % NaCl, 0.1 % KH₂PO₄, 0.05 % MgSO₄.7H₂O, 0.001 % FeSO₄.7H₂O, 1 % glucose, 0.185 % adenine 1.25 % KClO₃ and 2 % agar and 1 ml/L of a trace element solution pH 6.5. For protoplast regeneration 34.2 % sucrose was added.
(v) R agar contained 0.52 % v/v glycerol, 0.75 % v/v beet molasses, 0.5 % yeast extract, 300 mM NaCl, 0.2 m MgSO₄.7H₂O, 0.44 mM KH₂PO₄, 3.3 µM NH₄Fe(SO₄)₂.12 H₂O, 0.4 µM CuSO₄.5H₂O, 1.45 mM CaSO₄.2H₂O and 2 % agar. When required, NaNO₃ was added to a final concentration of 0.1 %.

*A. nidulans* FGSC A4 spores were isolated from R-agar plates and cultivated on YGG medium.

PDA: Potato Dextrose Agar, Oxoid, non-selective medium, prepared according to the supplier's instructions.

*R. emersonii* mycelia were grown on PDA or *Rasamsonia* agar medium. *Rasamsonia* agar medium contained per liter: 15 g of Salt fraction no.3, 30 g of cellulose, 7.5 g of Bacto peptone, 15 g of Grain flour, 5 g of KH₂PO₄, 1 g of CaCl₂.2H₂O, 20 g of Bacto agar, pH 6.0. The salt fraction no. 3 was fitting the disclosure of WO98/37179, Table 1. Deviations from the composition of this table were CaCl₂.2H₂O 1.0 g/l, KCI 1.8 g/L, citric acid 1H₂O 0.45 g/L (chelating agent). For spore batch preparation, strains were grown from stocks on *Rasamsonia* agar medium in 10 cm diameter Petri dishes for 5-7 days at 40°C. Strain stocks were stored at -80°C in 10% glycerol.

*Escherichia coli* DH5α was used for cloning purposes. Cells were grown at 37 °C in LB medium (1 % Bacto tryptone (Becton, Dickinson & Co.), 0.5 % Yeast Extract and 0.5% NaCl) supplemented with 50 µg/ml kanamycin, 100 µg/ml ampicillin, 100 µg/ml carbenicillin or 15 µg/ml chloramphenicol.

### Plasmids

pDONR P4-P1 R, pDONR 221 and pDONR P2R-P3 are multisite Gateway vectors; Kan^{R}Cm^{R} from Invitrogen, USA.
pDEST R4-R3 is a multisite Gateway vector; Amp^{R}Cm^{R} from Invitrogen, USA. pENTR221-niaD_{F1}-amdS-niaD_{F2} is a pDONR221 derivative with a [*niaD_{F1}-P_{Anid•gPdA}*-*Anid•amdS-niaD_{F2}*] cassette; Kan^{R} *Anid•amdS* Laboratory collection
pENTR41-niaD_{L} is pDONR P4-P1R with 5 prime-region of *Pchr•niaD;* Kan^{R} ; DSM lab collection
pENTR23-niaD_{R} is pDONR P2R-P3 with 3 prime-region of *Pchr*•*niaD;* Kan^{R} DSM lab collection
pAMPF21 is a *P. chrysogenum* / *E. coli* shuttle vector with *AMA1* region; Cm^{R} Phleo^{R;} Fierro et al., 1996 Curr Genet. 29(5):482-9.
pAMPF21* is pAMPF21 lacking specific restriction sites (*Hind*III, Asp718i) in the *AMA1* region; Cm^{R}; DSM lab collection
pBBK-001 is an *E. coli* plasmid containing a [P_{*Pchr*•}*_{pcbc}-DsRed.SKL-*T_{*pchr*•*penDE*}] cassette; Amp^{R}; Kiel et al., 2009 Funct Integr Genomics. 9(2):167-84.
pBBK-007 is a plasmid containing a [*P*_{*Anid*•*gpdA*}-*DsRed*.*SKL*-T_{*PChr*•*PenDE*}] cassette; Amp^{R}; Meijer et al., 2010 Appl Environ Microbiol. 76(17):5702-9.
pENTR221-stuffer is pDONR221 with portion of *P. chrysogenum ATG15* gene flanked by suitable restriction sites; Kan^{R}; Laboratory collection
pUG34-DsRed.SKL is an *E. coli* / S. *cerevisiae* shuttle vector, which contains the *Scer*•*HIS3* auxotrophic marker, the *ARS*/*CEN* replicon and the *DsRed.SKL* gene under the control of the *Scer*•*MET25* promoter (Kuravi et al, 2006 J Cell Sci. 119(19):3994-4001).

Standard recombinant DNA manipulations were carried out according to Sambrook et al. (1989 Molecular cloning, a Laboratory manual. Cold Spring Harbor Laboratory, Cold Spring Harbor, NY). Preparation of *P. chrysogenum* protoplasts and their transformation were performed in accordance with established protocols (Cantoral et al., 1987 Bio/Technol. 5, 494-497). Total DNA was isolated essentially as described by Kolar et al. (1988) Gene. 1988;62(1):127-34. In short, protoplasts were lysed in TES/SDS buffer (10 mM Tris-HCl pH 8.0, 50 mM EDTA, 150 mM NaCl, 1 % SDS) followed by phenol and chloroform extractions and ethanol precipitation. Spooled DNA was washed with 70 % ethanol, air-dried, dissolved in T₁₀E₁ (10 mM Tris-HCl pH 7.4 1 mM EDTA) and treated with RNAse (10 µg/ml). *A*. *nidulans* genomic DNA was isolated according to Chow and Kafer (see http://www.fgsc.net/fgn/chow.html).

*R. emersonii* genomic DNA was isolated from cultures grown for 16 hours in YGG medium at 42°C, 250 rpm, using the DNeasy plant mini kit (Qiagen, Hilden, Germany).

Restriction enzymes and other DNA modifying enzymes were used in agreement with the instructions of the suppliers (Fermentas Gmbh, St. Leon-Rot, Germany; Roche Diagnostics, Mannheim, Germany)). Polymerase chain reactions (PCR) were performed with Phusion polymerase (Fermentas Gmbh) for cloning purposes and Phire polymerase (Fermentas Gmbh) for colony PCR on transformants. DNA recombination reactions were performed according to the instructions of the multisite Gateway three-fragment vector construction kit (Invitrogen, USA). Southern blot analysis was performed with Hybond N⁺ membranes (G.E. Healthcare Limited, Little Chalfont, UK), the ECL Gold hybridization buffer (GE Healthcare Limited) and DNA fragments labeled with digoxigenin using the DIG labeling and detection system (Roche Diagnostics).

*P. chrysogenum, A. nidulans* and *A. niger* DNA sequences were taken from the site of the National Center for Biotechnology Information (NCBI) (http://www.ncbi.nim.nih.gov/). where also Blast analysis was performed. The *T. reesei cbh1* sequence was taken from the website of the DOE Joint Genome Institute (http://www.jgi.doe.gov/). *In silico* analysis of DNA sequences and construction of vector maps was carried out using Clone Manager 5 software (Scientific and Educational Software, Durham). Alignments of amino acid sequences were constructed using Clustal_X (Thompson et al., 1997 Nucleic Acids Res 25, 4876-82) and displayed with GeneDoc (http://www.psc.edu/biomed/genedoc).

Qualitative assays of Cbh1 activity: (i) *plate assay.* Protoplasts of P. *chysogenum* DS54465 were co-transformed with the *Δtif35::niaD*_{F1}*-amdS-niaD*_{F2} deletion cassette and pDSM-JAK-120 and selected on acetamide plates. To determine which transformants had received plasmid pDSM-JAK-120, mycelia of random transformants were placed on acetamide plates containing 100 µg/ml 4-methylumbelliferyl β-D cellobioside (MUC, Sigma, Saint Louis, Missouri, USA). To repress the expression of endogenous cellobiohydrolase activities the plates were also supplemented with 34.2 % sucrose. After 3-5 days of growth at 25 °C, the plates were visualized under UV light using a Gel Doc™ XR+ Molecular Imager (Bio-Rad, Hercules, CA, USA). Transformants that were able to convert MUC into the fluorescent substance 4-methylumbelliferone showed a clear fluorescent halo. These were also shown to harbour plasmid pDSM-JAK-120 by colony PCR.

(ii) *liquid assay.* Spores of *P. chrysogenum Δtif35* transformants stably maintaining plasmid pDSM-JAK-120 were inoculated for 2 days at 25 °C on YGG medium supplemented with 34.2 % sucrose to repress the expression of endogenous cellobiohydrolase activities. Subsequently, the cells were pelleted and aliquots of the spent medium were incubated in 50 mM sodiumacetate buffer pH 5.0, 300 µg/ml MUC for 1-16 h at 25 °C. Then the reactions were stopped by the addition of 1 volume of 10 % Na₂CO₃. Finally, the reaction mixtures were visualised under UV light using a Gel Doc™ XR+ Molecular Imager. Media that contained significant amounts of Cbh1 activity showed clear fluorescence. As control, spent media from identically grown *P*. *chrysogenum* DS54465 cells were used, which showed no significant activity.

### General techniques

Crude extracts of *Penicillium chrysogenum* cells were prepared as described previously (Kiel et al., 2009). Protein concentration was determined using the RC/DC Protein Assay (Bio-Rad, USA) or the Bio-Rad Protein Assay system using bovine serum albumin as a standard. SDS-polyacrylamide gel electrophoresis and Western blotting were performed in accordance with established protocols.

*Aspergillus niger* transformants were selected on acetamide media and colony purified according to standard procedures, for instance as described in EP 0 635 574 B. Examples of the general design of expression vectors for gene over-expression and disruption vectors, transformation, use of markers and media can be found in WO2005/095624 and EP 0 635 574 B.

*Rasamsonia emersonii* transformants were selected on phleomycin media and colony purified and tested according to procedures as described in WO2011/054899.

Gene replacement vectors were designed according to known principles and constructed according to routine cloning procedures. In essence, these vectors comprise approximately 1 - 2 kb flanking regions of the respective ORF sequences, to target for homologous recombination at the predestined genomic loci. They may contain the *A. nidulans* bi-directional *amd*S selection marker for transformation. The method applied for gene replacements in all examples herein uses linear DNA, which integrates into the genome at the homologous locus of the flanking sequences by a double cross-over, thus substituting the gene to be deleted by the *amdS* gene. Loss of the *amd*S marker can be select for by plating on fluoro-acetamide media.

*Analysis of fluorescence in colonies and cells.* Colonies showing DsRed fluorescence were identified using a Night Sea Blue Star high intensity LED flashlamp and VG1 filter glasses (Tektite Industries Inc., Trenton, N.J., USA; http://www.nightsea.com/gfp.htm). Although this lamp is mainly used for GFP fluorescence, it is also functional for DsRed, when fluorescence is strong.

Fluorescence microscopy studies were performed using a Zeiss Axioskop microscope (Carl Zeiss, Göttingen, Germany).

### Example 1 (comparative example)

In this comparative example, the stability of the *AMA1* plasmid in *P*. *chrysogenum* NHEJ-deficient cells was investigated.

To create an *AMA1*-containing control plasmid with a constitutively expressed *DsRed.SKL* gene, a 2108 bp DNA fragment comprising the [P*_{Anid·gpdA}-DsRed.SKL-T_{Pchr·penDE}*] cassette was isolated from plasmid pBBK-007 with *Eco*RV + *Not*I and cloned between the *BgI*II (blunted by Klenow treatment) and *Not*I sites of plasmid pAMPF21* yielding plasmid pDSM-JAK-109.

*P. chrysogenum* DS54465 protoplasts were transformed with plasmid pDSM-JAK-109 (Fig 1.), which contains a dominant phleomycin resistance marker and a constitutively produced DsRed.SKL protein (SEQ ID. NO. 35) that can be easily visualized by fluorescent techniques. DsRed.SKL ORF is shown in SEQ ID NO: 34. Upon illumination with the high intensity LED flashlamp, all Phleo⁺ transformants displayed red fluorescence. However, when mycelia was allowed to sporulate on non-selective R-agar plates, and the resulting spores plated on non-selective media, most of the resulting colonies had already lost the plasmid (>90 % non-fluorescent "white" colonies). This confirms the instable nature of *AMA1* plasmids in *P. chrysogenum* NHEJ-deficient cells.

In the following Examples we will show that replicating vectors, such as the *AMA1* plasmid, which contain an essential gene as selection marker are fully stable in a host that lacks the genomic copy of this gene. One way to do this is by co-transformation during which the essential gene is deleted, while simultaneously the lethal effect of its deletion is complemented by the presence of the essential gene on the replicating vector.

### Example 2 Preparation of expression construct pDSM-JAK-105 with inducible promoter.

As putative essential gene, to be used as marker for a novel vector, we choose the *P. chrysogenum tif35* gene (*Pc22g19890*), encoding the g subunit of the core complex of translation initiation factor 3 (eIF3g; Phan et al., 1998 Mol Cell Biol. 18(8):4935-46*)*. *Pchr•tif35* gene, ORF, cDNA and protein are shown in SEQ ID NO. 37, 38, 39 and 40, respectively Plasmid pDSM-JAK-105 allowing creation of a strain in which *P. chrysogenum tif35* is placed under the control of the inducible *niiA* promoter was constructed using Gateway Technology as follows.

A 3951 bp DNA fragment comprising the complete *P. chrysogenum niaD* gene and the *niiA* promoter region (nt 2778005 to 2781898 in Genbank AM920428.1) was amplified with the following oligonucleotides: using genomic DNA of strain DS17690 as template and recombined into vector pDONR 221 yielding plasmid pDSM-JAK-101.

A 1576 bp DNA fragment comprising a region approx. 1.5 kb upstream of the *P*. *chrysogenum tif35* coding sequence (CDS; nt 4686813 to 4688338 in Genbank AM920437.1) was amplified with the following oligonucleotides using P. *chrysogenum* DS54465 DNA as template and recombined into vector pDONR P4-P1R yielding plasmid pDSM-JAK-102.

A 1493 bp DNA fragment comprising the *P. chrysogenum tif35* CDS and terminator region (nt 4689913 to 4691355 in Genbank AM920437.1) was amplified with the following oligonucleotides: using *P. chrysogenum* DS54465 DNA as template and recombined into vector pDONR P2R-P3, yielding plasmid pDSM-JAK-103.

Plasmids pDSM-JAK-101, pDSM-JAK-102 and pDSM-JAK-103 were recombined with vector pDEST R4-R3, yielding pDSM-JAK-105 (Fig.2) which allowed for the creation of a strain in which *P. chrysogenum tif35* is placed under the control of the inducible *niiA* promoter. In this construct, the *niaD* gene encoding nitrate reductase functions as a selection marker.

### Example 3 Construction of a P. chrysogenum strain with a nitrate-inducible tif35 gene.

Plasmid pDSM-JAK-105 of Example 2 was linearized with *Aat*II in the vector region and transformed into protoplasts of *P. chrysogenum* DS58274. In this strain an inactivated *niaD* locus carries a *GFP.SKL* expression cassette allowing its use as both auxotropic marker as well as a transformation control. Using fluorescence microscopy, we observed that out of 52 nitrate-prototrophic transformants analysed, 44 had retained GFP fluorescence. Colony PCR using the following oligonucleotides:
DSM-JAK-109 5'-CAGTTTACACTCAACCCCAATCCAG-3' (SEQ ID NO.7)
3-prime-niaD-forward 5'-AGGTTGGTGGAGAAGCCATTAG-3' (SEQ ID NO.8)
showed that at least half of these carried the *P_{niiA}-tif35* locus correctly recombined at the *tif35* locus. Multiple independent [P*_{niiA}-tif35*]-containing transformants were identified, purified by sporulation and NO₃⁺ selection and further analysed. Conidiospores were produced on R agar plates supplemented with nitrate and allowed to germinate on plates containing either ammonium or nitrate as sole nitrogen source. Spore germination was fully absent on ammonium plates, but was normal on nitrate plates. This implies that *Pchr•tif35* is indeed an essential gene.

### Example 4 Construction of a P. chrysogenum tif35 deletion cassette.

To delete the genomic copy of the *P. chrysogenum tif35* gene, plasmid pDSM-JAK-106 (Fig. 3) was constructed by Gateway technology. A 1654 bp DNA fragment comprising the region downstream from the *P. chrysogenum tif35* terminator (nt 4691355 to 4692956 in Genbank AM920437.1) was amplified with the following oligonucleotides: using *P. chrysogenum* DS54465 DNA as template, recombined into vector pDONR P2R-P3, yielding plasmid pDSM-JAK-104. Plasmids pDSM-JAK-102, pENTR221-niaD_{F1}-amdS-niaD_{F2} and pDSM-JAK-104 were recombined with vector pDEST R4-R3, yielding plasmid pDSM-JAK-106 (Fig. 3).

### Example 5 Construction of an alternative P. chrysogenum tif35 deletion cassette.

For easier separation of the Δ*tif35* cassette from vector DNA, a derivative of plasmid pDSM-JAK-106 from Example 4 with an extra *Apa*I site was constructed. For the construction of this plasmid, a 1660 bp DNA fragment comprising the region downstream from the *P. chrysogenum tif35* terminator (nt 4691355 to 4692956 in Genbank AM920437.1) was amplified with the following oligonucleotides: using *P. chrysogenum* DS54465 DNA as template, recombined into vector pDONR P2R-P3, yielding plasmid pDSM-JAK-121. Plasmids pDSM-JAK-102, pENTR221-niaD_{F1}-amdS-niaD_{F2} and pDSM-JAK-121 were recombined with vector pDEST R4-R3, yielding plasmids pDSM-JAK-122.

### Example 6 Construction of AMA1 plasmid pDSM-JAK-108 containing P. chrysogenum tif35 and DsRed.SKL marker.

An *AMA1* plasmid containing *P. chrysogenum tif35* was constructed as follows. A 1368 bp DNA fragment comprising the constitutive promoter of the *Aspergillus nidulans AN0465* gene encoding the ribosomal protein S8 (nt 3414332 to 3415681 in Genbank BN001308) was amplified with oligonucleotides
DSM-JAK-201 5'- AGAGGTACCGAGTTATAGACGGTCCGGCATAGG -3' (SEQ ID. NO.12).
DSM-JAK-202 5'- AGAGGATCCGTTTGCTGTCTATGTGGGGGACTG -3' (SEQ ID. NO.13).
using genomic DNA from *A*. *nidulans* FGSC A4 (ATCC38163) as template. The PCR fragment was digested with *Asp*718i + *Bam*HI and cloned between the *Asp*718i and *Bam*HI sites of plasmid pBBK-001, thereby replacing the *P. chrysogenum pcbC* promoter. The resulting plasmid was designated pDSM-JAK-201.

An 886 bp DNA fragment comprising the terminator of the *A*. *nidulans act (AN6542)* gene encoding gamma actin (nt 2366704 to 2365833 in Genbank BN001301) was amplified with oligonucleotides
DSM-JAK-203 5'-GGGGTGCTTCTAAGGTATGAGTCGCAA-3' (SEQ ID. NO.14).
DSM-JAK-204 5'-AGAACGCGTTAACGCAGGGTTTGAGAACTCCGATC -3' (SEQ ID. NO.15).
using *A*. *nidulans* FGSC A4 DNA as template. The PCR fragment was digested with *Mlu*I and cloned between the *Sma*I and *Mlu*I sites of plasmid pDSM-JAK-201, thereby replacing the *P. chrysogenum penDE* terminator. The resulting plasmid was designated pDSM-JAK-202.

A 2971 bp fragment containing the [*P_{AN0465}*-*DsRed*.*SKL*-*T_{Anid•act}*] expression cassette was isolated from plasmid pDSM-JAK-202 with *Hpa*I and *Kpn*I and cloned into plasmid pAMPF21*, a derivative of plasmid pAMPF21 that was modified by removing specific restriction sites (*Hind*III, *Asp*718i) in the *AMA1* region, digested with *Hind*III (blunted by Klenow treatment)+Kpnl, thus yielding plasmid pDSM-JAK-107.

A 3036 bp DNA fragment comprising the *tif35* coding sequence together with its promoter and terminator regions (nt 4688344 to 4691352 in Genbank AM920437.1) was amplified with oligonucleotides
DSM-JAK-111 5'-AGAGGATCCGAGGAAGACGTGATCAGAGTAAGC-3' (SEQ ID. NO.16).
DSM-JAK-112
5'-GAAAGCGGCCGCGGTACCGTGCTTGGGATGTTCCATGGTAGC-3' (SEQ ID. NO.17).
using genomic *P. chrysogenum* DS54465 DNA. The PCR fragment was digested with *Not*I and *Bam*HI and cloned between the *NotV* and *Bg*III sites of plasmid pDSM-JAK-107, yielding plasmid pDSM-JAK-108 (Fig 4).

In this way an *E. coli* / *P. chrysogenum* shuttle vector was constructed which contains the *Pchr•tif35* expression cassette, the *AMA1* replicon for extra-chromosomal replication, and a constitutively expressed *DsRed.SKL* gene that can be easily visualized by fluorescent techniques. The [*P_{AN0465}*-*DsRed*.*SKL*-*T_{Anid•act}*] and *Pchr•tif35* expression cassettes as present on plasmid pDSM-JAK-108 are shown in SEQ ID No. 36 and 37, respectively. Since the expression signals of the *DsRED.SKL* cassette on this plasmid originate from the *A. nidulans* genome, plasmid pDSM-JAK-108 has no significant similarity with the genome of a *P. chrysogenum* strain other than *tif35,* nor to most other filamentous fungi including *Aspergillus niger.*

### Example 7 Stabilised vector host-system in P. chrysogenum

Plasmid pDSM-JAK-108 was co-transformed in circular form with a *P*. *chrysogenum* Δ*tif35* cassette into protoplasts of *P. chrysogenum* DS54465 or DS61187. The Δ*tif35* cassette was released either from pDSM-JAK-106 (Example 3) by *Apa*I + partial *Bcl*I digestion (yielding a 9119 bp DNA fragment) or from pDSM-JAK-122 (Example 4) by *Apa*I digestion (yielding a 9224 bp DNA fragment) and purified from agarose gel.

Transformants were selected on acetamide plates. Red fluorescent colonies harbouring the *DsRed.SKL*-expressing plasmid were identified with high frequency (30-50 %). In the majority of the cases plasmid pDSM-JAK-108 was present in a fully intact form as demonstrated by colony PCR using oligonucleotides DSM-JAK-201 (SEQ ID. NO.12) and DSM-JAK-204 (SEQ ID. NO. 15) that amplify a 2971 bp fragment containing the [*P_{AN0465}*-*DsRed*.*SKL*-T_{*Anid*•*act*}] expression cassette (SEQ ID. No.36), by Southern blotting and by retransformation into *E. coli* DH5α followed by extensive restriction analysis. We observed that the red fluorescent phenotype was fully stable during continued mycelial growth on non-selective media and also upon conidiospore formation and germination on non-selective medium for at least ten cycles. Also curing the strain of the *amdS* marker by selection on replication slippage events using fluoroacetamide plates did not affect the segregational and recombinational stability of the plasmid. This implies the presence of a fully stable replicating plasmid in *P*. *chrysogenum* cells.

Deletion of the genomic copy of *tif35* in transformants was demonstrated by colony PCR using the following oligonucleotide combinations:
DSM-JAK-109: 5'-CAGTTTACACTCAACCCCAATCCAG-3' (SEQ ID. NO. 7) + 5-prime-niaD-return: 5'- CACGTAGCATACAACCGTGTCG -3' (SEQ ID. NO. 18) (expected 1647 bp) and
3-prime-niaD-forward 5'- AGGTTGGTGGAGAAGCCATTAG-'3 (SEQ ID. NO. 8) + DSM-JAK-110 5'- GATGCCTTGTGGGAAATTAACCAG -'3 (SEQ ID. NO. 19). (expected 1776 bp)

These should only amplify a DNA fragment of the indicated size upon correct recombination at the *tif35* locus. Multiple independent PCR positive transformants were identified and purified by sporulation and selection of single spores on acetamide selection plates. Southern blot analysis showed correct deletion of *tif35.* Multiple independent Δ*tif35* strains carrying a replicating plasmid with the complementing *tif35* gene were identified.

### Example 8 Construction of marker free strains

In the Δ*tif35* strains, the *Anid•amdS* marker is flanked by a 1.5 kb repeat comprising part of *P. chrysogenum niaD* (F1 and F2), allowing loss of the marker by replication slippage. To obtain marker-free strains, four independently isolated Δ*tif35* strains were placed on sporulation agar and streaked out to single spore on fluoroacetamide plates. From each plate two independent colonies were selected, purified by another round of sporulation and re-selection of single spores on fluoroacetamide plates. Southern blot analysis showed correct removal of the *amdS* marker from the *tif35* locus.

### Example 9 The stability of AMA1 plasmid pDSM-JAK-108 carrying a tif35 expression cassette is dependent on the deletion of the genomic copy of the tif35 gene.

To demonstrate that the stability of plasmid pDSM-JAK-108 was determined by the absence of the genomic copy of the *tif35* gene and not caused by recombination with the genome, an additional copy of *P. chrysogenum tif35* was placed at the genomic *niaD* locus. To this end plasmid pDSM-JAK-116 was constructed by Gateway technology as follows:
A 3070 bp DNA fragment comprising the *tif35* CDS together with its promoter and terminator regions (nt 4688343 to 4691352 in Genbank AM920437.1) was amplified with oligonucleotides using *P. chrysogenum* DS54465 genomic DNA as template and recombined into plasmid pDONR 221, yielding plasmid pDSM-JAK-115.

Plasmids pENTR41-niaD_{L}, pDSM-JAK-115 and pENTR23-niaD_{R} were recombined with vector pDEST R4-R3 yielding plasmid pDSM-JAK-116 (Fig. 5).

The *[niaD*_{L}-*tif35-niaD*_{R}] integration cassette was released from plasmid pDSM-JAK-116 by *Not*I + *Kpn*I digestion, purified from agarose gels (as a 6778 bp DNA fragment), and transformed into protoplasts of two independently isolated strains of pDSM-JAK-108 (Δ*tif35*::*niaD*_{F1}-*amdS*-*niaD*_{F2}). Transformants were selected on chlorate plates, followed by colony PCR using oligonucleotides
DSM-JAK-126 5'- GTTCTTGAATAGCCGAGGACTCAC -3' (SEQ ID. NO.22)
DSM-JAK-127 '5'- CATCCTCCCCTTCTGTTGGCATAG -3' (SEQ ID. NO.23)
that should only amplify a DNA fragment of 1923 bp upon correct integration of the *tif35* gene at the *niaD* locus. Multiple independent PCR-positive transformants were identified. All of these had lost the red fluorescent phenotype, indicating a loss of the replicating plasmid pDSM-JAK-108. Eight transformants were further analysed by Southern blotting and demonstrated correct integration of *tif35* at the *niaD* locus, the presence of the original *Δtif35::niaD_{F1}*-*amdS*-*niaD_{F2}* locus and the absence of the replicating plasmid pDSM-JAK-108. Thus, plasmid pDSM-JAK-108 is highly stable in a strain lacking *tif35,* but becomes mitotically unstable again when chromosomal *tif35* expression is restored, confirming its extra-chromosomal nature.

### Example 10 Construction of an AMA1-containing plasmid expressing a Trichoderma reesei cbh1 gene and the P. chrysogenum tif35 gene.

To demonstrate that the newly developed stable host/vector system can be used for biotechnological purposes, we expressed the *Trichoderma reesei cbh1* gene encoding a secreted cellobiohydrolase from a replicating plasmid carrying *Pchr•tif35* as selection marker. An expression cassette comprising a codon pair-optimized *T. reesei cbh1* gene flanked by non-homologous *A. nidulans* expression signals was constructed as follows.

A 1157 bp DNA fragment comprising the constitutive promoter of the *A*. *nidulans tef* gene *(AN4218)* encoding the translation elongation factor alpha (nt 1654144 to 1655266 in Genbank BN001302.1) was amplified with oligonucleotides using *A*. *nidulans* FGSC A4 genomic DNA as template. The PCR fragment was digested with *Hind*III + *Bgl*II and cloned between the *Hind*III and *Bgl*II sites of plasmid pENTR221-stuffer, yielding plasmid pDSM-JAK-203.

A 705 bp DNA fragment comprising the terminator of the *A*. *nidulans trpC* gene (*AN0648*) encoding anthranilate synthase component 2 (nt 2848474 to 2849165 in Genbank BN001308.1) was amplified with oligonucleotides
DSM-JAK-210 5'-AGAAGATCTGATCGTTGGTGTCGATGTCAGCTC-3' (S E Q ID. NO. 26)
DSM-JAK-211 5'- GGGGTACACAGTACACGAGGACTTCTAG -3' (SEQ ID. NO. 27)
using A. *nidulans* FGSC A4 DNA as template. The PCR fragment was digested with *Bg*III and cloned between the *Bgl*II and *Sma*I sites of plasmid pDSM-JAK-203, yielding plasmid pDSM-JAK-206 (Fig. 6).

The wild type *T. reesei cbh1* cDNA (SEQ ID. No. 28) was obtained from the website of the DOE Joint Genome Institute and optimized for expression in *P*. *chrysogenum* and *A*. *niger.*

A 1583 bp DNA fragment comprising the codon pair optimized *Tree•cbh1* cDNA sequence was synthesized at GeneArt AG (Regensburg, Germany), digested with *Sfi*1 and cloned into *Sfi*1-linearized vector pMK-RQ (Gene Art). The resulting plasmid was designated pDSM-JAK-117(Fig. 7).

A 1564 bp DNA fragment comprising the codon pair optimized *Tree•cbh1* cDNA sequence was isolated from pDSM-JAK-117 with *Eco*RI + *Bam*HI and cloned between the *Eco*RI and *Bgl*II sites of plasmid pDSM-JAK-206, yielding pDSM-JAK-118.

A 3387 bp DNA fragment comprising the P_{*Anid*•}*_{tef}-Tree*•*cbh1^{opt}*-T_{*Anid*•*trpC*} expression cassette was isolated from pDSM-JAK-118 with *Kpn*I + *Sma*I and cloned between the *Kpn*I and *Hind*III (blunted by Klenow treatment) sites of plasmid pAMPF21*, yielding plasmid pDSM-JAK-119.

A 3036 bp DNA fragment comprising the *tif35* coding sequence together with its promoter and terminator regions (nt 4688344 to 4691352 in Genbank AM920437.1) was amplified with oligonucleotides DSM-JAK-111 (SEQ ID. NO. 16) and DSM-JAK-112 (SEQ ID. NO. 17) using genomic *P. chrysogenum* DS54465 DNA. The PCR fragment was digested with *Not*I and *Bam*HI and cloned between the *Not*I and *Bgl*II sites of plasmid pDSM-JAK-119, yielding plasmid pDSM-JAK-120 (Fig. 8).

### Example 11 Production of a heterologous protein from a stabilised AMA1-plasmid in P. chrysogenum.

Plasmid pDSM-JAK-120 was co-transformed in circular form with the *P*. *chrysogenum Δtif35::niaD*_{F1}*-amdS-niaD*_{F2} cassette from pDSM-JAK-122 (Example 5) into protoplasts of *P. chrysogenum* DS54465. Transformants were selected on acetamide plates. Multiple independent transformants harbouring the *Tree•cbh1^{opt}* expressing plasmid were identified with high frequency (30-40 %) by colony PCR using oligonucleotides DSM-JAK-205 (SEQ ID. NO.24) and DSM-JAK-211 (SEQ ID. NO.27) that amplify the [P*_{Anid•tef} - Tree•cbh1^{opt} -* T_{*Anid*•trpC}] cassette. Furthermore, these colonies were analysed for the production of active *Tree•cbh1^{opt}* enzyme on acetamide selection plates supplemented with 4-methylumbelliferyl β-D-cellobioside (MUC). The plasmid-containing transformants indeed secreted cellobiohydrolase as determined by Mass Spec and activity measurements. The stability of transformants was demonstrated by at least four rounds of successive sporulation/germination on non-selective R-agar plates followed by colony formation from single spores on acetamide plates, a procedure that does not select for the plasmid.

### Example 12 AMA1 plasmid stabilisation in A.niger.

The same *AMA1* plasmid which was used in the previous Examples for the transformation of *Penicillium,* was subsequently used to test the system of the present invention in the biotechnologically important filamentous fungus *A. niger.* First, the *A*. *niger tif35* gene was identified by alignment (An16g05260) *Anig•tif35* gene, ORF, cDNA and protein are shown in SEQ ID NO. 29, 30, 31 and 32, respectively. An *Anig•tif35::Anid•amdS* deletion cassette (SEQ ID No. 33) was constructed by cloning a functional *amd*S cassette, comprising the *A. nidulans amdS* ORF and terminator, expressed from the *A*. *nidulans gpdA* promoter, between 5' and 3' flanking regions of *Anig•tif35.* To this end, the flanking regions were PCR amplified from genomic DNA of WT2 using primers comprising unique restriction sites (*Not*1, *Asc*I and *Fse*I, as indicated in Fig. 9) to facilitate cloning into a suitable *E. coli* vector Then A. *niger* strain WT 2 was transformed according to routine procedures with the *AMA1* plasmid pDSM-JAK-108 (Fig. 4), containing the DsRed.SKL marker and the *Pchr•tif35* homologue and a linear *Not*I fragment containing the *Anig•tif35::Anid•amdS* deletion cassette. Correct integration by double homologous recombination would result in substitution of the *Anig•tif35*ORF (including part of its promoter) by *amd*S. Approximately 350 transformants were obtained by selection on acetamide medium and about 90% were red when irradiated with blue light. Colonies of the non-transformed WT 2 did not show the red colour but had the normal "white" colour. A subset of 40 red transformants was inspected in more detail.

These transformants were colony purified by cultivation on non-selective (PDA) medium (Potato Dextrose Agar, Oxoid) after which spores were re-streaked on PDA plates to obtain single colonies. From each transformants, virtually all single colony isolates remained red. The red colour persisted even after five subsequent cycles of sporulation and single colony isolation on PDA medium as described above. This showed that the DsRed.SKL marker was stably present even after prolonged cultivation on non-selective medium. In our case we tested for 10 subsequent cycles of sporulation and single colony isolation. PCR diagnostics confirmed that in all 40 transformants tested, the genomic *Anig•tif35* was substituted by the *amd*S cassette. Southern blot and transformation of *E. coli* with total DNA from these same transformants confirmed the presence of the intact episomal pDSM-JAK-108. This showed that the plasmid pDSM-JAK-108 was stably present even after prolonged cultivation on non-selective medium. These data imply that plasmid pDSM-JAK-108 is a suitable basis for the construction of multi-purpose vectors that can be stably maintained in multiple filamentous ascomycetes.

### Example 13 Identification of the ReKu80 gene of Rasamsonia emersonii and construction of deletion vectors.

Genomic DNA of *Rasamsonia emersonii* strain CBS393.64 was sequenced and analyzed. The gene with translated protein annotated as homologues to known *ReKu80* gene was identified.

Sequences of the *R. emersonii ReKu80* gene, comprising the genomic sequences of the open reading frames (ORF) (with introns) and approximately 2500 bp of the 5' and 3' flanking regions, cDNA and protein sequences, are shown in SEQ ID NOs: 41 to 43 respectively. Two replacement vectors for *ReKu80,* pEBA1001 and pEBA1002, were constructed according to routine cloning procedures (see Figures 10 and 11). The insert fragments of both vectors together can be applied in the so-called "bipartite gene-targeting" method (Nielsen *et al.,* 2006, 43: 54-64). This method is using two non-functional DNA fragments of a selection marker which are overlapping (see also WO2008113847 for further details of the bipartite method) together with gene-targeting sequences. Upon correct homologous recombination the selection marker becomes functional by integration at a homologous target locus. The deletion vectors pEBA1001 and pEBA1002 were designed as described in WO 2008113847, to be able to provide the two overlapping DNA molecules for bipartite gene-targeting.

The pEBA1001 vector comprises a 2500 bp 5' flanking region of the *ReKu80* ORF for targeting in the *ReKu80* locus, a lox66 site, and the 5' part of the ble coding region driven by the *A.nidulans gpdA* promoter (Figure 10). The pEBA1002 vector comprises the 3' part of the ble coding region, the *A.nidulans trpC* terminator, a lox71 site, and a 2500 bp 3' flanking region of the *ReKu80* ORF for targeting in the *ReKu80* locus (Figure 11).

### Example 14 Cloning of pEBA513 for transient expression of cre recombinase.

pEBA513 was constructed by DNA2.0 (Menlo Park, USA) and contains the following components: expression cassette consisting of the *A.niger* glaA promoter, ORF encoding cre-recombinase (AAY56380) and *A.nidulans* niaD terminator; expression cassette consisting of the *A.nidulans gpdA* promoter, ORF encoding hygromycin B resistance protein and *P. chrysogenum* penDE terminator (Genbank: M31454.1, nucleotides 1750-2219); pAMPF21 derived vector containing the AMA1 region and the CAT chloramphenicol resistance gene. Figure 12 represents a map of pEBA513.

### Example 15 Inactivation of the ReKu80 gene in Rasamsonia emersonii.

Linear DNA of the deletion constructs pEBA1001 and pEBA1002 were isolated and used to transform *Rasamsonia emersonii* CBS393.64 using method as described earlier in WO2011\054899. These linear DNAs can integrate into the genome at the *ReKu80* locus, thus substituting the *ReKu80* gene by the *ble* gene as depicted in Figure 13. Transformants were selected on phleomycin media and colony purified and tested according to procedures as described in WO2011/054899. Growing colonies were diagnosed by PCR for integration at the *ReKu80* locus using a primer in the gpdA promoter of the deletion cassette and a primer directed against the genomic sequence directly upstream of the 5' targeting region. From a pool of approximately 250 transformants, 4 strains showed a removal of the genomic *ReKu80* gene.

Subsequently, 3 candidate *ReKu80* knock out strains were transformed with pEBA513 to remove the *ble* selection marker by transient expression of the cre recombinase. pEBA513 transformants were plated in overlay on regeneration medium containing 50 µg/ml of hygromycin B. Hygromycin-resistant transformants were grown on PDA containing 50 µg/ml of hygromycin B to allow expression of the cre recombinase. Single colonies were plated on non-selective *Rasamsonia* agar medium to obtain purified spore batches. Removal of the ble marker was tested phenotypically by growing the transformants on media with and without 10 µg/ml of phleomycin. The majority (>90%) of the transformants after transformation with pEBA513 (with the cre recombinase) were phleomycin sensitive, indicating removal of the pEBA1001 and pEBA1002-based ble marker. Removal of the pEBA513 construct in ble-negative strains was subsequently diagnosed phenotypically by growing the transformants on media with and without 50 µg/ml of hygromycin. Approximately 50% of the transformants lost hygromycin resistance due to spontaneously loss of the pEBA513 plasmid. Deletion of the *ReKu80* gene and the absence of the pEBA513 plasmid was confirmed by PCR analysis and Soutern blotting

Strain deltaKu80-2 was selected as a representative strain with the Ku80 gene inactivated.

### Example 16 Construction of a R. emersonii ReTif35 deletion cassette

Genomic DNA of *Rasamsonia emersonii* strain CBS393.64 was sequenced and analyzed. The gene with translated protein annotated as *ReTif35* was identified. Sequences of the *R. emersonii ReTif35* gene, comprising the genomic sequence of the ORF and approximately 1500 bp of the 5' and 3' flanking regions, cDNA and protein sequence, are shown in sequence listings 44 to 46.

Gene replacement vectors for *R. emersoniic ReTif35* gene were designed using the bipartite gene-targeting method and constructed according to routine cloning procedures (see Figures 14 and 15). The pEBA1007 construct comprises a 1500 bp 5' flanking region of the *ReTif35* ORF for targeting in the *ReTif35* locus, a lox66 site, and the 5' part of the ble coding region driven by the *A.nidulans gpdA* promoter (Figure 14). The pEBA1008 construct comprises the 3' part of the ble coding region, the *A.nidulans trpC* terminator, a lox71 site, and a 1500 bp 3' downstream flanking region of the *ReTif35* ORF for targeting in the *ReTif35* locus (Figure 15).

### Example 17 Stabilised vector host-system in R. emersonii

*Rasamsonia emersonii* CBS393.64 strain was co-transformed with circular plasmid pDSM-JAK-108 and linear DNA of the *ReTif35* deletion constructs pEBA1007 and pEBA1008 using method earlier described in patent WO2011054899. The *ReTif35* deletion constructs integrate at the *ReTif35* locus, thus substituting the *ReTif35* gene by the *ble* gene as depicted in Figure 16. To compensate for the deletion of essential gene *ReTif35,* pDSM-JAK-108 was co-transformed carrying the *P. chrysogenum tif35* expression expression cassette. In order to easily detect the presence of the pDSM-JAK-108 plasmid, the plasmid also contains a *DsRed.SKL* expression cassette. Transformants were selected on phleomycin media as described in WO2011054899.

Red fluorescent colonies harbouring the *tif35-* and *DsRed.SKL*-expressing plasmid were identified with high frequency (∼30%). Red transformants were colony purified by cultivation on non-selective *Rasamsonia* agar medium after which spores were re-streaked on *Rasamsonia* agar medium plates to obtain single colonies. From each transformant, all single colony isolates remained red. The red colour persisted even after nine subsequent cycles of sporulation and single colony isolation on *Rasamsonia* agar medium as described above. This showed that the DsRed.SKL marker was stably present even after prolonged cultivation on non-selective medium.

In contrast, in transformants that were co-transformed with pDSM-JAK-108 and pAN8 that still contained the essential gene *ReTif35* in its genome, some red colonies were observed 4 days after transformation, but after 7 days no red colonies were observed at all. These data demonstrate that without selection using the essential gene *tif35* the *AMA1* plasmid is rapidly lost during propagation in *Rasamsonia emersonii.*

PCR diagnostics and Southern blots confirmed that in all tested red fluorescent transformants, the genomic *R. emersonii ReTif35* gene was substituted by the *ble* cassette. In order to determine whether pDSM-JAK-108 was still episomal in the cells, Southern blots were performed using pDSM-JAK-108 plasmid as probe. To determine whether pDSM-JAK-108 was intact in the cells, total DNA was isolated from red fluorescent transformants to transform *E. coli* and *E. coli* colonies were analysed for the presence of intact pDSM-JAK-108. Both Southern blot analysis and restriction enzyme analysis of DNA isolated from *E.coli* transformants confirmed the presence of the intact episomal pDSM-JAK-108 in red fluorescent *R. emersonii* transformants. This showed that the plasmid pDSM-JAK-108 was stably present even after prolonged cultivation on non-selective medium.

In conclusion, these findings indicate that in *R. emersonii* strains in which the essential gene *ReTif35* is deleted, the episomal *AMA1* plasmid pDSM-JAK-108 expressing the *Pchr*•tif35 gene is mitotically stable.

### Example 18 Construction of a P. chrysogenum aur1 deletion cassette.

To demonstrate that the use of an essential gene as a tool to stabilize replicating plasmids in filamentous fungi is not limited to the *tif35* gene, we utilized an alternative essential gene to stabilize an *AMA1* plasmid in *P. chrysogenum.* For this we chose to use the *A. nidulans aur1* gene encoding the enzyme phosphatidylinositol:ceramide phosphoinositol transferase, which is required for sphingolipid synthesis. In multiple fungal species this gene has been demonstrated to be essential. To delete the genomic copy of the *P. chrysogenum aur1 (Pc12g15520)* gene, plasmid pDSM-JAK-139 (Fig. 21) was constructed by Gateway technology. Two DNA fragments of 1538 bp and 1406 bp comprising the region upstream and downstream from the *P*. *chrysogenum aur1* gene, respectively, (nt 3709510 to 3710991 and 3712509 to 3713859 in Genbank AM920427.1) were amplified with the following oligonucleotide combinations:
DSM-JAK-164
   5'-GGGGACAACTTTGTATAGAAAAGTTGGGCCCAACGCATGTGTACGAGAGTCAAGG -3' (SEQ ID NO: 47) +
DSM-JAK-165
   5'- GGGGACTGCTTTTTTGTACAAACTTGAGACGGAAGGAGATCGCGTAACAG -3' (SEQ ID NO: 48) and
DSM-JAK-166
   5'- GGGGACAGCTTTCTTGTACAAAGTGGGGCGCAGTCCATTCTTGCATCTAC -3' (SEQ ID NO: 49) +
DSM-JAK-167
   5'- GGGGACAACTTTGTATAATAAAGTTGGGCCCAGCCACTTCTTGTATCACGGAT-3'
   (SEQ ID NO: 50), repectively,
using *P. chrysogenum* DS54465 DNA as template, recombined into vector pDONR P4-P1R and pDONR P2R-P3, respectively, yielding plasmids pDSM-JAK-137 and pDSM-JAK-138. Plasmids pDSM-JAK-137, pENTR221-niaD_{F1}-amdS-niaD_{F2} and pDSM-JAK-138 were recombined with vector pDEST R4-R3, yielding plasmid pDSM-JAK-139 (Fig. 21).

### Example 19 Construction of AMA1 plasmid pDSM-JAK-136 containing Aspergillus nidulans aur1 and DsRed.SKL marker.

An *AMA1* plasmid containing *A*. *nidulans aur1* was constructed as follows. A 3438 bp DNA fragment comprising the *aur1* coding sequence together with its promoter and terminator regions (nt 351475 to 348062 in Genbank BN001303.1) was amplified with oligonucleotides
DSM-JAK-162 5'- AGAGAGGATCCGAGTTGGCCAGTTGACAACCTGAG -3' (SEQ ID. NO: 51) and
DSM-JAK-163 5'- AGAGAGCGGCCGCGAGTATGAGCGATCGACACGAATG -3' (SEQ ID NO. 52),
using genomic *A. nidulans* FGSC A4 DNA as template. The PCR fragment was digested with *Not*I and *Bam*HI and cloned between the *Not*I and *Bgl*II sites of plasmid pDSM-JAK-107, yielding plasmid pDSM-JAK-136 (Fig. 20). In this way an *E. coli* / *P. chrysogenum* shuttle vector was constructed which contains the *Anid•aur1* expression cassette, the *AMA1* replicon and the *DsRed.SKL* gene. Plasmid pDSM-JAK-136 has no significant similarity with the genome of a *P. chrysogenum* strain other than the *aur1* coding sequence, nor to most other filamentous fungi including *Aspergillus niger.*

### Example 20 Stabilised vector host-system in P. chrysogenum using aur1

Plasmid pDSM-JAK-136 (Example 20) was co-transformed in circular form with a *P. chrysogenum* Δ*aur1* cassette into protoplasts of *P. chrysogenum* DS54465. The *P*. *chrysogenum* Δ*aur1* cassette was released by *Apa*I digestion from pDSM-JAK-139 (Example 21), yielding a 9145 bp fragment, and purified from agarose gel.

Transformants were selected on acetamide plates. As described above (Example 7), red fluorescent colonies harbouring the *DsRed.SKL*-expressing plasmid were identified with high frequency. In the majority of the cases plasmid pDSM-JAK-136 was present in a fully intact form as demonstrated by colony PCR using oligonucleotides DSM-JAK-162 (SEQ ID. NO: 51) and DSM-JAK-163 (SEQ ID. NO: 52) that amplify a 3438 bp fragment containing the *Anid•aur1* expression cassette (SEQ ID. NO: 53), by Southern blotting and by retransformation into *E. coli* DH5α followed by extensive restriction analysis. We observed that the red fluorescent phenotype was fully stable during continued mycelial growth on non-selective media and also upon conidiospore formation and germination on non-selective medium for at least two cycles. This again implies the presence of a fully stable replicating plasmid in *P*. *chrysogenum* cells.

Deletion of the genomic copy of *aur1* in transformants was demonstrated by colony PCR using the following oligonucleotide combinations:
DSM-JAK-168
   5'- AGCTTTGACGCTAGATTGGAGATG -3' (SEQ ID. NO: 54) + 5-prime-niaD-return (SEQ ID. NO. 18) (expected 1647 bp) and
DSM-JAK-169
   5'- CAAGCAAGCCATCTCAACAAGTGC -3' (SEQ ID. NO: 55) + 3-prime-niaD-forward (SEQ ID. NO. 8) (expected 1531 bp)

These should only amplify a DNA fragment of the indicated size upon correct recombination at the *aur1* locus. Multiple independent PCR positive transformants were identified and purified by sporulation and selection of single spores on acetamide selection plates. Southern blot analysis showed correct deletion of *aur1.* Multiple independent Δ*aur1* strains carrying a replicating plasmid with the complementing *Anid•aur1* expression cassette were identified.

### Example 21 Construction of a Saccharomyces cerevisiae TIF35 deletion cassette.

To demonstrate that the use of the *AMA1* replicon in the stable replicating plasmid is not essential for the method described here, we utilized an alternative replicon to stabilize plasmids in fungi. Since no other replicons are available for filamentous fungi besides *AMA1,* we chose to utilize a low-copy CEN/ARS containing plasmid with baker's yeast *Saccharomyces cerevisiae* as a host. The *S*. *cerevisiae TIF35* gene was chosen as essential gene to stabilize the replicating plasmid.

To delete the genomic copy of the S. *cerevisiae TIF35 (YDR429C)* gene, a Δ*tif35::loxP-KanMX4-loxP* deletion cassette of 1715 bp was prepared by PCR with the oligonucleotides
DSM-JAK-139
   5'-TACTCGCTGTATTGAAAGGATCAAAAGACCAAAGACCACCAGGAATAATGCCAGCT GAAGCTTCGTACGC -3' (SEQ ID NO: 56) and
DSM-JAK-140
using plasmid pUG6 as template. The sequence of the deletion cassette is given in SEQ ID. NO: 58. Before use the PCR fragment was purified from agarose gel.

### Example 22 Construction of CEN/ARS plasmids pDSM-JAK-134 and pDSM-JAK-135 containing Saccharomyces cerevisiae TIF35 and DsRed.SKL marker.

A *CEN*/*ARS* plasmid containing *S*. *cerevisiae TIF35* was constructed as follows. First, we provided plasmid pUG34-DsRed.SKL with a constitutively expressed *DsRed.SKL* gene by replacing the *S*. *cerevisiae MET25* promoter by the *S*. *cerevisiae TDH3 (YGR192C)* promoter. A 728 bp DNA fragment comprising the *Scer•TDH3* promoter region (nt 884500 to 883790 in Genbank BK006941.2) was amplified with oligonucleotides
DSM-JAK-148
   5'- GAATAAAAAAGAGCTCACGCTTTTTCAGTTCGAGTTTATC -3'
   (SEQ ID. NO: 59)
and DSM-JAK-149
   5'- GTTAATAGCAACTCTAACCATGGTTTGTTTGTTTATGTGTG -3'
   (SEQ ID. NO: 60),
using genomic *S*. *cerevisiae* BY4742 DNA as template. The PCR fragment was digested with *Sac*I and *Nco*I and cloned between the *Sac*I and *Nco*I sites of plasmid pUG34-DsRed.SKL, yielding plasmid pDSM-JAK-133 (Fig. 17). In this way an *E. coli* / *S*. *cerevisiae* shuttle vector was constructed which contains the *Scer•HIS3* auxotrophic marker, the *ARS*/*CEN* replicon and the *DsRed.SKL* gene. This plasmid is used as the control plasmid during stability experiments (Example 23). Subsequently, the *Scer•H*/*S3* marker of pDSM-JAK-133 was replaced by the *S*. *cerevisiae TIF35* gene. A 1449 bp DNA fragment comprising the *Scer•TIF35* gene and its promoter region (nt 1325908 to 1324469 in Genbank BK006938.2) was amplified with oligonucleotides
DSM-JAK-137
   5'- GCTTATGGTGGTGGTGCTTCTTATAG -3'
(SEQ ID. NO: 61)
   and DSM-JAK-138
5'- AGAGGGTACCTGTGCATCTATTCCTTAACCTTAGG -3'
   (SEQ ID. NO: 62),
using genomic *S*. *cerevisiae* BY4742 DNA as template. The PCR fragment was digested with either *Kpn*I or *Acc*65I and cloned between the *Eco*47III + *Kpn*I or *Eco*47III + *Bsi*WI sites of plasmid pDSM-JAK-133, respectively, yielding plasmids pDSM-JAK-134 (Fig. 18) and pDSM-JAK-135 (Fig. 19).

### Example 23 Stabilization of CEN/ARS plasmids in the yeast Saccharomyces cerevisiae

Plasmids pDSM-JAK-134 and pDSM-JAK-135 (Example 22) were co-transformed in circular form with the *Scer•tif35* deletion cassette (Example 21) into competent cells of *S*. *cerevisiae* BY4742. Transformants were selected on geneticin (G418) plates. Using a led lamp red fluorescent colonies harbouring the *DsRed.SKL-*expressing plasmid were identified with high frequency (>50 %). In the majority of the cases plasmid pDSM-JAK-134 or pDSM-JAK-135 was present in a fully intact form as demonstrated by retransformation into *E. coli* DH5α followed by extensive restriction analysis. As a control, we transformed plasmid pDSM-JAK-133 into competent cells of *S. cerevisiae* BY4742 with selection on histidine prototrophy. Using Fluorescence Assisted Cell Sorting (FACS), we observed that the red fluorescent phenotype in cells with either pDSM-JAK-134 or pDSM-JAK-135 was fully stable during continued growth on non-selective media for at least 38 generations. This implies the presence of a fully stable replicating plasmid in *S. cerevisiae* cells. In contrast, *S. cerevisiae* cells containing pDSM-JAK-133 lost the red fluorescence (approx. 1 % per generation) when grown in medium supplemented with histidine.

Deletion of the genomic copy of *TIF35* in transformants was demonstrated by colony PCR using the following oligonucleotides:
DSM-JAK-146
   5'- AGTACGGTCATTGGACCTGGAATC -3'
   (SEQ ID. NO: 63)
and DSM-JAK-147
   5'- CGTTCCATGCACCTCCATGAATGT -'3
   (SEQ ID. NO: 64)

These should either amplify a DNA fragment of 2249 bp upon correct recombination at the *TIF35* locus or one of 1454 bp when the wild type locus is present. Multiple independent PCR positive transformants were identified.

### Example 24 Determination of the copy number of stable plasmids in P. chrysogenum by quantitative PCR analysis.

To analyse the copy number of the stable replicating plasmids in *P*. *chrysogenum* co-transformants, we performed quantitative PCR (qPCR) using total genomic DNA. The gene copy numbers were determined with a Miniopticon™ system (Bio Rad) using the Bio Rad CFX manager software in which the C(t) values were determined automatically by regression. The SensiMix™ SYBRmix (Bioline, Alphen aan den Rijn, The Netherlands) was used as a master mix for qPCR with 0.4 µM primers and 100 ng total DNA in a 25 µl reaction volume. Copy numbers were calculated from duplicate experiments.

Copy number determination of the stable replicating plasmids pDSM-JAK-108 and pDSM-JAK-120 carrying the essential *tif35* gene, in *P. chrysogenum* co-transformants (Examples 7 and 11, respectively) were performed using the primers DSM-JAK-174
5' - CCACCGTTGTCCGCGAACAT - 3' (SEQ ID NO: 65)
   and DSM-JAK-175
5' - TCCTTCTCGGCCTCCTTAGC - 3' (SEQ ID NO: 66),
which amplify a 178 bp DNA fragment of *P. chrysogenum tif35.* As reference, two primers,
Act-F3
   5' - CTGGCGGTATCCACGTCACC - 3' (SEQ ID NO: 67)
and Act-R3
   5' - AGGCCAGAATGGATCCACCG - 3' (SEQ ID NO: 68)
were used that amplify a 300 bp fragment of the gene encoding *P*. *chrysogenum* γ-actin (Genbank accession number AF056975) that is present in one copy in the genome. The untransformed strains DS54465 and DS61187 were used as controls, carrying each a single copy of *tif35* in their genomes.

In two independent *P. chrysogenum* DS54465 Δ*tif35::niaD-amdS-niaD* [pDSM-JAK-108] strains (Example 7), plasmid pDSM-JAK-108 was present in approximately 8 copies per genome, while in *P. chrysogenum* DS61187 Δ*tif35::niaD-amdS-niaD* [pDSM-JAK-108] (Example 7) and *P. chrysogenum* DS54465 Δ*tif35::niaD-amdS-niaD* [pDSM-JAK-120] co-transformants (Example 11) the plasmid copy number was 4 and 3- 4 per genome, respectively.

To determine the copy number of plasmid pDSM-JAK-136 carrying the *A*. *nidulans aur1* gene in *P. chrysogenum* DS54465 Δ*aur1::niaD-amdS-niaD* [pDSM-JAK-136] transformants (Example 20), we used the primers: DSM-JAK-178
5' - GGCTGGCTGTTAGTCAACTG - 3' (SEQ ID NO: 69) and
   DSM-JAK-179
5' - AGGAGGCTGACCTCGATTGT - 3' (SEQ ID NO: 70)
that amplify a 181 bp DNA fragment of the region of plasmid pDSM-JAK-136 comprising the *A. nidulans AN0465* promoter (Example 6). Again the gene encoding gamma actin was used as a reference. Since the P*_{AN0465}* DNA fragment is lacking in the *P. chrysogenum* genome, in this case a strain carrying 4 copies of the P*_{AN0465}* region per genome, DS61187 Δ*tif35::niaD-amdS-niaD* [pDSM-JAK-108] (Example 7), was used as a reference, while strain DS54465, carrying 0 copies/genome was used as negative control. We observed that pDSM-JAK-136 was present in 10 to 11 copies per genome. We presume that the higher copy number of this plasmid as compared to plasmids pDSM-JAK-108 and pDSM-JAK-120 is caused by the use of the heterologous *aur1* gene, which may be less expressed than the homologous *P. chrysogenum aur1* gene thereby driving an increase in copy number.

## Claims

1. Method for the production of a biological compound of interest comprising culturing a filamentous fungal host cell deficient in an essential gene, comprising a vector, said vector comprising at least said essential gene and an autonomous replication sequence, wherein the host cell comprises a recombinant polynucleotide construct comprising a polynucleotide encoding a biological compound of interest or a compound involved in the synthesis of a biological compound of interest under conditions conducive to the production of the biological compound of interest and
isolating the compound of interest from the culture broth,
wherein a recombinant polynucleotide construct encoding the biological compound of interest or a compound involved in the synthesis of the biological compound of interest is located on said vector.

2. Method for the production of a biological compound of interest comprising producing a filamentous fungal host cell according to a method which comprises:
a. providing a filamentous fungal host cell and a vector which comprises at least a gene essential for said host cell and an autonomous replication sequence, wherein a recombinant polynucleotide construct encoding the compound of interest or a compound involved in the synthesis of the biological compound of interest is located on said vector; and
b. co-transforming the host cell with the vector and a disruption construct for said essential gene to render the host cell deficient in the essential gene;
culturing such a host cell under conditions conducive to the production of the biological compound of interest and isolating the compound of interest from the culture broth.

3. Method according to claim 1 or 2, wherein the host cell is, preferably inducibly, increased in its efficiency of homologous recombination (HR), decreased in its efficiency of non-homologous recombination (NHR) or decreased in its ratio of non-homologous recombination/homologous recombination (NHR/HR).

4. Method according to any one of the preceding claims, wherein the host cell deficiency in the essential gene is inducible.

5. Method according to any one of the preceding claims, wherein the essential gene is an essential gene in fungi.

6. Method according to any one of the preceding claims, wherein the essential gene is the *tif35* or *aur1* gene.

7. Method according to any one of the preceding claims, wherein the host cell is an *Aspergillus, Chrysosporium, Penicillium, Rasamsonia, Talaromyces* or *Trichoderma.*

8. Method according to claim 7, wherein the host cell is *Aspergillus niger, Penicillium chrysogenum,* or *Rasamsonia emersonii.*

9. Method according to any one of the preceding claims, wherein the vector contains control sequences from a species other than the host species.

10. Method according to any one of the preceding claims, wherein the biological compound of interest is a biopolymer or a metabolite.

11. Method according to claim 10, wherein the biopolymer is a polypeptide.

12. Method according to claim 11, wherein the polypeptide is an antibody or parts thereof, an antigen, a clotting factor, an enzyme, a hormone or a hormone variant, a receptor or parts thereof, a regulatory protein, a structural protein, a reporter, or a transport protein, protein involved in secretion process, protein involved in folding process, chaperone, peptide amino acid transporter, glycosylation factor, transcription factor, synthetic peptide or oligopeptide, intracellular protein.

13. Method according to claim 12, wherein the enzyme is a protease, ceramidase, epoxide hydrolase, aminopeptidase, acylase, aldolase, hydroxylase, aminopeptidase, lipase, non-ribosomal synthetase, polyketide synthetase, oxidoreductase, transferase, hydrolase, lyase, isomerase, catalase, cellulase, chitinase, cutinase, deoxyribonuclease, dextranase, esterase, carbohydrase, e.g. cellulase such as endoglucanase, β-glucanase, cellobiohydrolase or β-glucosidase, GH61-enzyme, hemicellulase, pectinolytic enzyme such as xylanase, xylosidase, mannanase, galactanase, galactosidase, pectin methyl esterase, pectin lyase, pectate lyase, endo polygalacturonase, exopolygalacturonase, rhamnogalacturonase, arabanase, arabinofuranosidase, arabinoxylan hydrolase, galacturonase, lyase, or amylolytic enzyme; hydrolase, isomerase, or ligase, phosphatase such as phytase, esterase such as lipase, phospholipase, galactolipase, proteolytic enzyme, dairy enzyme such as chymosin, oxidoreductase such as oxidase, transferase, isomerase, phytase, aminopeptidase, asparaginase, amylase, carbohydrase, carboxypeptidase, endo-protease, metallo-protease, serine-protease, catalase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, protein deaminase, invertase, laccase, lipase, mannosidase, mutanase, oxidase, pectinolytic enzyme, peroxidase, phospholipase, polyphenoloxidase, ribonuclease, transglutaminase, or glucose oxidase, hexose oxidase, monooxygenase.

14. Method according to any one of the preceding claims wherein the host cell is already capable to produce the biological compound of interest.

## Patentansprüche

1. Verfahren zur Produktion einer biologischen Verbindung von Interesse, umfassend Kultivieren einer an einem essenziellen Gen defizienten Fadenpilzwirtszelle, die einen Vektor umfasst, wobei der Vektor mindestens das essenzielle Gen und eine autonome Replikationssequenz umfasst, wobei die Wirtszelle ein rekombinantes Polynukleotidkonstrukt umfasst, das ein Polynukleotid umfasst, das für eine biologische Verbindung von Interesse oder eine Verbindung, die an der Synthese einer biologischen Verbindung von Interesse beteiligt ist, codiert,
unter Bedingungen, die der Produktion der biologischen Verbindung von Interesse förderlich sind,
und
Isolieren der Verbindung von Interesse aus der Kulturbrühe,
wobei sich ein rekombinantes Polynukleotidkonstrukt, das für die biologische Verbindung von Interesse oder für eine Verbindung, die an der Synthese der biologischen Verbindung von Interesse beteiligt ist, codiert, auf dem Vektor befindet.

2. Verfahren zur Produktion einer biologischen Verbindung von Interesse, umfassend Produzieren einer Fadenpilzwirtszelle gemäß einem Verfahren, das Folgendes umfasst:
a. Bereitstellen einer Fadenpilzwirtszelle und eines Vektors, der mindestens ein Gen, das für die Wirtszelle essenziell ist, und eine autonome Replikationssequenz umfasst, wobei sich ein rekombinantes Polynukleotidkonstrukt, das für die Verbindung von Interesse oder für eine Verbindung, die an der Synthese der biologischen Verbindung von Interesse beteiligt ist, codiert, auf dem Vektor befindet; und
b. Kotransformieren der Wirtszelle mit dem Vektor und einem Disruptionskonstrukt für das essenzielle Gen, um die Wirtszelle an dem essenziellen Gen defizient zu machen,
Kultivieren solch einer Wirtszelle unter Bedingungen, die der Produktion der biologischen Verbindung von Interesse förderlich sind, und
Isolieren der Verbindung von Interesse aus der Kulturbrühe.

3. Verfahren nach Anspruch 1 oder 2, wobei die Wirtszelle vorzugsweise induzierbar in ihrer homologen Rekombinationseffizienz (HR-Effizienz) erhöht ist, in ihrer nichthomologen Rekombinationseffizienz (NHR-Effizienz) vermindert ist oder in ihrem Verhältnis zwischen nichthomologer Rekombination/homologer Rekombination (NHR/HR) vermindert ist.

4. Verfahren nach einem der vorhergehenden Ansprüche, wobei das Defizienz der Wirtszelle an dem essenziellen Gen induzierbar ist.

5. Verfahren nach einem der vorhergehenden Ansprüche, wobei das essenzielle Gen ein in Pilzen essenzielles Gen ist.

6. Verfahren nach einem der vorhergehenden Ansprüche, wobei das essenzielle Gen das *tif35-* oder *aur1*-Gen ist.

7. Verfahren nach einem der vorhergehenden Ansprüche, wobei die Wirtszelle ein(e) *Aspergillus, Chrysosporium, Penicillium, Rasamsonia, Talaromyces* oder *Trichoderma* ist.

8. Verfahren nach Anspruch 7, wobei die Wirtszelle *Aspergillus niger, Penicillium chrysogenum* oder *Rasamsonia emersonii* ist.

9. Verfahren nach einem der vorhergehenden Ansprüche, wobei der Vektor Kontrollsequenzen von einer Spezies, bei der es sich nicht um die Wirtsspezies handelt, enthält.

10. Verfahren nach einem der vorhergehenden Ansprüche, wobei die biologische Verbindung von Interesse ein Biopolymer oder ein Metabolit ist.

11. Verfahren nach Anspruch 10, wobei das Biopolymer ein Polypeptid ist.

12. Verfahren nach Anspruch 11, wobei das Polypeptid ein Antikörper oder Teile davon, ein Antigen, ein Gerinnungsfaktor, ein Enzym, ein Hormon oder eine Hormonvariante, ein Rezeptor oder Teile davon, ein Regulationsprotein, ein Strukturprotein, ein Reporter oder ein Transportprotein, ein Protein, das an einem Sekretionsvorgang beteiligt ist, ein Protein, das an einem Faltungsvorgang beteiligt ist, ein Chaperon, ein Peptidaminosäuretransporter, ein Glycosylierungsfaktor, ein Transkriptionsfaktor, ein synthetisches Peptid oder Oligopeptid, ein intrazelluläres Protein ist.

13. Verfahren nach Anspruch 12, wobei das Enzym eine Protease, Ceramidase, Epoxidhydrolase, Aminopeptidase, Acylase, Aldolase, Hydroxylase, Aminopeptidase, Lipase, nichtribosomale Synthetase, Polyketidsynthetase, Oxidoreduktase, Transferase, Hydrolase, Lyase, Isomerase, Catalase, Cellulase, Chitinase, Cutinase, Desoxyribonuklease, Dextranase, Esterase, Carbohydrase, z.B. Cellulase wie Endoglucanase, β-Glucanase, Cellobiohydrolase oder β-Glucosidase, GH61-Enzym, Hemicellulase, pectinolytisches Enzym wie Xylanase, Xylosidase, Mannanase, Galactanase, Galactosidase, Pectinmethylesterase, Pectinlyase, Pectatlyase, Endopolygalacturonase, Exopolygalacturonase, Rhamnogalacturonase, Arabanase, Arabinofuranosidase, Arabinoxylanhydrolase, Glacturonase, Lyase oder amylolytisches Enzym; Hydrolase, Isomerase oder Ligase, Phosphatase wie Phytase, Esterase wie Lipase, Phospholipase, Galactolipase, proteolytisches Enzym, Molkereienzym wie Chymosin, Oxidoreduktase wie Oxidase, Transferase, Isomerase, Phytase, Aminopeptidase, Asparaginase, Amylase, Carbohydrase, Carboxypeptidase, Endoprotease, Metalloprotease, Serinprotease, Catalase, Chitinase, Cutinase, Cyclodextringlycosyltransferase, Desoxyribonuklease, Esterase, alpha-Galactosidase, beta-Galactosidase, Glucoamylase, alpha-Glucosidase, beta-Glucosidase, Halogenperoxidase, Proteindeaminase, Invertase, Laccase, Lipase, Mannosidase, Mutanase, Oxidase, pectinolytisches Enzym, Peroxidase, Phospholipase, Polyphenoloxidase, Ribonuklease, Transglutaminase oder Glucoseoxidase, Hexoseoxidase, Monooxygenase ist.

14. Verfahren nach einem der vorhergehenden Ansprüche, wobei die Wirtszelle bereits fähig ist, die biologische Verbindung von Interesse zu produzieren.

## Revendications

1. Méthode de production d'un composé biologique d'intérêt, comprenant la culture d'une cellule hôte fongique filamenteuse déficiente en un gène essentiel, comprenant un vecteur, ledit vecteur comprenant au moins ledit gène essentiel et une séquence de réplication autonome, où la cellule hôte comprend une construction polynucléotidique recombinée comprenant un polynucléotide codant pour un composé biologique d'intérêt ou un composé impliqué dans le synthèse d'un composé biologique d'intérêt,
dans des conditions favorables à la production du composé biologique d'intérêt, et
l'isolement du composé d'intérêt à partir du milieu de culture,
où une construction polynucléotidique recombinée codant pour le composé biologique d'intérêt ou un composé impliqué dans la synthèse du composé biologique d'intérêt est située sur ledit vecteur.

2. Méthode de production d'un composé biologique d'intérêt, comprenant la production d'une cellule hôte fongique filamenteuse selon la méthode, comprenant :
a. la fourniture d'une cellule hôte fongique filamenteuse et d'un vecteur comprenant au moins un gène essentiel pour ladite cellule hôte et une séquence de réplication autonome, où une construction polynucléotidique recombinée codant pour le composé d'intérêt ou un composé impliqué dans la synthèse du composé biologique d'intérêt est située sur ledit vecteur ; et
b. la co-transformation de la cellule hôte par le vecteur et une construction de disruption pour ledit gène essentiel afin de rendre la cellule hôte déficiente en ce gène essentiel ;
la culture d'une telle cellule hôte dans des conditions favorables à la production du composé biologique d'intérêt, et
l'isolement du composé d'intérêt à partir du milieu de culture.

3. Méthode selon la revendication 1 ou 2, dans laquelle la cellule hôte, préférablement de manière inductible, voit son activité augmentée pour la recombinaison homologue (RH), son activité réduite pour la recombinaison non homologue (RNH), ou son rapport réduit de recombinaison non homologue/recombinaison homologue (RNH/RH).

4. Méthode selon l'une quelconque des revendications précédentes, dans laquelle la déficience de la cellule hôte pour le gène essentiel est inductible.

5. Méthode selon l'une quelconque des revendications précédentes, dans laquelle le gène essentiel est un gène essentiel chez le champignon.

6. Méthode selon l'une quelconque des revendications précédentes, dans laquelle le gène essentiel est le gène *tif35* ou *aur1.*

7. Méthode selon l'une quelconque des revendications précédentes, dans laquelle la cellule hôte est *Aspergillus, Chrysosporium, Penicillium, Rasamsonia, Talaromyces* ou *Trichoderma.*

8. Méthode selon la revendication 7, dans laquelle la cellule hôte est *Aspergillus niger, Penicillium chrysogenum,* ou *Rasamsonia emersonii.*

9. Méthode selon l'une quelconque des revendications précédentes, dans laquelle le vecteur contient des séquences de contrôle issues d'une espèce autre que l'espèce hôte.

10. Méthode selon l'une quelconque des revendications précédentes, dans laquelle le composé biologique d'intérêt est un biopolymère ou un métabolite.

11. Méthode selon la revendication 10, dans laquelle le biopolymère est un polypeptide.

12. Méthode selon la revendication 11, dans laquelle le polypeptide est un anticorps ou des parties de celui-ci, un antigène, un facteur de coagulation, une enzyme, une hormone ou un variant d'hormone, un récepteur ou des parties de celui-ci, une protéine régulatrice, une protéine structurelle, un rapporteur, ou une protéine de transport, une protéine impliquée dans le processus de sécrétion, une protéine impliquée dans le processus de repliement, une chaperonne, un transporteur de peptide/acide aminé, un facteur de glycosylation, un facteur de transcription, un peptide ou oligopeptide synthétique, une protéine intracellulaire.

13. Méthode selon la revendication 12, dans laquelle l'enzyme est une protéase, une céramidase, une époxyde hydrolase, une aminopeptidase, une acylase, une aldolase, une hydroxylase, une aminopeptidase, une lipase, une synthétase non ribosomale, une polycétide synthétase, une oxydoréductase, une transférase, une hydrolase, une lyase, une isomérase, une catalase, une cellulase, une chitinase, une cutinase, une désoxyribonucléase, une dextranase, une estérase, une carbohydrase, par exemple une cellulase telle que l'endoglucanase, la β-glucanase, la cellobiohydrolase ou la β-glucosidase, une enzyme GH61, une hémicellulase, une enzyme pectinolytique telle que la xylanase, la xylosidase, la mannanase, la galactanase, la galactosidase, la pectine méthyl estérase, la pectine lyase, la pectate lyase, l'endo-polygalacturonase, l'exo-polygalacturonase, la rhamnogalacturonase, l'arabanase, l'arabinofuranosidase, l'arabinoxylane hydrolase, la galacturonase, la lyase, ou une enzyme amylolytique ; une hydrolase, une isomérase, ou une ligase, une phosphatase telle que la phytase, une estérase telle que la lipase, la phospholipase, la galactolipase, une enzyme protéolytique, une enzyme du lait telle que la chymosine, une oxydoréductase telle que l'oxydase, une transférase, une isomérase, une phytase, une aminopeptidase, une asparaginase, une amylase, une carbohydrase, une carboxypeptidase, une endoprotéase, une métalloprotéase, une sérine protéase, une catalase, une chitinase, une cutinase, une cyclodextrine glycosyltransférase, une désoxyribonucléase, une estérase, une alpha-galactosidase, une bêtagalactosidase, une glucoamylase, une alpha-glucosidase, une bêta-glucosidase, une halopéroxydase, une protéine désaminase, une invertase, une laccase, une lipase, une mannosidase, une mutanase, une oxydase, une enzyme pectinolytique, une peroxydase, une phospholipase, une polyphénol oxydase, une ribonucléase, une transglutaminase, ou une glucose oxydase, une hexose oxydase, une monooxygénase.

14. Méthode selon l'une quelconque des revendications précédentes, dans laquelle la cellule hôte est déjà capable de produire le composé biologique d'intérêt.
